# EUROPEAN PATENT APPLICATION

(11) **EP 2 292 621 A1**
(43) Date of publication of application: **09.03.2011**
(21) Application number: 10176800.0
(22) Date of filing: 24.08.2005
(51) Int. Cl.: C07D 487/04, C07D 498/14, C07D 513/14, A61K 31/5517, A61K 31/553, C07D 417/10, A61K 31/55, A61K 31/495, A61K 31/38

(54) **Vasopressin v1a antagonists**

(30) Priority: 24.08.2004 US 603557 P; 24.08.2004 EP 04104062
(62) Divisional of application: 05773384.2
(71) Applicant: Vantia Limited, Southampton Science Park Chilworth Hampshire SO16 7NP (GB)
(72) Inventor: Andrzej, Roman, Batt, Southampton, Hampshire SO17 2LJ (GB); Baxter, Andrew, John, Romsey, Hampshire SO51 7HU (GB); Heeney, Celine, Southampton, Hampshire SO14 6TQ (GB); Stockley, Martin, Lee, Southampton, Hampshire SO16 8FY (GB); Roe, Michael Bryan, Ringwood, Hampshire BH24 1QU (GB); Hudson, Peter, 2300, Copenhagen (DK); Handy, Rachel, Chandler's Ford, Hampshire SO53 2PD (GB)
(74) Representative: Hallybone, Huw George

(57) **Abstract**

The present invention relates to compounds with vasopressin V1a antagonist activity and to pharmaceutical compositions comprising such compounds. The present invention also relates to the use of vasopressin V1a antagonists for the treatment of certain physiological disorders, such as Raynaud's disease and dysmenorrhoea (primary dysmenorrhoea and/or secondary dysmenorrhoea).

## Description

### Field of the Invention

The present invention relates to compounds with vasopressin V₁ₐ antagonist activity and to pharmaceutical compositions comprising such compounds. The present invention also relates to the use of vasopressin V₁ₐ antagonists for the treatment of certain physiological disorders, such as Raynaud's disease and dysmenorrhoea (primary dysmenorrhoea and/or secondary dysmenorrhoea).

Priority is claimed from EP patent application number 04104062 and US patent application number 60/603557, both filed 24 August 2004.

### Background

The neurophyseal hormones vasopressin (VP) and oxytocin (OT) are cyclic nonapeptides secreted by the posterior pituitary gland.

Only one OT receptor has so far been well characterised, while three VP receptors are known. These are designated the V₁ₐ, V_{1b} and V₂ receptors.

Vasopressin acts on the blood vessels, where it is a potent vasoconstrictor, and on the kidneys, where it promotes water reuptake leading to an antidiuretic effect.

The V₁ₐ, V_{1b}, and V₂, as well as the OT receptors, are members of the super-family of seven transmembrane receptors known as G-protein coupled receptors. The V₁ₐ receptor mediates phospholipase C activation and intracellular calcium mobilisation. Localisation of the receptors includes blood platelets, blood vessels, hepatocytes, brain and uterus-cervix. Thus a V₁ₐ antagonist may have effects on any or all of these tissues. For example, selective V₁ₐ antagonists have been cited as having clinical utility in dysmenorrhoea, pre-term labour, hypertension, Raynaud's disease, brain oedema, motion sickness, hyperlipemia, small cell lung cancer, depression, anxiety, hyponatremia, liver cirrhosis and congestive heart failure.

With respect to dysmenorrhoea it has been proposed that myometrial activity is markedly increased in women with dysmenorrhoea during menstruation. It is proposed that the myometrial ischemia caused by increased uterine contractility might explain the menstrual pain. Furthermore, on the first day of menstruation, higher plasma concentrations of vasopressin have been measured in dysmenorroeic women than in controls.

In healthy women without dysmenorrhoea, intravenous infusion of lysine-vasopressin resulted in decreased uterine blood flow, increased uterine contractility and slight to moderate dysmenorrhoea-like pain, these effects being inhibited by a selective human V₁ₐ receptor antagonist. (Bossmar, T. et al., BRITISH JOURNAL OF OBSTETRICS AND GYNAECOLOGY (1997 Apr), 104(4), 471-7). Also, it is known that vasopressin contracts human uterine arteries in a dose-dependent and V₁ₐ-mediated fashion.

The above evidence suggests that a V₁ₐ antagonist would be an appropriate and effective treatment for dysmenorrhoea (primary dysmenorrhoea and/or secondary dysmenorrhoea). Further evidence is taken from the clinical study carried out on the selective V₁ₐ antagonist SR49059 ("Effect of SR49059, an orally active V₁ₐ vasopressin receptor antagonist, in the prevention of dysmenorrhea". Brouard, R. et al., BRITISH JOURNAL OF OBSTETRICS AND GYNAECOLOGY (2000), 107(5), 614-619). It was found that there was a dose-related decrease in pain and a dose-related decrease in the amount of additional pain-killer taken compared to patients taking placebo.

The International Patent Application WO 03/016316 A1, published 27 February 2003, discloses a number of compounds which are claimed to be oxytocin agonists and to find use in the treatment of male erectile dysfunction. No V₁ₐ antagonist activity is reported. The European Patent Application EP 1 449 844 A1, published 25 August 2004, discloses a number of compounds which are claimed to be V₁ₐ antagonists and to find use in the treatment of primary dysmenorrhoea.

There exists a need for treatments for conditions which are associated with the V₁ₐ receptors. There further continues to exist a need for alternative V₁ₐ antagonists. Simple synthesis and oral availability are additional desirable characteristics.

### Disclosure of the Invention

According to an aspect, the present invention relates to compounds, preferably VP antagonists, and in particular specific antagonists of the V₁ₐ receptor, and pharmaceutically acceptable salts thereof.

According to another aspect, the present invention relates to pharmaceutical compositions comprising these compounds, which compositions are useful for the treatment of, inter alia, dysmenorrhoea (primary dysmenorrhoea and/or secondary dysmenorrhoea).

According to another aspect, the present invention relates to the use of the compounds for the manufacture of a pharmaceutical composition for treatment of dysmenorrhoea.

According to further aspects, the present invention relates to the medical use and to the use of the above mentioned compounds and compositions in therapy and to therapeutic methods wherein the above mentioned compounds and compositions are used.

### Detailed Description of the Invention

According to an aspect the invention concerns a compound according to general formula **1a**, or a compound which is a tautomer or a pharmaceutically acceptable salt thereof, 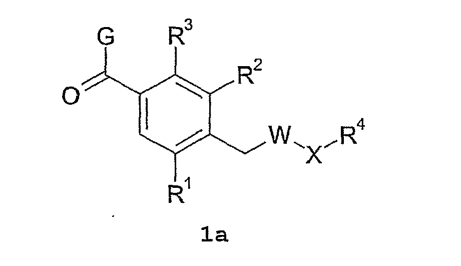 wherein G is a group selected among general formula **2a**, **3a, 4a**, **5a**, and **6a,** 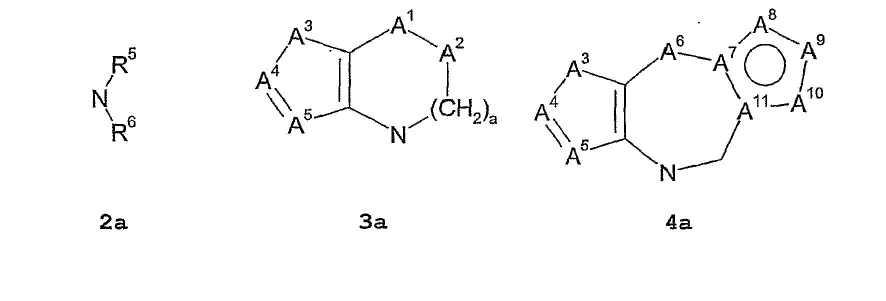 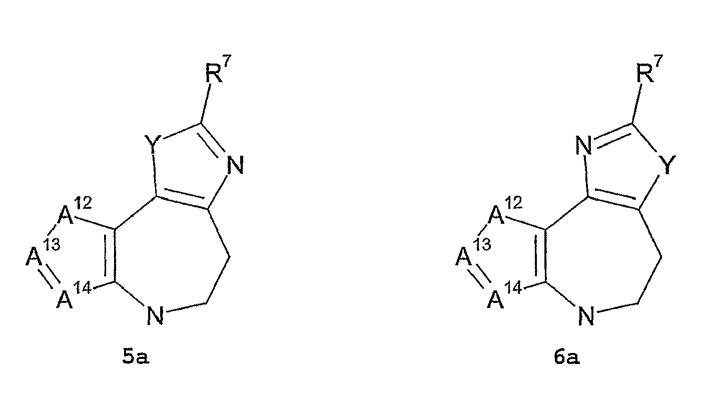 wherein:
A¹ is selected among CH₂, CH(OH), NH, N-alkyl, O and S;
A² is selected among CH₂, CH(OH), C(=O) and NH;
A³ and A¹² are independently selected among S, NH, N-alkyl, -C(R⁸)=CH-, -C(R⁸)=N-, -N=C(R⁸)- and -CH=C(R⁸)-;
A⁴ and A¹³ are independently selected among C(R⁹) and N;
A⁵ and A¹⁴ are independently selected among C(R¹⁰) and N;
A⁶ is selected among CH₂, NH, N-alkyl and O;
A⁷ and A¹¹ are independently selected among C and N;
A⁸ and A⁹ are independently selected among CH, N, NH, N(CH₂)_{b}R¹¹ and S ;
A¹⁰ is selected among -CH=CH-, CH, N, NH, N-(CH₂)_{b}-R¹¹ and S;
wherein the ring constituted by A⁷, A⁸, A⁹, A¹⁰ and A¹¹ is aromatic;
R¹, R² and R³ are independently selected among H, alkyl, 0-alkyl, NO₂, F, Cl and Br;
R⁴ is selected among H, alkyl, aryl, heteroaryl, (CH₂)-R¹², and 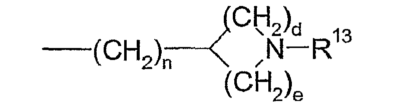
R⁵ and R⁶ are independently selected among alkyl, aryl, -(CH₂)_{f}-aryl, and -(CH₂)_{f}-heteroaryl;
R⁷ is selected among H, alkyl, aryl, heteroaryl, and - (CH₂)_{g}-R¹⁴;
R⁸, R⁹ and R¹⁰ are independently selected among H, alkyl, alkoxy, F, Cl, Br, CN, NH₂, NO₂, NH(alkyl), N(alkyl)₂; with the provisos that when G is **3a**, and R⁴ is H, alkyl, aryl, heteroaryl or -(CH₂)_{c}-R¹², then the ring containing A³, A⁴ and A⁵ is substituted in at least one position by alkyl, alkoxy, F, Cl, Br, CN, NH₂, NO₂, NH (alkyl) or N(alkyl)₂; and
when G is **4a**, R⁴ is H, alkyl, aryl, heteroaryl or -(CH₂)_{c}-R¹², and A⁸ is NH, NCH₃ or S, then the ring containing A³, A⁴ and A⁵ is substituted in at least one position by alkyl, alkoxy, F, Cl, Br, CN, NH₂, NO₂, NH(alkyl) or N(alkyl)₂;
R¹¹ and R¹² are independently selected among H, alkyl, aryl, heteroaryl, F, OH, O-alkyl, S-alkyl, O-acyl, NH₂, NH-alkyl, N(alkyl)₂, NH-acyl, N(alkyl)-acyl, CO₂H, CO₂-alkyl, CONH₂, CONH-alkyl, CON(alkyl)₂, CN and CF₃; R¹³ is selected among H, alkyl, aryl, heteroaryl, - (CH₂)ₕ-R¹⁵ and Z-R¹⁶;
R¹⁴ and R¹⁵ are independently selected among H, alkyl, alkenyl, aryl, heteroaryl, F, OH', O-alkyl, S-alkyl, O-acyl, NH₂, NH-alkyl, N(alkyl)₂, NH-acyl, N(alkyl)-acyl, CO₂H, CO₂-alkyl, CO-alkyl, CO-aryl, CONH₂, CONH-alkyl, CON(alkyl)₂, alkenyl-CO₂-alkyl, alkenyl-aryl, CN and CF₃;
R¹⁶ is selected among H, alkyl, alkenyl, aryl, heteroaryl, O-aryl, -(CH₂)ᵢ-R¹⁷, cyclopropyl-aryl and O-(CH₂)ᵢ-R¹⁷;
R¹⁷ is selected among H, alkyl, aryl, heteroaryl, F, OH, O-alkyl, S-alkyl, O-acyl, NH₂, NH-alkyl, N(alkyl)₂, NH-acyl, N(alkyl)-acyl, CO₂H, CO₂-alkyl, CONH₂, CONH-alkyl, CON(alkyl)₂, CN and CF₃;
W is selected among O and NH;
X is selected among (CH₂)ₘ, C(=O) and S(=O)ⱼ
Y is selected among O, S, NH and N-alkyl;
Z is selected among -C(=O), -C(=O)-O and -S(=O)ₖ;
a is selected among 1 and 2;
b and c are independently selected among 0, 1, 2 and 3;
d, e and f are independently selected among 1 and 2;
g, h and i are independently selected among 1, 2 and 3;
j and k are independently selected among 1 and 2; and
m and n are independently selected among 0, 1 and 2.

According to an aspect, R¹⁶ may further be selected among 0-alkyl and O-alkenyl, and Z is different from -C(=O)-O.

It appears that the compounds of formula **1a** of claim 1 are subject to the following constraints. When G is **3a,** and R⁴ is H, alkyl, aryl, heteroaryl or -(CH₂)_{c}-R¹², then the ring containing A³, A⁴ and A⁵ is substituted in at least one position by alkyl, alkoxy, F, Cl, Br, CN, NH₂, NO₂, NH(alkyl) or N(alkyl)₂. When G is **4a**, R⁴ is H, alkyl, aryl, heteroaryl or -(CH₂)_{c}-R¹², and A⁸ is NH, NCH₃ or S, then the ring containing A³, A⁴ and A⁵ is substituted in at least one position by alkyl, alkoxy, F, Cl, Br, CN, NH₂, NO₂, NH(alkyl) or N(alkyl)₂. The ring constituted by A7, A8, A9, A10 and A11 is aromatic, and accordingly the groups must satisfy certain requirements. When A10 is -CH=CH- the ring is a six membered ring. As such, it can only comprise atoms of the type -C(R)= and -N=. Hence A7 and A11 must both be C and A8 and A9 must be either CH or N. When A10 is not -CH=CH- then the ring is a five-membered ring. In this case one, and only one, of the atoms in the ring must be S or a trigonal nitrogen. In this context, a "trigonal nitrogen" is a nitrogen atom linked covalently to three different atoms. Two of these atoms are the immediate neighbours to the nitrogen atom in the five-membered ring. The third is a hydrogen, carbon or other atom linked to the five-membered ring. Thus it follows that, when A10 is not -CH=CH- then one (and only one) of A7, A8, A9, A10 and A11 must be S or a trigonal nitrogen. Hence the selection of A7, A8, A9, A10 and A11 is subject to the following restrictions. If A10 is not -CH=CH- then one of A8, A9 and A10 is NH, N-(CH2)b-R11 or S or one of A7 and A11 is N. Not more than one of A8, A9 and A10 may be NH, N-(CH2)b-R11 or S. A7 and A11 may not both simultaneously be N. Neither A7 nor A11 may be N if one of A8, A9 and A10 is NH, N-(CH2)b-R11 or S.

According to aspects of the invention, additional and preferred embodiments of the invention are as set out below, in the description and the claims.

The term "alkyl" includes saturated hydrocarbon residues including:
- linear groups up to 10 atoms (C₁-C₁₀). Examples of such alkyl groups include, but are not limited to, C₁ - methyl, C₂ - ethyl, C₃ - propyl and C₄- n-butyl;
- branched groups of between 3 and 10 atoms (C₃-C₁₀).
   Examples of such alkyl groups include, but are not limited to, C₃ - iso-propyl, C₄ - sec-butyl, C₄ - isobutyl, C₄ - tert-butyl and C₅ - neo-pentyl;
- cyclic groups of between 3 and 8 atoms (C₃-C₈).
   Examples of such groups include but are not limited to, C₃ - cyclopropyl, C₄ - cyclobutyl, C₅ - cyclopentyl and C₆ - cyclohexyl;
- combinations of linear, branched and cyclic groups.
   Examples of such groups include, but are not limited to,

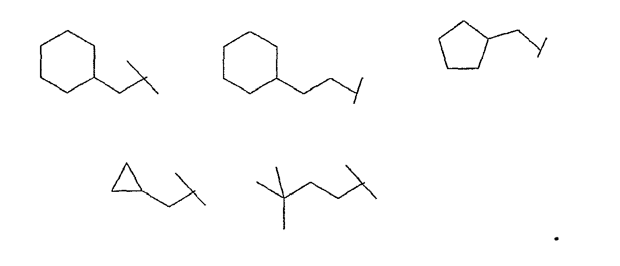

The term "alkoxy" is used to denote O-alkyl groups.

The term "alkenyl" includes monounsaturated hydrocarbon residues including
- linear groups of between two and six atoms (C₂-C₆). Examples of such alkenyl groups include, but are not limited to, C₂ - vinyl, C₃ - 1-propenyl, C₃ - allyl and C₄ - 2-butenyl;
- branched groups of between 3 and 8 atoms (C₃-C₈). Examples of such alkenyl groups include, but are not limited to, C₄ - 2-methyl-2-propenyl and C₆ - 2,3-dimethyl-2-butenyl;
- cyclic groups of between 3 and 8 atoms (C₃-C₈). Examples of such groups include, but are not limited to, C₅ - 3-cyclopentenyl and C₆ - 1-cyclohexenyl.

The term "aryl" includes optionally substituted phenyl and optionally substituted naphthyl. Examples of such aryl groups include, but are not limited to, phenyl, 2-tolyl, 3-chlorophenyl, 4-chlorophenyl, 3-fluorophenyl, 2-methoxyphenyl , 3-methoxyphenyl, 4-methoxyphenyl, 2,5-difluorophenyl, 1-naphthyl and 2-naphthyl.

The term "heteroaryl" includes optionally substituted heterocycles. Such heteroaryl groups include, but are not limited to, pyridyl, 2-chloropyridyl, 4-methylpyridyl, thienyl, 3-chlorothienyl, 2,3-dimethylthiophenyl, furyl, 2-methylfuryl, pyrrole, N-methylpyrrole, oxazole, imidazole, pyrazole and triazole.

The term "acyl" denotes a group R-C(=O), where R is H, a saturated or unsaturated hydrocarbon moiety of up to seven carbon atoms or an optionally substituted phenyl, optionally substituted pyridyl or optionally substituted thienyl group. Examples of acyl groups include, but are not limited to: formyl, acetyl, pivaloyl, benzoyl and nicotinoyl.

Certain compounds of the present invention are capable of forming salts with acids or bases. For example, compounds containing one or more basic nitrogen atoms can form addition salts with mineral and organic acids such as hydrochloric acid, sulphuric acid, phosphoric acid, acetic acid, trifluoroacetic acid, methanesulphonic acid, citric acid and benzoic acid. Compounds containing acidic groups can form salts with bases. Examples of such salts include the sodium, potassium, calcium, triethylammonium and tetraethylammonium salts. Furthermore, compounds that have both acidic and basic groups can form internal salts (zwitterions). Insofar as these salts are pharmaceutically acceptable, they are included within the scope of the invention.

Certain compounds within the scope of the present invention may exist as tautomers. For example, where G¹ is general formula **4a (4b, 4c, 4d)** and X² is NH the resulting imidazole can exist as its tautomer which is defined by G¹ as general formula **5a (5b, 5c, 5d)** and X² as NH. All such tautomers are considered to be within the scope of the present invention.

The compounds according to the present invention may have one or more stereogenic centres ("asymmetric carbon atoms") and so may exhibit optical isomerism. The scope of the present invention includes all epimers, enantiomers and diastereomers of compounds according to the present invention, including single isomers, mixtures and racemates.

According to an aspect, it is preferred that G is selected among:

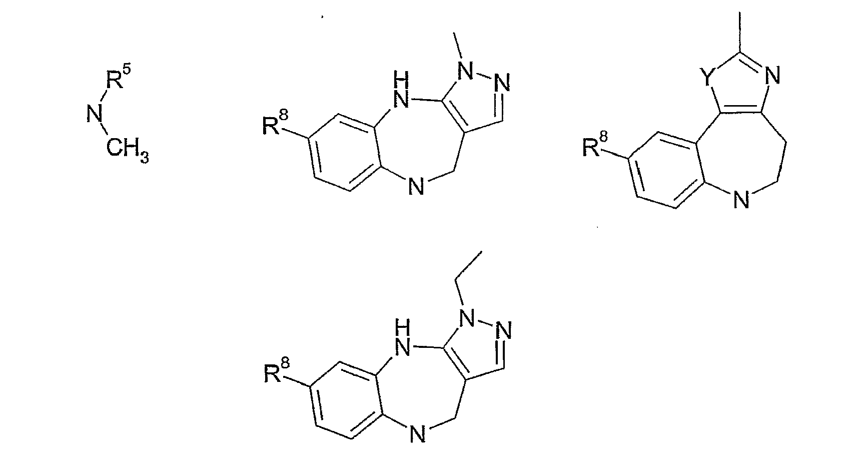

According to an aspect, it is further preferred that G is selected among:

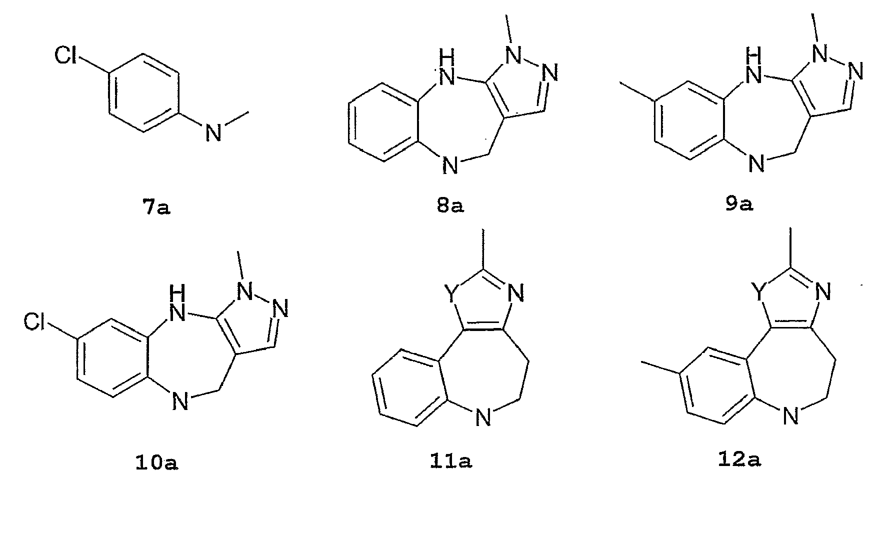

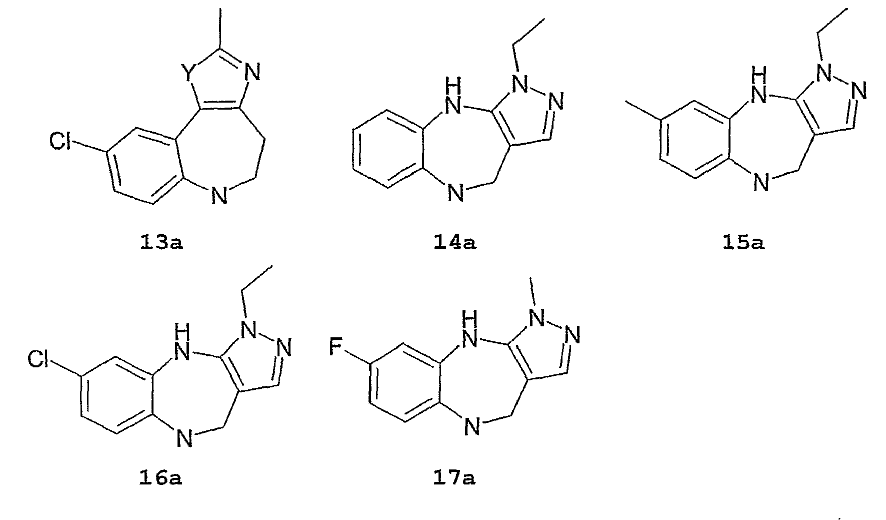

According to an aspect, it is preferred that at least one of R¹, R² and R³ is other than hydrogen; preferably one of R¹, R² and R³ is selected among methyl, chlorine and fluorine, and the others are hydrogen.

According to different aspects, it is preferred that W is NH; R⁴ is alkyl; d is 2 and e is 2; R¹³ is alkyl; and/or n is 0.

According to an aspect, it is preferred that R² is H, R⁴ is a piperidine where d is 2 and e is 2, W is NH, X is C(=O) and n is 0, as shown in formula **18a**: 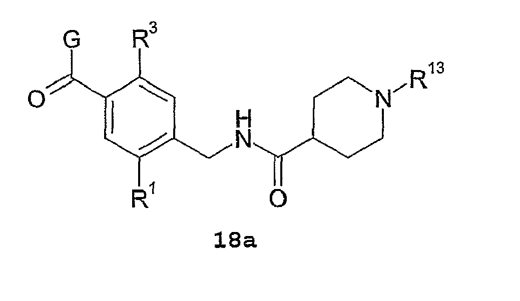 wherein R¹ is selected among methyl, chlorine and fluorine, and R³ is H; or R¹ is H, and R³ is selected among methyl, chlorine and fluorine, and R¹³ is alkyl.

According to an aspect, it is preferred that X is C(=O), n is 0 and R⁴ is a piperidine where d is 2 and e is 2, as shown in formula **19a**: 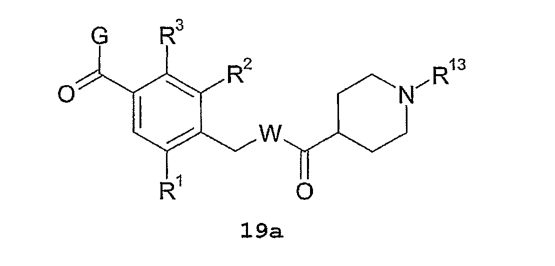 wherein G is selected among general formulae **7a** to **17a,** and R¹³ is alkyl.

According to an aspect, it is preferred that R² is H and W is NH as shown in formula **20a**: 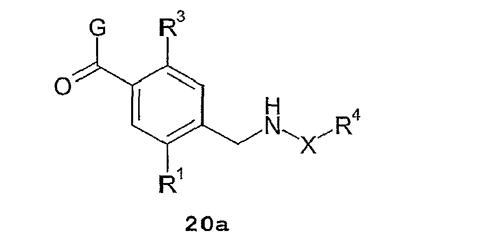 wherein either R¹ is selected among methyl, chlorine and fluorine, and R³ is H; or R¹ is H, and R³ is selected among methyl, chlorine and fluorine; and G is selected from general formulae **7a** to **17a**.

According to an aspect, it is preferred that R² is H, W is NH and X is C(=O) as shown in formula **21a**: 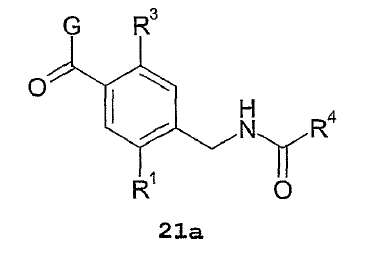 wherein R¹ is selected among methyl, chlorine and fluorine, and R³ is H; or R¹ is H, and R³ is selected among methyl, chlorine and fluorine; and R⁴ is alkyl.

According to an aspect, it is preferred that X is C(=O) as shown in formula **22a**: 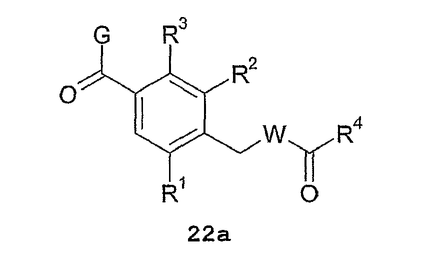 wherein G is selected among general formulae **7a** to **17a**, and R⁴ is alkyl.

According to an aspect, it is preferred that R² and R³ are both H, W is NH and X is C(=O) as shown in formula **23a:** 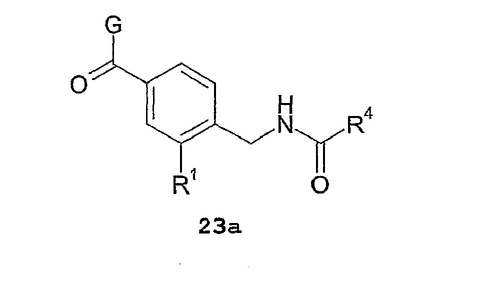 wherein G is selected among general formulae 9a, **10a, 15a, 16a and 17a,** R¹ is selected among methyl, chlorine and fluorine, and R⁴ is alkyl.

According to an aspect, it is preferred that R² and R³ are both H, R⁴ is a piperidine where d is 2 and e is 2, W is NH, X is C(=O) and n is 0 as shown in formula **24a:** 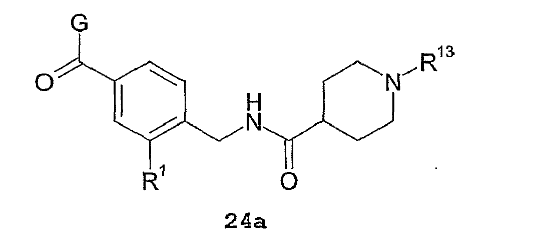 wherein G is selected among general formulae **9a**, **10a, 15a, 16a** and **17a,** R¹ is selected among methyl, chlorine and fluorine, and R¹³ is alkyl.

According to an aspect, particularly preferred compounds are provided in claim 17.

According to an aspect of the present invention, it relates to the compounds of claim 18. According to an aspect, the ring containing A3, A4 and A5 according to general formulae 2b and 3b is substituted in at least one position by alkyl, alkoxy, F, Cl, Br, CN, NH2, NO2, NH(alkyl) or N(alkyl)_{2.}

It appears that the compounds of formula **1b** of claim 18 are subject to following constraints. As the ring constituted by A⁷, A⁸, A⁹, A¹⁰ and A¹¹ is aromatic, the groups must satisfy certain requirements. When A¹⁰ is - CH=CH- the ring is a six membered ring. As such, it can only comprise atoms of the type -C(R)= and -N=. Hence A⁷ and A¹¹ must both be C and A⁸ and A⁹ must be either CH or N. When A¹⁰ is not -CH=CH- then the ring is a five-membered ring. In this case one, and only one, of the atoms in the ring must be S or a trigonal nitrogen. In this context, a "trigonal nitrogen" is a nitrogen atom linked covalently to three different atoms. Two of these atoms are the immediate neighbours to the nitrogen atom in the five-membered ring. The third is a hydrogen, carbon or other atom linked to the five-membered ring. Thus it follows that, when A¹⁰ is not - CH=CH- then one, and only one, of A⁷, A⁸, A⁹, A¹⁰ and A¹¹ must be S or a trigonal nitrogen. Hence the selection of A⁷, A⁸, A⁹, A¹⁰ and A¹¹ is subject to the following restrictions. If A¹⁰ is not -CH=CH- then one of A⁸, A⁹ and A¹⁰ is NH, N-(CH₂)_{d}-R⁵ or S or one of A⁷ and A¹¹ is N. Not more than one of A⁸, A⁹ and A¹⁰ may be NH, N (CH₂)_{d}-R⁵ or S. A⁷ and A¹¹ may not both simultaneously be N. Neither A⁷ nor A¹¹ may be N if one of A⁸, A⁹ and A¹⁰ is NH, N-(CH₂)_{d}-R⁵ or S.

According to an aspect, particularly preferred compounds are provided in claim 30.

According to an aspect of the present invention, it relates to the compounds of claim 31. It appears that the compounds of formula **1c** of claim 31 are subject to following constraints. The ring constituted by A⁷, A⁸, A⁹, A¹⁰ and A¹¹ is aromatic, and accordingly the groups must satisfy certain requirements. When A¹⁰ is -CH=CH-the ring is a six membered ring. As such, it can only comprise atoms of the type -C(R)= and -N=. Hence A⁷ and A¹¹ must both be C and A⁶ and A⁹ must be either CH or N. When A¹⁰ is not -CH=CH- then the ring is a five-membered ring. In this case one, and only one, of the atoms in the ring must be S or a trigonal nitrogen. In this context, a "trigonal nitrogen" is a nitrogen atom linked covalently to three different atoms. Two of these atoms are the immediate neighbours to the nitrogen atom in the five-membered ring. The third is a hydrogen, carbon or other atom linked to the five-membered ring. Thus it follows that, when A¹⁰ is not - CH=CH- then one (and only one) of A⁷, A⁸, A⁹, A¹⁰ and A¹¹ must be S or a trigonal nitrogen. Hence the selection of A⁷, A⁸, A⁹, A¹⁰ and A¹¹ is subject to the following restrictions. If A¹⁰ is not -CH=CH- then one of A⁸, A⁹ and A¹⁰ is NH, N-(CH₂)_{c}-R⁶ or S or one of A⁷ and A¹¹ is N. Not more than one of A⁸, A⁹ and A¹⁰ may be NH, N-(CH₂)_{c}-R⁶ or S. A⁷ and A¹¹ may not both simultaneously be N. Neither A⁷ nor A¹¹ may be N if one of A⁸, A⁹ and A¹⁰ is NH, N-(CH₂)_{c}-R⁶ or S.

According to an aspect, particularly preferred compounds are provided in claim 45.

According to an aspect of the present invention, it relates to the compounds of claim 46. It appears that the compounds of formula **1d** of claim 46 are subject to the following constraints. The ring constituted by A⁷, A⁸, A⁹, A¹⁰ and A¹¹ is aromatic, and accordingly the groups must satisfy certain requirements. When A¹⁰ is - CH=CH- the ring is a six membered ring. As such, it can only comprise atoms of the type -C(R)= and -N=. Hence A⁷ and A¹¹ must both be C and A⁸ and A⁹ must be either CH or N. When A¹⁰ is not -CH=CH- then the ring is a five-membered ring. In this case one, and only one, of the atoms in the ring must be S or a trigonal nitrogen. In this context, a "trigonal nitrogen" is a nitrogen atom linked covalently to three different atoms. Two of these atoms are the immediate neighbours to the nitrogen atom in the five-membered ring. The third is a hydrogen, carbon or other atom linked to the five-membered ring. Thus it follows that, when A¹⁰ is not - CH=CH- then one (and only one) of A⁷, A⁸, A⁹, A¹⁰ and A¹¹ must be S or a trigonal nitrogen. Hence the selection of A⁷, A⁸, A⁹, A¹⁰ and A¹¹ is subject to the following restrictions. If A¹⁰ is not -CH=CH- then one of A⁸, A⁹ and A¹⁰ is NH, N-(CH₂)_{c}-R¹¹ or S or one of A⁷ and A¹¹ is N. Not more than one of A⁸, A⁹ and A¹⁰ may be NH, N-(CH₂)_{c}-R¹¹ or S. A⁷ and A¹¹ may not both simultaneously be N. Neither A⁷ nor A¹¹ may be N if one of A⁸, A⁹ and A¹⁰ is NH, N- (CH₂)_{c}-R¹¹ or S.

According to an aspect, particularly preferred compounds are provided in claim 69.

The particularly preferred compounds of the invention are V₁ₐ antagonists of high activity.

According to an aspect, at least one of the compounds, identified as follows, is not part of the present invention: claim 1 and ex. 286 (3-Pyridin-3-yl-4,5-dihydro-isoxazole-5-carboxylic acid [4-(4-dimethylamino-benzylcarbamoyl)-phenyl]-amide) of US 6 583 141; ex. 5 and 6 of WO 02/04403 (N-[4-(N-Methyl-N-phenylaminocarbonyl)-phenhylmethyl]-3-(4'-trifluormethylbiphenyl-2-carbonylamino)benzoic acid amide and N-[4-(N-Methyl-N-phenylaminocarbonyl)-phenhylmethyl]-3-(biphenyl-2-carbonylamino)benzoic acid amide); abstract, J. Org. Chem. USSR, vol. 18, no. 6, 1982, p. 1115-1119 (p-(N,N-diethylcarbamoyloxy)-N,N-diethylbenzamide); p. 3, 1. 16 - p. 4, 1. 11, ex. 1-4, 11, and 14-19 of WO 01/29005; p. 3, 1. 9 - p. 5, 1. 23, ex. 11, 28-31, 38, and 71-77 of WO 02/00626; and p. 3, 1. 1 - p. 4, 1. 17, ex. 12-18 of WO 03/016316.

According to an aspect, the invention concerns a pharmaceutical composition comprising a compound according to the invention as an active agent.

The invention is further related to pharmaceutical compositions incorporating a compound according to the invention used as a V₁ₐ antagonist, which compositions are particularly useful for medical indications such as the treatment of dysmenorrhoea (primary dysmenorrhoea and/or secondary dysmenorrhoea), pre-term labour, hypertension, Raynaud's disease, brain oedema, motion sickness, hyperlipemia, small cell lung cancer, depression, anxiety, hyponatremia, liver cirrhosis and congestive heart failure.

Any excipients used will depend on the intended nature of the formulation, which will, in turn, depend on the intended route of administration. Administration may be oral, transmucosal (such as sublingual, buccal, intranasal, vaginal and rectal), transdermal or by injection (such as subcutaneous, intramuscular and intravenous). Oral administration is generally preferred. For oral administration, the formulation may be a tablet, a capsule or a sachet. Other formulations include dry powders, solutions, suspensions, suppositories and the like.

According to an aspect, the invention concerns the use of a compound of the invention for the manufacture of a medicament for the treatment of a disease selected among dysmenorrhoea (primary dysmenorrhoea and/or secondary dysmenorrhoea), pre-term labour, hypertension, Raynaud's disease, brain oedema, motion sickness, hyperlipemia, small cell lung cancer, depression, anxiety, hyponatremia, liver cirrhosis and congestive heart failure.

The compounds according to the present invention may be useful for treatment of several diseases, disorders or conditions. The term "treatment" used herein relates to both treatment in order to cure or alleviate a disease, disorder or a condition, and to treatment in order to prevent the development of a disease, disorder or a condition. The treatment may either be performed in an acute or in a chronic way. The human or animal to be treated, i.e. the patient, may be any human or non-human mammal in need of treatment according to the invention.

The administration of the compositions of the present invention will generally be under the control of a physician. The physician will determine the amount of composition to be administered and the dosing schedule, taking into account the patient's physical condition and the therapeutic goals.

Further aspects of the invention relates to methods of treatment of the above mentioned diseases, disorders or conditions. According to a method according to the invention a therapeutically effective amount of the compound, or of the pharmaceutical composition described above, is administered to a patient in need of this treatment. According to different aspects of the invention, it concerns a method of treatment of a disorder selected among dysmenorrhoea (primary dysmenorrhoea and/or secondary dysmenorrhoea), pre-term labour, hypertension, Raynaud's disease, brain oedema, motion sickness, hyperlipemia, small cell lung cancer, depression, anxiety, hyponatremia, liver cirrhosis and congestive heart failure.

The term "therapeutically effective amount" relates to an amount that will lead to the desired therapeutical effect. The therapeutically effective amount will be determined by the attending physician taking into consideration all appropriate factors. Generally a single dose will comprise between 0.1 mg and 1000 mg, preferably between 1 mg and 250 mg, of the active compound according to the invention. The dose may be given on a single occasion or repeatedly. When given repeatedly, it may be given at regular intervals, such as once, twice or three times daily, or on demand, according to the condition being treated.

The pharmaceutical composition according to the present invention may be presented in any form that is known in the art. For example, the formulation may be presented as a tablet, capsule, powder, suppository, cream, solution or suspension, or in a more complex form such as an adhesive patch. The formulation will generally include one or more excipients, such as diluents, bulking agents, binding agents, dispersants, solvents, preservatives, flavouring agents and the like. The formulation may also include one or more additional phar-macologically active species. Preferably the formulation includes no such additional active agents.

When used as therapeutic agents, the compositions of the present invention may be administered by any appropriate route that is known in the art. For example, they may be administered by the oral, buccal, sublingual, rectal, intra-vaginal, nasal, pulmonary or transdermal routes. Alternatively, they may be given by injection, including intravenous, subcutaneous and intramuscular injection.

The compounds of the present invention can be prepared using standard chemical manipulations. These are described in detail in WO 03/016316 A1, pages 12-17. (Hudson, P. J. et al., "Diazacycloalkanes as Oxytocin Agonists").

The following examples are to be considered as enabling and not limiting for the invention.

### Examples

The following abbreviations are used:
- AIBN: 2,2'-azobisisobutyronitrile
- Boc: carboxylic acid tert-butyl ester or tert- butoxycarbonylamino
- Bu: butyl - alkyl residues may be further denoted as n (normal, i.e. unbranched), i (iso) and t (tertiary)
- DIEA: N,N-diisopropylethylamine
- DMAP: 4-(dimethylamino)pyridine
- DMF: dimethylformamide
- Et: ethyl
- EtOAc: ethyl acetate
- HOBt: 1-hydroxybenzotriazole
- HPLC: High performance liquid chromatography
- H: hour(s)
- Me: methyl
- Min: minute(s)
- MS: mass spectrum
- NMR: nuclear magnetic resonance spectrum - NMR spec- tra were recorded in CDCl₃ at a frequency of 270MHz unless otherwise indicated
- OVA: ornithine vasotocin analogue
- pet.: petroleum ether boiling in the range 60-80°C
- Ether:
- Ph: phenyl
- Pn: pentyl
- Pr: propyl
- THF: tetrahydrofuran
- Tos: toluene-4-sulphonyl
- WSCD: water-soluble carbodiimide (*N*-ethyl-*N*'-(3- dimethylaminopropyl)carbodiimide hydrochloride

Examples E1 - E125 describe the synthesis of intermediates and compounds of the present invention. Example A describes how compounds can be assayed based on their ability to inhibit the cellular consequences of AVP stimulation on intact cells. Example B describes tablets for oral administration comprising a compound according to the invention.

### Example E1

### 6-Chloro-3-methyl-3,4,9,10-tetrahydro-2,3,4,9-tetraazabenzo[f]azulene

### Example E1.1

### 5-(4-Chloro-2-nitro-phenylamino)-1-methyl-1H-pyrazole-4-carboxylic acid ethyl ester.

Sodium hydride (60% dispersion in oil, 7.28g, 180mmol) was added portionwise to a solution of ethyl 5-amino-1-methylpyrazole-4-carboxylate (21.8g, 148mmol) in anhydrous THF at 0°C. The mixture was allowed to warm to room temperature and stirred for 1h. A solution of 4-chloro-2-fluoronitrobenzene (22.6g, 129mmol) in anhydrous THF (50ml) was added dropwise. The resultant deep purple solution was stirred at room temperature for 18h then poured into ice-cold 1N hydrochloric acid. The resulting mixture was extracted with dichloromethane (200ml x 2), and the combined organic extracts were washed with brine and concentrated *in vacuo*. The residue was purified by flash chromatography on silica gel (eluant; 60% pet ether:40% EtOAc) to yield the title compound (27.5g, 62%).

### Example E1.2

### 5-(2-Amino-4-chloro-phenylamino)-1-methyl-1H-pyrazole-4-carboxylic acid ethyl ester.

Zinc powder (26.17g, 400mmol) was added to a suspension of 5-(4-chloro-2-nitro-phenylamino)-1-methyl-1H-pyrazole-4-carboxylic acid ethyl ester from Example E1.1 (26.0g, 80mmol) in methanol/acetic acid (10:1, 330ml) at room temperature. After the exothermic reaction which followed, the resulting suspension was stirred at room temperature for 18h before being filtered through Celite® filter agent. The filtrate was concentrated *in vacuo.* The residue was dissolved in EtOAc, and the solution was washed with saturated NaHCO₃ and brine, and concentrated *in vacuo.* The residue was purified by flash chromatography on silica gel (eluant; 65% EtOAc:35% pet ether to 80% EtOAc:20% pet ether) to yield the title compound (18.41g, 78.0%).

### Example E1.3

### 6-Chloro-3-methyl-4,9-dihydro-3H-2,3,4,9-tetraazabenzo[f]azulen-10-one

Sodium (methylsulphinyl)methanide (53.4mmol) was prepared from sodium hydride (60% dispersion in oil, 2.14g, 53.4mmol) and anhydrous dimethyl sulphoxide (35ml) by heating at 65°C until a solution was observed. To this was added a solution of 5-(2-amino-4-chloro-phenylamino)-1-methyl-1H-pyrazole-4-carboxylic acid ethyl ester from Example E1.2 (9.02g, 30.5mmol) in anhydrous dimethyl sulphoxide (20ml), and stirring continued at 65°C for 30min. The mixture was poured into ice (200ml), and the resulting solid collected and purified by recrystallisation from methanol/EtOAc to yield the title compound (5.56g, 73.3%).

### Example E1.4

### 6-Chloro-3-methyl-3,4,9,10-tetrahydro-2,3,4,9-tetraazabenzo[f]azulene

To a suspension of 6-chloro-3-methyl-4,9-dihydro-3H-2,3,4,9-tetraaza-benzo[f]azulen-10-one from Example E1.3 (5.56g, 22.4 mmol) in anhydrous THF (200ml) at 0°C was added lithium aluminium hydride (4.24g, 112mmol), and the resulting suspension was heated at reflux for 18h, then allowed to cool to room temperature. A further portion of lithium aluminium hydride (4.24g, 112mmol) was added, and the mixture was heated at reflux for 18h. The mixture was cooled to 0°C, 35% ammonia solution (10ml) was added dropwise over 15 min and the mixture was stirred at room temperature for 30min. The resulting suspension was filtered through Celite® filter agent and the filtrate concentrated *in vacuo.* The residue was purified by flash chromatography on silica gel (eluant; 0.5% 35% ammonia:5% methanol:dichloromethane) to yield the title compound (3.88g, 74%).

### Example E2

### 3,6-Dimethyl-3,4,9,10-tetrahydro-2,3,4,9-tetraazabenzo[f]azulene

### Example E2.1

### 1-Methyl-5-(4-methyl-2-nitro-phenylamino)-1H-pyrazole-4-carboxylic acid ethyl ester.

Sodium hydride (60% dispersion in oil, 7.28g, 180mmol) was added portionwise to a solution of ethyl-5-amino-1-methylpyrazole-4-carboxylate (21.8g, 148mmol) in anhydrous THF at 0°C. The mixture was allowed to warm to room temperature and stirred for 1h then a solution of 3-fluoro-4-nitrotoluene (20g, 129mmol) in anhydrous THF (50ml) was added dropwise. The resultant deep purple solution was stirred at room temperature for 18h then poured into ice-cold 1N hydrochloric acid. The resulting mixture was extracted with dichloromethane (200ml x2), and the combined organic extracts were washed with brine and concentrated *in vacuo.* The residue was purified by flash chromatography on silica gel (eluant; 60% pet ether:40% EtOAc) to yield the title compound (28.00g, 61%).

### Example E2.2

### 5-(2-Amino-4-methyl-phenylamino)-1-methyl-1H-pyrazole-4-carboxylic acid ethyl ester

Tin (II) chloride (86.65g, 457.0mmol) was added to a solution of 1-methyl-5-(4-methyl-2-nitro-phenylamino)-1H-pyrazole-4-carboxylic acid ethyl ester from Example E2.1 (28.00g, 91.4mmol) in methanol and the mixture heated at reflux for 3 days. The solvent was removed *in vacuo* and the residue taken up in EtOAc (400ml) and cooled to 0°C. 35% Ammonia solution was added to pH 14, and the mixture was stirred for 15min before being filtered through Celite^{®} filter agent. The filtrate was washed with 2M NH₃ and brine and concentrated *in vacuo.* The residue was purified by flash chromatography on silica gel (eluant; 6% methanol:94% dichloromethane rising to 10% methanol:90% dichloromethane) to yield the title compound (12.8g, 52%).

### Example E2.3

### 3,6-Dimethyl-4,9-dihyd-ro-3H-2,3,4,9-tetraazabenzo[f]azulen-10-one

Sodium (methylsulphinyl)methanide (22.7mmol) was prepared from sodium hydride (60% dispersion in oil, 912mg, 22.7mmol) and anhydrous dimethyl sulphoxide (5.5ml) by heating at 65°C until a solution was observed. To this was added a solution of 5-(2-amino-4-methyl-phenylamino)-1-methyl-1H-pyrazole-4-carboxylic acid ethyl ester from Example E2.2 (3.56g, 13.0mmol) in anhydrous dimethyl sulphoxide (10ml), and stirring continued at 65°C for 30 min. The mixture was then poured into ice (200ml), the resulting solid collected and purified by flash chromatography on silica gel (eluant; 10%methanol:90% dichloromethane) to yield the title compound (1.12g, 38%).

### Example E2.4

### 3,6-Dimethyl-3,4,9,10-tetrahydro-2,3,4,9-tetraazabenzo[f]azulene

To a suspension of 3,6-dimethyl-4,9-dihydro-3H-2,3,4,9-tetraaza-benzo[f]azulen-10-one from Example E2.3 (2.35g, 10.3mmol) in anhydrous THF (100ml) at 0°C was added lithium aluminium hydride (1.56g, 41.2mmol), and the resulting suspension was heated at reflux for 18h then allowed to cool to room temperature. A further portion of lithium aluminium hydride (781 mg, 20.6mmol) was added, and the mixture was heated at reflux for 3h. The mixture was cooled to 0°C, 35% ammonia solution (10ml) was added dropwise over 15 min and the mixture was stirred at room temperature for 30min. The resulting suspension was filtered through Celite^{®} filter agent and the filtrate concentrated *in vacuo.* The residue was purified by flash chromatography on silica gel (eluant; 10% methanol:90% dichloromethane) to yield the title compound (1.60g, 72%).

Examples E2a-g are described in WO 03/016316 A1, (Hudson, P. et al. "Diazacycloalkanes as Oxytocin Agonists"), pages 26-31.

### Example E2h

### 4-(tert-Butoxycarbonylamino-methyl)-3-methyl-benzoic acid

### Example E2h.1

### 4-Cyano-3-methyl-benzoic acid methyl ester

A mixture of 4-cyano-3-methyl-benzoic acid from Example E2d (1.5g, 9.3mmol) and thionyl chloride (5ml, 68.5mmol) in dichloromethane (20ml) was heated at reflux for 2 h then solvents were removed *in vacuo* and azeotroped with toluene. The residue was redissolved in dichloromethane (25ml). Methanol (10ml) was added and the solution was stirred at room temperature for 2h then concentrated *in vacuo.* The residue was redissolved in EtOAc, washed with saturated NaHCO₃ then brine, dried and concentrated *in vacuo* to yield the title compound (1.6g, 100%).

### Example E2h.2

### 4-Aminomethyl-3-methyl-benzoic acid methyl ester

A solution of 4-cyano-3-methyl-benzoic acid methyl ester from Example E2h.1 (1.6g, 9.3mmol) in methanol (50ml) to 0°C was treated with cobalt(II) chloride hexahydrate (5.1g, 18.6mmol). The mixture was stirred for 15min at room temperature then sodium borohydride (3.5g, 93mmol) was added portionwise. The reaction mixture was stirred for 90min then concentrated NH₃ (5ml) was added dropwise. The mixture was warmed up to room temperature over 30min, filtered through Celite^{®} filter agent, washed with methanol and the filtrate was concentrated *in vacuo.* The residue was purified by flash chromatography on silica gel (eluant; 1% 35% ammonia:10% methanol:89% dichloromethane) to yield the title compound (670mg, 37%).

### Example E2h.3

### 4-(tert-Butoxycarbonylamino-methyl)-3-methyl-benzoic acid methyl ester

Di-tert-butyl dicarbonate (900mg, 4.1mmol) was added to a solution of 4-aminomethyl-3-methyl-benzoic acid methyl ester from Example E2h.2 (670mg, 3.7mmol) in dichloromethane (50ml) and triethylamine (to pH9) at room temperature. The mixture was stirred for 1h then concentrated *in vacuo.* The residue was redissolved in EtOAc, washed with 0.3N KHSO₄ then brine, dried and concentrated *in vacuo* to yield the title compound (1.04g, 100%).

### Example E2h.4

### 4-(tert-Butoxycarbonylamino-methyl)-3-methyl-benzoic acid

4-(tert-Butoxycarbonylamino-methyl)-3-methyl-benzoic acid methyl ester from Example E2h.3 (1.04g, 3.7mmol) was dissolved in dioxan (20ml). 1N NaOH solution (5.6ml, 5.6mmol) was added and the mixture was stirred for 18h at room temperature then concentrated *in vacuo.* The residue was redissolved in EtOAc, washed with 1N KHSO₄ then brine, dried and concentrated *in vacuo* to yield the title compound (800mg, 81%).

### Example E3

### 4-(tert-Butoxycarbonylamiao-methyl)-3-fluoro-benzoic acid

### Example E3.1

### 3-Fluoro-4-methylbenzoic acid methyl ester

Thionyl chloride (9.62g, 81mmol) was added to a solution of 3-fluoro-4-methylbenzoic acid (5.0 g, 32.4mmol) in toluene (50ml). The mixture was stirred at room temperature for 1.5h and heated at reflux for 3h. The solvent was removed *in vacuo* and the residue was taken up in methanol (30ml) and CH₂Cl₂ (30ml) and stirred for 18h. The mixture was evaporated *in vacuo* and the residue was taken up in EtOAc (50ml), washed with saturated NaHCO₃ solution (3x75ml), dried and evaporated *in vacuo* to yield the title compound (4.5g, 83%).

### Example E3.2

### 4-Bromomethyl-3-fluorobenzoic acid methyl ester

3-Fluoro-4-methylbenzoic acid methyl ester from Example E3.1 (4.5g, 26.6mmol) was dissolved in carbon tetrachloride (150ml). AIBN (457mg, 2.7mmol) and N-bromosuccinimide (5.2g, 29.3mmol) were added and the mixture was heated at reflux for 18h. The mixture was allowed to cool and further portions of AIBN (457mg, 2.7mmol) and N-bromosuccinimide (5.2g, 29.3mmol) were added. The mixture was heated at reflux for 56h. The mixture was allowed to cool and evaporated *in vacuo.* The residue was purified by flash chromatography on silica gel (eluant; 10% EtOAc:90% pet ether) to yield the title compound (2.7g, 41%).

### Example E3.3

### 4-Azidomethyl-3-fluorobenzoic acid methyl ester

Sodium azide (609mg) was added to a solution of 4-bromomethyl-3-fluorobenzoic acid methyl ester from Example E3.2 (2.1g, 8.5mmol) in DMF (30ml). The mixture was stirred for 18h, diluted with EtOAc, washed with water and brine and concentrated *in vacuo* to give a colourless oil identified as the title compound (1.8 g, 100%).

### Example E3.4

### 4-Aminomethyl-3-fluorobenzoic acid methyl ester

Hydrogen was passed through a degassed solution of 4-azidomethyl-3-fluorobenzoic acid methyl ester from Example E3.3 (2.11g, 10mmol) in methanol containing 10% palladium on carbon for 2h. The reaction mixture was filtered through Celite® filter agent and the filtrate was concentrated *in vacuo* to give a colourless oil identified as the title compound (1.51 g, 83%).

### Example E3.5

### 4-(tert-Butoxycarbonylamino-methyl)-3-fluorobenzoic acid methyl ester

To a solution of 4-aminomethyl-3-fluorobenzoic acid methyl ester from Example E3.4 4 (1.5g, 8.2mmol) in dichloromethane (20ml) were added di-tert-butyl dicarbonate (2.3g, 11mmol) and triethylamine (1.4ml, 10mmol). The mixture was stirred for 18h, washed with 0.3M KHSO₄ and brine and concentrated *in vacuo.* The residue was purified by flash chromatography on silica gel (eluant; 10% EtOAc:90% pet. ether) to give a white solid identified as the title compound (1.4 g, 60%).

### Example E3.6

### 4-(tert-Butoxycarbonylamino-methyl)-3-fluorobenzoic acid

To a solution of 4-(tert-butoxycarbonylamino-methyl)-3-fluorobenzoic acid methyl ester from Example E3.5 (640mg, 2.25mmol) in dioxan (40ml) was added 1N NaOH (4.5ml, 4.5mmol). The mixture was stirred for 18h, diluted with EtOAc, washed with 1N KHSO₄, water and brine and concentrated *in vacuo* to give a white solid identified as the title compound (608 mg, 100%).

### Example E4

### 1-(3,3-Dimethyl-butyl)-piperaz:inedihydrochloride

### Example E4.1

### 4-(3,3-Dimethyl-butyl)-piperazine-1-carboxylic acid tert-butyl ester

To a solution of 1-boc-piperazine (8.19g, 43.9mmol) in methanol/acetic acid (99:1, v/v, 100ml) was added 3,3-dimethylbutyraldehyde (4.0g, 40.0mmol) and the resulting mixture was stirred at room temperature for 1h. Sodium cyanoborohydride (3.27g, 51.9mmol) was added, and the resulting mixture was stirred at room temperature for 18h then concentrated in vacuo. The residue was dissolved in EtOAc and the resulting solution was washed with saturated NaHCO₃, water and brine, dried and concentrated *in vacuo.* The residue was purified by flash chromatography on silica gel (eluant; 5% methanol: 95% EtOAc) to yield the title compound (9.1g, 84%).

### Example E4.2

### 1-(3,3-Dimethyl-butyl)-piperazine dihydrochloride

A solution of 4-(3,3-dimethyl-butyl)-piperazine-1-carboxylic acid tert-butyl ester from Example E4.1 (9.1g, 31.8mmol) in methanol (100ml) was treated with 4N HC1/dioxan (100ml) and the mixture was stirred at room temperature for 30min then concentrated *in vacuo.* The residue was triturated with diethyl ether and the resulting solid recrystallised from methanol/diethyl ether to yield 1-(3,3-dimethyl-butyl)-piperazine dihydrochloride (7.4g, 90%).

### Example E5

### 2-Methyl-1,4,5,6-tetrahydro-1,3,6-triaza-benzo[e]azulene

### Example E5.1

### 2-(Toluene-4-sulfonylamino)-benzoic acid methyl ester

Methyl anthranilate (110g, 0.73mol) was dissolved in dichloromethane (1 litre) at 0°C and triethylamine (115ml, 0.8mol) was added. Tosyl chloride (133g, 0.745mol) was added portionwise at 0°C. The reaction mixture was stirred for 30min at 0°C and 64h at room temperature. The mixture was reduced *in vacuo.* The residue was dissolved in EtOAc and the organic layer was washed with 5% KHCO₃(aq) solution, 1N HCl solution and brine, dried, filtered and concentrated *in vacuo.* The residue was crystallised from EtOAc/hexane to yield the title compound (181g, 81%).

### Example E5.2

### 2-[(3-Ethoxycarbonyl-propyl)-(toluene-4-sulfonyl)-amino]-benzoic acid methyl ester

2-(Toluene-4-sulfonylamino)-benzoic acid methyl ester from Example E5.1 (100g) was dissolved in DMF (250ml). Potassium carbonate (125g) and ethyl 4-bromobutanoate (60g) were added and the mixture was heated at 80°C for 18h. The mixture was cooled to room temperature, filtered and reduced *in vacuo.* The residue was partitioned between chloroform and 1M HCl solution. The aqueous layer was extracted with chloroform. The organic layers were combined, washed with brine, dried, filtered and concentrated *in vacuo.* The material was crystallised from EtOAc/hexane and dried in a vacuum oven at 60°C for 3 hours to yield the title compound (98.6g, 72%).

### Example E5.3

### 5-Hydroxy-1-(toluene-4-sulfonyl)-2,3-dihydro-1H-benzo[b]azepine-4-carboxylic acid methyl ester and 5-hydroxy-1-(toluene-4-sulfonyl)-2,3-dihydro-1H-benzo[b]azepine-4-carboxylic acid ethyl ester

2-[3-Ethoxycarbonyl-propyl)-(toluene-4-sulfonyl)-amino]-benzoic acid methyl ester from Example E5.2 (98.6g, 0.235mol) was taken up in warm toluene (600ml) and was added dropwise to a mixture of potassium tert-butoxide (40g) in toluene (1litre refluxing under Dean-Stark conditions. The mixture was heated at reflux under Dean-Stark condidtions for 1h further and cooled to room temperature. It was diluted with EtOAc (500ml) and washed with 1M HCl solution, saturated NaHCO₃(aq) and brine. The organic layer was dried, filtered and reduced *in vacuo.* The residue was precipitated from EtOAc/hexane and dried in a vacuum oven to yield a mixture of 5-hydroxy-1-(toluene-4-sulfonyl)-2,3-dihydro-1H-benzo[b]azepine-4-carboxylic acid methyl ester and 5-hydroxy-1-(toluene-4-sulfonyl)-2,3-dihydro-1H-benzo[b]azepine-4-carboxylic acid ethyl ester (53.5g).

### Example E5.4

### 1-(Toluene-4-sulfonyl)-1,2,3,4-tetrahydrobenzo[b]azepin-5-one

A mixture of 5-hydroxy-1-(toluene-4-sulfonyl)-2,3-dihydro-1H-benzo[b]azepine-4-carboxylic acid methyl ester and 5-hydroxy-1-(toluene-4-sulfonyl)-2,3-dihydro-1H-benzo[b]azepine-4-carboxylic acid ethyl ester from Example E5.3 were heated at reflux in a mixture of ethanol (100ml), acetic acid (300ml), concentrated HCl (100ml) and water (50ml) for 18h. The mixture was cooled to room temperature, diluted with water (800ml) and extracted with chloroform. The combined organic extracts were dried, filtered and reduced *in vacuo.* The residue was crystallised twice from methanol to yield the title compound (44g, 60% over two steps).

### Example E5.5

### 1,2,3,4-Tetrahydro-benzo[b]azepin-5-one

Polyphosphoric acid (25g) was heated at 100°C under nitrogen until it could be stirred. 1-(Toluene-4-sulfonyl)-1,2,3,4-tetrahydro-benzo[b]azepin-5-one from Example E5.4 (2.6g, 8.26mmol) was added portionwise and the reaction mixture was heated at 100°C for 1.5h. It was poured into ice and basified with 2M NaOH(aq). The aqueous layer was extracted twice with dichloromethane. The organic extracts were combined, washed with brine, dried and reduced *in vacuo.* The residue was purified by flash chromatography on silica gel (eluant; 40% EtOAc: 60% hexane) to yield the title compound (1.05g, 79%).

### Example E5.6

### 1-Benzoyl-1,2,3,4-tetrahydro-benzo[b]azepin-5-one

1,2,3,4-Tetrahydro-benzo[b]azepin-5-one from Example E5.5 (480mg, 2.98mmol) was dissolved in a mixture of dichloromethane (30ml) and triethylamine (1.3ml). Benzoyl chloride (0.46g, 3.28mmol) was added and the reaction mixture was heated for at reflux for 2h. The mixture was cooled and reduced *in vacuo*. The residue was dissolved in EtOAc and washed with 1M KHSO₄(aq), water and brine. The organic layer was dried, filtered and reduced *in vacuo.* The residue was purified by flash chromatography on silica gel (eluant; 50% EtOAc: 50% hexane) to yield the title compound (440mg, 56%).

### Example E5.7

### 1-Benzoyl-4-bromo-1,2,3,4-tetrahydro-benzo[b]azepin-5-one

1-Benzoyl-1,2,3,4-tetrahydro-benzo[b]azepin-5-one from Example E5.6 (54.2g, 205mmol), N-bromosuccinimide (3.6g, 20.4mmol) and bromine (11.1ml, 214.7mmol) were dissolved in dichloromethane (1.0litre). Triethylamine (30ml, 215mmol) was added dropwise over 30 minutes then the reaction mixture was heated at reflux for 4h. Additional bromine (1.1ml, 21.5mmol) and triethylamine (3.0ml, 21.5mmol) were added and the reaction mixture was heated at reflux for a further 4h. Additional bromine (1.1ml, 21.5mmol) and triethylamine (3.0ml, 21.5mmol) were added again and the reaction mixture was heated at reflux for a further 4h. On cooling to room temperature, the reaction solution was washed with 5% aqueous sodium metabisulfate solution (150ml) and the aqueous phase was diluted with water (600ml). The organic phase was separated, washed with saturated NaHCO₃(aq), dried over sodium sulphate and filtered. The filtrate was diluted with EtOAc (100ml), filtered through a silica pad (eluant; CH₂Cl₂) and reduced *in vacuo* to yield the title compound (73.6g) which was used without further purification.

### Example E5.8

6-Benzoyl-2-methyl-1,4,5,6-tetrahydro-1,3,6-triazabenzo[e]azulene

1-Benzoyl-4-bromo-1,2,3,4-tetrahydro-benzo[b]azepin-5-one (67.5g, 200mmol) from Example E5.7, acetamidine hydrochloride (92.7g, 980mmol) and potassium carbonate (136.0g, 980mmol) were suspended in acetonitrile (2.01itres) and heated at reflux for 17h under nitrogen. Additional acetamidine hydrochloride (18.5g, 200mmol) and potassium carbonate (27.7g, 200mmol) were added and the reaction mixture was heated at reflux for a further 6h. Additional acetamidine hydrochloride (18.5g, 200mmol) and potassium carbonate (27.7g, 200mmol) were added again and the reaction mixture was heated at reflux for a further 6h. On cooling to room temperature, the reaction mixture was filtered and the filtrate was concentrated *in vacuo.* The residue was taken up in dichloromethane (1.41itres), washed with water (500ml), dried over sodium sulfate, filtered and reduced *in vacuo.* The crude products were purified by flash chromatography on silica gel (eluant; 45% EtOAc: 45% acetonitrile: 10% methanol) to yield the title compound (26.7g, 34%) and 6-benzoyl-2-methyl-5,6-dihydro-4H-1-oxa-3,6-diaza-benzo[e]azulene (3.3g, 4%).

### Example E5.9

### 2 -Methyl-1,4,5,6-tetrahydro-1,3,6-triazabenzo[e]azulene

6-Benzoyl-2-methyl-1,4,5,6-tetrahydro-1,3,6-triazabenzo[e]azulene from Example E5.8 (160mg, 0.53mmol) was dissolved in a 6M HCl/dioxan solution (50ml) and heated at reflux for 18h. The reaction mixture was cooled to room temperature and reduced *in vacuo.* The residue was partitioned between EtOAc and saturated NaHCO₃(aq) and the layers were separated. The organic layer was washed with brine, dried, filtered and reduced *in vacuo* to yield the title compound (69mg, 66%). **Example E5a**

### 1-Benzyl-2-mothyl-1,4,5,6-tetrahydro-1,3,6-triazabenzo[e]azulene

### Example E5a.1

### (1-Benzyl-2-methyl-4,5-dihydro-1H-1,3,6-triazabenzo[e]azulen-6-yl)-phenyl-methanone

Sodium hydride (60% dispersion in oil, 905mg, 22.5mmol) was placed in anhydrous THF (200ml) and cooled down to 0°C. 6-Benzoyl-2-methyl-1,4,5,6-tetrahydro-1,3,6-triaza-benzo[e]azulene from Example E5.8 (4.9g, 16.lmmol) was added dropwise and the mixture was stirred for 1 h at room temperature. Benzyl bromide (2.31ml, 19.3mmol) and potassium iodide (1.34g, 8.0mmol) were added and the mixture was stirred for 16h. The solution was diluted with EtOAC, washed with saturated NaHCO₃ then brine, dried and concentrated *in vacuo.* The residue was purified by flash chromatography on silica gel (eluant; EtOAc) to yield the title compound (5.73g, 90%).

### Example E5a.2

### 1-Benzyl-2-methyl-1,4,5,6-tetrahydro-1,3,6-triazabenzo[e]azulene

(1-Benzyl-2 -methyl-4,5-dihydro-1H-1,3,6-triazabenzo[e]azulen-6-yl)-phenyl-methanone from Example E5a.l (5.73g, 14.6mmol) was placed in methanol (50ml) and a 6M HCl aqueous solution (200ml) was added. The reaction mixture was heated at reflux for 18h then concentrated *in vacuo.* The residue was dissolved in dichloromethane, basified with saturated NaHCO₃ then washed with brine, dried and concentrated *in vacuo* to yield the title compound (3.30g, 78%).

### Example E6

### 2-Methyl-5,6-dihydro-4H-1-oxa-3,6-diaza-benzo[e]azulene

### 6-Benzoyl-2-methyl-5,6-dihydro-4H-1-oxa-3,6-diazabenzo[e]azulene from Example E5.8 (1.0g, 3.25mmol) was reacted with a 6M HCl/dioxan solution (100ml) using an analogous procedure to that described for Example E5.9 to yield the title compound (540mg, 82%).

### Example E7

### 2-Methyl-5,6-dihydro-4H-3-thia-1,6-diazabenzo[e]azulene

### Example E7.1

### (2-Methyl-4,5-dihydro-3-thia-1,6-diaza-benzo[e]azulen-6-yl)-phenyl-methanone

To a solution of 1-benzoyl-4-bromo-1,2,3,4-tetrahydrobenzo[b]azepin-5-one (1.0g, 2.9mmol) from Example E5.7 in ethanol (50 ml) was added thioacetamide (0.75g, 10mmol). The solution was stirred for 16h. The resultant suspension was reduced in volume by evaporation and cooled. The precipitate was collected by filtration and the solid was washed with cold ethanol and dried to yield the title compound as a white solid (0.65g, 70%).

### Example E7.2

### 2-Methyl-5,6-dihydro-4H-3-thia-1,6-diazabenzo[e]azulene

A suspension of (2-methyl-4,5-dihydro-3-thia-1,6-diazabenzo[e]azulen-6-yl)-phenyl-methanone from Example E7.1 (0.42g, 1.3mmol) in 6M hydrochloric acid (45ml) was heated at reflux for 16h. The solution was cooled and treated with saturated NaHCO₃(aq) (100ml). Additional solid NaHCO₃ was added until the solution was basic. The mixture was extracted with dichloromethane and the organic extracts were dried and reduced *in vacuo* to yield the title compound as a yellow oil (0.21g, 76%).

### Example E9

### 4-(tert-Butoxycarbonylamino-methyl)-cyclohexanecarboxylic acid

To a solution of 4-aminomethyl-cyclohexanecarboxylic acid (20.0g, 127.39mmol) in dioxan (400ml) was added 1N KHCO₃ (300ml, 300mmol) and di-tert-butyl dicarbonate (33.3g, 129.57mmol). The mixture was stirred for 18h and concentrated in vacuo. The aqueous residue was washed with ether, then acidified with 1N KHSO₄ and extracted with EtOAc (x 3). The combined organic extracts were washed with water and brine and concentrated in vacuo to give a white solid identified as the title compound (31.9g, 98%).

### Example E10

### Cyclopropanecarboxylic acid [4-(6-chloro-3-methyl-4,10-dihydro-3H-2,3,4,9-tetraaza-benzo[f]azulene-9-carbonyl)-cyclohexylmethyl]-amide

### Example E10.1

### [4-(6-Chloro-3-methyl-4,10-dihydro-3H-2,3,4,9-tetraazabenzo[f]azulene-9-carbonyl)-cyclohexylmethyl]-carbamic acid tert-butyl ester

A solution of 4-(tert-butoxycarbonylamino-methyl)-cyclohexanecarboxylic acid from Example E9 (510mg, 2.0mmol) in dichloromethane (25 ml) at room temperature was treated with DIEA (0.70ml, 4.0mmol), PyBrop (2.40g, 5.lmmol), and 6-chloro-3-methyl-3,4,9,10-tetrahydro-2,3,4,9-tetraaza-benzo[f]azulene from Example E1 (422mg, 1.8mmol) and the resulting solution was heated at reflux for 20h and allowed to cool to room temperature. The solution was diluted with dichloromethane, washed with brine and concentrated in vacuo. The residue was purified by flash chromatography on silica gel (eluant EtOAc) to yield the title compound (775mg, 91%).

### Example E10.2

### (4-Aminomethyl-cyclohexyl)-(6-chloro-3-methyl-4,10-dihydro-3H-2,3,4,9-tetraaza-benzo[f]azulen-9-yl)-methanone hydrochloride

A solution of [4-(6-chloro-3-methyl-4,10-dihydro-3H-2,3,4,9-tetraaza-benzo[f]azulene-9-carbonyl)-cyclohexylmethyl]-carbamic acid tert-butyl ester from Example E10.1 (775mg, 1.63mmol) was reacted with 4N HCl/dioxan using an analogous procedure to that described for Example E4.2 to yield the title compound (655mg, 100%).

### Example E10.3

### Cyclopropanecarboxylic acid [4-(6-chloro-3-methyl-4,10-dihydro-3H-2,3,4,9-tetraaza-benzo[f]azulene-9-carbonyl)-cyclohexylmethyl]-amide

DIEA (0.50ml, 2.90mmol) and cyclopropanecarbonyl chloride (0.045ml, 0.50mmol) were added to a solution of (4-aminomethyl-cyclohexyl)-(6-chloro-3-methyl-4,10-dihydro-3H-2,3,4,9-tetraaza-benzo[f]azulen-9-yl)-methanone hydrochloride from Example E10.2 (220mg, 0.53mmol) in dichloromethane (5ml). The mixture was stirred for 2h then diluted with dichloromethane, washed with water and concentrated in vacuo. The residue was purified by preparative HPLC (eluant; 0.5% 35% ammonia/10% methanol/90% dichloromethane) to give a white solid identified as the title compound (132 mg, 60%).

### Example E11

### 3-Methyl-3,4,9,10-tetrahydro-2,3,4,9-tetraazabenzo[f]azulene

### Example E11.1

### 1-Methyl-5-(2-nitro-phenylamino)-1H-pyrazole-4-carboxylic acid ethyl ester

Sodium hydride (60% dispersion in oil, 7.0g, 170mmol) was added portionwise to a suspension of 5-amino-1-methyl-1H-pyrazole-4-carboxylic acid ethyl ester (21.1g, 125mmol) in anhydrous THF (300ml) at 0°C. The mixture was allowed to warm to room temperature and stirred for 0.75h then cooled to 0°C. 1-fluoro-2-nitrobenzene (17.6g, 125mmol) was added and the resultant suspension was stirred at room temperature for 18h. EtOAc and 0.3M KHSO4 were added and separated. The aqueous layer was extracted with EtOAc and the combined organic layers were washed with brine, dried and concentrated in vacuo. The residue was purified by flash chromatography on silica gel (eluant 50% hexanes/50% ethyl acetate) to yield the title compound (20.8g, 58%).

### Example E11.2

### 5-(2-Amino-phenylamino)-1-methyl-1H-pyrazole-4-carboxylic acid ethyl ester

1-Methyl-5-(2-nitro-phenylamino)-1H-pyrazole-4-carboxylic acid ethyl ester (20.8g, 72mmol) from Example E11.1 was dissolved in methanol (330ml) and hydrogenated over 10% Pd/C catalyst for 4h. The mixture was filtered through Celite® filter agent and the filtrate was concentrated in vacuo to give a white solid identified as the title compound (16.2g, 87%).

### Example E11.3

### 3-Methyl-4,9-dihydro-3H-2,3,4,9-tetraazabenzo[f]azulen-10-one

Sodium (methylsulphinyl)methanide (29.7mmol) was prepared from sodium hydride (60% dispersion in oil, 1.19g, 29.7mmol) and anhydrous dimethyl sulphoxide (7ml) by heating at 65°C until a solution was observed. To this was added a solution of 5-(2-aminophenylamino)-1-methyl-1H-pyrazole-4-carboxylic acid ethyl ester from Example E11.2 (3.63g, 16.9mmol) in anhydrous dimethyl sulphoxide (10ml), and stirring continued at 65°C for 2.5h. The mixture was poured into ice (100ml), and the resulting solid collected and purified by recrystallisation from methanol/EtOAc/60-80 pet ether to yield the title compound (1.46g, 40%).

### Example E11.4

### 3-Methyl-3,4,9,10-tetrahydro-2,3,4,9-tetraazabenzo[f]azulene

To a suspension of 3-methyl-4,9-dihydro-3H-2,3,4,9-tetraaza-benzo[f]azulen-10-one from Example E11.3 (3.01g, 14.1 mmol) in anhydrous THF (100ml) at 0°C was added lithium aluminium hydride (2.13g, 56.2mmol), and the resulting suspension was heated at reflux for 18h, then allowed to cool to room temperature. The mixture was cooled to 0°C, 35% ammonia solution (10ml) was added dropwise over 15 min and the mixture was stirred at room temperature for 30min. The resulting suspension was filtered through Celite® filter agent and the filtrate concentrated in vacuo. The residue was purified by flash chromatography on silica gel (eluant; 2% methanol:chloroform rising to 5% methanol:chloroform) to yield the title compound (1.60g, 57%).

### Example E12

### [4-(Isobutylamino-methyl)-cyclohexyl]-(3-methyl-4,10-dihydro-3H-2,3,4,9-tetraaza-benzo[f]azulen-9-yl)-methanone

A solution of isobutyraldehyde (0.36mg, 0.005mmol) in 1,2-dichloroethane (0.05ml) was added to a solution of (4-Aminomethyl-cyclohexyl)-(3-methyl-4,10-dihydro-3H-2,3,4,9-tetraaza-benzo[f]azulen-9-yl)-methanone hydrochloride from Example E10.2 (1.88mg, 0.005mmol) in 1,2-dichloroethane (0.05ml) and DIEA (0.0026ml). The mixture was stirred at room temperature for 1h then a solution of sodium triacetoxyborohydride (1.59mg, 0.0075mmol) in DMF (0.05ml) was added. The mixture was stirred for 20h at room temperature then solvents were removed *in vacuo* to yield the title compound. (ESI)+: [M+H]+= 396.4

### Example E13

### [4-(3,6-Dimethyl-4,10-dihydro-3H-2,3,4,9-tetraazabenzo[f]azulene-9-carbonyl)-cyclohexylmethyl]-carbamic acid 4-nitro-benzyl ester

A solution of 4-nitrobenzyl chloroformate (1.08mg, 0.005mmol) in dichloromethane (0.05ml) was added to a solution of (4-Aminomethyl-cyclohexyl)-(3,6-dimethyl-4,10-dihydro-3H-2,3,4,9-tetraaza-benzo[f]azulen-9-yl)-methanone hydrochloride (Compound number 149) (1.95mg, 0.005mmol) in dichloromethane (0.05ml) and triethylamine (0.0035ml). The mixture was stirred at room temperature for 1h then solvents were removed *in vacuo* to yield the title compound. (ESI)+: [M+H]+= 533.3

### Example E14

### (6-Chloro-3-methyl-4,10-dihydro-3H-2,3,4,9-tetraazabenzo[f]azulen-9-yl)-(4-hexylaminomethyl-cyclohexyl)-methanone

A solution of 1-bromohexane (0.83mg, 0.005mmol) in DMF(0.05ml) was added to a solution of (4-Aminomethylcyclohexyl)-(6-chloro-3-methyl-4,10-dihydro-3H-2,3,4,9-tetraaza-benzo[f]azulen-9-yl)-methanone hydrochloride from Example E10.2 (2.05mg, 0.005mmol) in DMF (0.05ml) and triethylamine (0.0021ml). The mixture was stirred for 20h at room temperature then solvents were removed *in vacuo* to yield the title compound. (ESI)+: [M+H]+= 458.4

### Example E15

### 1-(3,3-Dimethyl-butyl)-piperidine-4-carboxylic acid

### Example E15.1

### 1-(3,3-Dimethyl-butyl)-piperidine-4-carboxylic acid ethyl ester

3,3-Dimethylbutyraldehyde (5.26ml, 42.0mmol) was added to a solution of ethyl isonipecotate (6.6g, 42.0mmol) in methanol/acetic acid (99:1, v/v, 50ml) and the mixture was stirred at room temperature for 1h. Sodium cyanoborohydride (3.43g, 54.6mmol) was added, and the mixture was stirred at room temperature for 4 days then concentrated *in vacuo*. The residue was purified by flash chromatography on silica gel (eluant; 25% EtOAc:75% cyclohexane) to yield the title compound (7.16g, 710).

### Example E15.2

### 1-(3,3-Dimethyl-butyl)-piperidine-4-carboxylic acid

Lithium hydroxide monohydrate (1.37g, 32.6mmol) was added to a solution of 1-(3,3-dimethyl-butyl)-piperidine-4-carboxylic acid ethyl ester from Example E15.1 (7.16g, 29.7mmol) in THF (50ml) and water (5ml). The mixture was stirred at room temperature for 18h then concentrated *in vacuo*. The residue was purified by flash chromatography on silica gel through an isolute (eluant; 90% dichloromethane:9% methanol:1% acetic acid) to yield the title compound (5.51g, 87%).

### Example E15a

### 1-Cyclopropylmethyl-piperidine-4-carboxylic acid

The title compound was prepared from cyclopropanecarboxaldehyde and ethyl isonipecotate using similar procedures to those described for Example E15.

### Example E16

**1-(3,3-Dimethyl-butyl)-piperidine-4-carboxylic acid [4-(3-methyl-4,10-dihydro-3H-2,3,4,9-tetraazabenzo[f]azulene-9-carbonyl)-cyclohexylmethyl]-amide**

### Example E16.1

### 4-(3,3-Dimethyl-butyl)-piperazine-4-carboxylic acid [4-(3-methyl-4,10-dihydro-3H-2,3,4,9-tetraazabenzo[f]azulene-9-carbonyl)-cyclohexylmethyl]-amide

HBTU (84mg, 0.22mmol) was added to a solution of 1-(3,3-dimethyl-butyl)-piperidine-4-carboxylic acid from Example E15 (42mg, 0.17mmol) in DMF (5ml) and DIEA (to pH9). The mixture was stirred at room temperature for 1 h. (4-Aminomethyl-cyclohexyl)-(3-methyl-4,10-dihydro-3H-2,3,4,9-tetraaza-benzo[f]azulen-9-yl)-methanone hydrochloride (Compound Number 150) (42mg, 0.11mmol) was added and the mixture was stirred at room temperature for 18h. Solvents were removed *in vacuo* and azeotroped with toluene. The residue was redissolved in EtOAc, washed with saturated NaHCO₃ then brine, dried and concentrated *in vacuo*. The residue was purified by preparative HPLC (eluant; 10% methanol:90% dichloromethane) to give a white solid identified as the title compound (34mg, 57%).

### Example E17

### N-[4-(3,6-Dimethyl-4,10-dihydro-3H-2,3,4,9-tetraazabenzo[f]azulene-9-carbonyl)-cyclohexylmethyl]-2-piperidin-4-yl-acetamide hydrochloride

A solution of 4-({[4-(3,6-dimethyl-4,10-dihydro-3H-2,3,4,9-tetraaza-benzo[f]azulene-9-carbonyl)-cyclohexylmethyl]-carbamoyl}-methyl)-piperidine-1-carboxylic acid tert-butyl ester (Compound Number 228)(259mg, 0.45mmol) was reacted with 4N HCl/dioxan using an analogous procedure to that described for Example E4.2 to yield the title compound (230mg, 93%).

### Example E18

### N-[4-(3,6-Dimethyl-4,10-dihydro-3H-2,3,4,9-tetraazabenzo[f]azulene-9-carbonyl)-cyclohexylmethyl]-methanesulfonamide

A solution of methanesulfonyl chloride (0.57mg, 0.005mmol) in dichloromethane (0.05ml) was added to a solution of (4-aminomethyl-cyclohexyl)-(3,6-dimethyl-4,10-dihydro-3H-2,3,4,9-tetraaza-benzo[f]azulen-9-yl)-methanone (Compound number 149) (1.95mg, 0.005mmol) in dichloromethane (0.05ml) and triethylamine (0.0035ml). The mixture was stirred at room temperature for 1h then solvents were removed *in vacuo* to yield the title compound. (ESI)+: [M+H]+= 432.1

### Example E19

### N-[4-(3,6-Dimethyl-4,10-dihydro-3H-2,3,4,9-tetraazabenzo[f]azulene-9-carbonyl)-cyclohexylmethyl]-2-(1-methanesulfonyl-piperidin-4-yl)-acetamide

A solution of methanesulfonyl chloride (0.57mg, 0.005mmol) in dichloromethane (0.05ml) was added to a solution of N-[4-(3,6-dimethyl-4,10-dihydro-3H-2,3,4,9-tetraaza-benzo[f]azulene-9-carbonyl)-cyclohexylmethyl]-2-piperidin-4-yl-acetamide hydrochloride from Example E17 (2.57mg, 0.005mmol) in dichloromethane (0.05ml) and triethylamine (0.0035ml). The mixture was stirred at room temperature for 1h then solvents were removed *in vacuo* to yield the title compound. (ESI)+: [M+H]+= 557.4

### Example E20

### 2-(1-Acetyl-piperidin-4-yl)-N-[4-(3,6-dimethyl-4,10-dihydro-3H-2,3,4,9-tetraaza-benzo[f]azulene-9-carbonyl)-cyclohexylmethyl]-acetamide

A solution of acetyl chloride (0.39mg, 0.005mmol) in dichloromethane (0.05ml) was added to a solution of N-[4-(3,6-dimethyl-4,10-dihydro-3H-2,3,4,9-tetraazabenzo[f]azulene-9-carbonyl)-cyclohexylmethyl]-2-piperidin-4-yl-acetamide hydrochloride from Example E17 (2.57mg, 0.005mmol) in dichloromethane (0.05ml) and triethylamine (0.0035ml). The mixture was stirred at room temperature for 1h then solvents were removed *in vacuo* to yield the title compound. (ESI)+: [M+H]+= 521.5

### Example E21

### 1-Isobutyl-piperidine-4-carboxylic acid [4-(3-methyl-4,10-dihydro-3H-2,3,4,9-tetraaza-benzo[f]azulene-9-carbonyl)-cyclohexylmethyl]-amide

A solution of isobutyraldehyde (0.36mg, 0.005mmol) in 1,2-dichloroethane (0.05ml) was added to a solution of piperidine-4-carboxylic acid [4-(3-methyl-4,10-dihydro-3H-2,3,4,9-tetraaza-benzo[f]azulene-9-carbonyl)-cyclohexylmethyl]-amide hydrochloride (Compound number 238) (2.43mg, 0.005mmol) in 1,2-dichloroethane (0.05ml) and DIEA (0.0026ml). The mixture was stirred at room temperature for 1h then a solution of sodium triacetoxyborohydride (1.59mg, 0.0075mmol) in DMF (0.05ml) was added. The mixture was stirred for 20h at room temperature then solvents were removed *in vacuo* to yield the title compound. (ESI)+: [M+H]+= 507.4

### Example E22

### N-[4-(6-Chloro-3-methyl-4,10-dihydro-3H-2,3,4,9-tetraaza-benzo[f]azulene-9-carbonyl)-cyclohexylmethyl]-3-(1-hexyl-piperidin-4-yl)-propionamide

A solution of 1-bromohexane (0.83mg, 0.005mmol) in DMF(0.05ml) was added to a solution of N-[4-(6-chloro-3-methyl-4,10-dihydro-3H-2,3,4,9-tetraazabenzo[f]azulene-9-carbonyl)-cyclohexylmethyl]-3-piperidin-4-yl-propionamide hydrochloride (Compound number 237) (2.57mg, 0.005mmol) in DMF (0.05ml) and triethylamine (0.0021ml). The mixture was stirred for 20h at room temperature then solvents were removed *in vacuo* to yield the title compound. (ESI)+: [M+H]+= 597.6/599.6

### Example E23

### 4-({[4-(3,6-Dimethyl-4,10-dihydro-3H-2,3,4,9-tetraazabenzo[f]azulene-9-carbonyl)-cyclohexylmethyl]-carbamoyl}-methyl)-piperidine-1-carboxylic acid 4-nitro-benzyl ester

A solution of 4-nitrobenzyl chloroformate (1.08mg, 0.005mmol) in dichloromethane (0.05ml) was added to a solution of N-[4-(3,6-Dimethyl-4,10-dihydro-3H-2,3,4,9-tetraaza-benzo[f]azulene-9-carbonyl)-cyclohexylmethyl]-2-piperidin-4-yl-acetamide hydrochloride from Example E17 (2.58mg, 0.005mmol) in dichloromethane (0.05ml) and triethylamine (0.0035ml). The mixture was stirred at room temperature for 1h then solvents were removed *in vacuo* to yield the title compound. (ESI)+: [M+H]+= 658.5

### Example E24

### 4-(3,3-Dimethyl-butyl)-piperazine-1-carboxylia acid [4-(3,6-dimethyl-4,10-dihydro-3H-2,3,4,9-tetraazabenzo[f]azulene-9-carbonyl)-cyclohexylmethyl]-amide

DIEA (0.30ml, 1.7mmol) and 1,1'-carbonyldiimidazole (39mg, 0.24mmol) were added to a solution of (4-aminomethyl-cyclohexyl)-(3,6-dimethyl-4,10-dihydro-3H-2,3,4,9-tetraaza-benzo[f]azulen-9-yl)-methanone hydrochloride (Compound number 149) (78mg, 0.20mmol) in DMF (4.0ml) and the mixture was stirred at room temperature for 2h. 1-(3,3-Dimethyl-butyl)-piperazine dihydrochloride from Example E4 (56mg, 0.23mmol) was added and the mixture was stirred at room temperature for 18h. The mixture was diluted with EtOAc, washed with brine, dried and concentrated in vacuo. The residue was purified by preparative HPLC (eluant; 10% methanol:90% dichloromethane) to give a white solid identified as the title compound (32mg, 29%).

### Example E25

### 3-Chloro-6,7,8,9-tetrahydro-5-oxa-9-azabenzocycloheptene

### Example E25.1

### N-(4-Chloro-2-hydroxy-phenyl)-benzamide

2-Amino-5-chlorophenol (1.45g, 10mmol) was dissolved in EtOAc (30ml) and water (20ml). Sodium bicarbonate (1.25g, 15mmol) then benzoyl chloride (1.42g, 10mmol) were added and the mixture was stirred at room temperature for 1h. The layers were partitioned and the organic layer was washed with brine, dried and concentrated *in vacuo*. The residue was triturated with diethyl ether to yield the title compound (2.05g, 82%).

### Example E25.2

### (3-Chloro-7,8-dihydro-6H-5-oxa-9-aza-benzocyclohepten-9-yl)-phenyl-methanone

N-(4-Chloro-2-hydroxy-phenyl)-benzamide from Example E25.1 (1.0g, 4mmol) was dissolved in acetonitrile (10ml) and dichloromethane (15ml). 1,3-Dibromopropane (3.26g, 16mmol), aliquat 336 (170mg, 0.4mmol) and sodium hydroxide (750mg, 16mmol) were added and the mixture was heated at 60°C for 3h. The solid was filtered off and the filtrate concentrated *in vacuo*. The residue was purified by flash chromatography on silica gel (eluant; 30% EtOAc:70% hexane) to yield the title compound (783mg, 83%).

### Example E25.3

### 3-Chloro-6,7,8,9-tetrahydro-5-oxa-9-azabenzocycloheptene

(3-Chloro-7,8-dihydro-6H-5-oxa-9-aza-benzocyclohepten-9-yl)-phenyl-methanone from Example E25.2 (783mg, 2.7mmol) was dissolved in dioxan (10ml) and 6M HCl aqueous solution (50ml) was added. The mixture was heated at reflux for 20h then concentrated *in vacuo* and azeotroped with toluene. The residue was dissolved in diethyl ether and water, basified with NaHCO₃ and the layers were partitioned. The organic layer was washed with brine, dried and concentrated *in vacuo* to yield the title compound (280mg, 47%).

### Example E26

### N-Cyclohexyl-N'-methyl-benzene-1,2-diamine

### Example E26.1

### Cyclohexyl-(2-nitro-phenyl)-amine

A mixture of 2-fluoronitrobenzene (5.0g, 35.4mmol), cyclohexylamine (4.5ml, 39.0mmol) and potassium carbonate (17.1g, 124mmol) in acetonitrile (100ml) was heated at reflux for 2 days. The mixture was diluted with EtOAC, washed with water then brine, dried and partially concentrated *in vacuo*. The solid which precipitated was collected and washed with hexane to yield the title compound (6.7g, 86%).

### Example E26.2

### N-Cyclohexyl-benzene-1,2-diamine

Cyclohexyl-(2-nitro-phenyl)-amine from Example E26.1 (3.0g, 13.6mmol) was dissolved in methanol (100ml) and tin (II) chloride (12.9g, 68.1mmol) was added. The mixture was stirred for 20h at room temperature then heated at reflux for 18h and concentrated *in vacuo*. The residue was placed in EtOAC (100ml), cooled down to 0°C and pH was adjusted to 14 with conc. NH₃. The precipitate was filtered off and washed with EtOAc. The filtrate was washed with 2M NH₃, water then brine, dried and concentrated *in vacuo* to yield the title compound (2.25g, 86%).

### Example E26.3

### N-Cyclohexyl-N'-methyl-benzene-1,2-diamine

Potassium carbonate (2.45g, 17.7mmol) and iodomethane (0.81ml, 13.0mmol) were added to a solution of N-cyclohexyl-benzene-1,2-diamine from Example E26.2 (2.25g, 11.8mmol) in DMF (10ml). The mixture was stirred for 6h at room temperature then poured into water and extracted with EtOAc. The organic layer was washed with water then brine, dried and concentrated *in vacuo*. The residue was purified by flash chromatography on silica gel (eluant; 5% EtOAc:95% hexane) to yield the title compound (950mg, 39%).

### Example E27

### 3-Fluoro-4-hydroxy-benzoic acid ethyl ester

A solution of 3-fluoro-4-hydroxy-benzoic acid (5.16g, 33.1mmol) in ethanol (100ml) was treated with conc. sulphuric acid (5ml), and the mixture heated at reflux for 4 days. The volatiles were removed *in vacuo*, and the aqueous residue was basified with saturated NaHCO₃ and extracted twice with diethyl ether. The combined organic extracts were dried and concentrated *in vacuo* to yield the title compound (5.16g, 85%).

### Example E28

### 2-Chloro-4-hydroxy-benzoic acid ethyl ester

A solution of 2-chloro-4-hydroxy-benzonitrile (5.0g, 32.6mmol) in ethanol (125ml) was treated with conc. sulphuric acid (25ml) and the mixture heated at reflux for 3 days. Conc. sulphuric acid (25ml) was added and the mixture was heated at reflux for another 2 days. The volatiles were removed *in vacuo*, and the aqueous residue was basified with saturated NaHCO₃ and extracted 4 times with diethyl ether. The combined organic extracts were dried and concentrated *in vacuo* to yield the title compound (2.5g, 38%).

### Example E29

### 4-Hydroxy-3-methyl-benzoic acid methyl ester

### 4-Amino-3-methyl-benzoic acid methyl ester (5.25g, 32.0mmol) was treated with a 35% solution of sulphuric acid (50ml) and the mixture was stirred and heated until dissolution then cooled to 0°C. Sodium nitrite (2.82g, 41.6mmol) in water (50ml) was added dropwise and the mixture was stirred for 5min at 0°C. Urea was added to destroy the excess nitrite. Copper nitrate (121g, 320mmol) in water (11) was added then copper oxide (4.25g, 32.0mmol). The mixture was warmed up to room temperature over 30 min and extracted with EtOAc (x 3). The organics were combined, washed with brine, dried and concentrated in vacuo. The residue was purified by flash chromatography on silica gel (eluant; 30% EtOAc:70% hexane) to yield the title compound (2.2g, 42%).

### Example E30

**4-(3-Carboxy-propyl)-3-methyl-benzoic acid methyl ester**

### Example E30.1

### 4-((E)-2-tert-Butoxycarbonyl-vinyl)-3-methyl-benzoic acid methyl ester

Tetrakis(triphenylphosphine)palladium (0) (5.0g, 4.33mmol) was added to a stirred solution of methyl 4-bromo-3-methylbenzoate (9.93g, 43.3mmol), tert-butyl acrylate (50ml, 341.3mmol) and sodium acetate (35.8g, 436.4mmol) in DMA (350ml). The mixture was heated at 140°C for 5h, filtered through Celite® filter agent and the filtrate was concentrated *in vacuo*. The residue was redissolved in EtOAc, washed with 0.3M KHSO₄ then brine, dried and concentrated *in vacuo*. The residue was purified by flash chromatography on silica gel (eluant; 15% EtOAc:85% hexane to 20% EtOAc:80% hexane) to yield the title compound (4.81g, 40%).

### Example E30.2

### 4-(2-tert-Butoxycarbonyl-ethyl)-3-methyl-benzoic acid methyl ester

4-((E)-2-tert-Butoxycarbonyl-vinyl)-3-methyl-benzoic acid methyl ester from Example E30.1 (4.00g, 14.5mmol) was dissolved in methanol (100ml) and hydrogenated over 10% Pd/C catalyst (480mg) for 5h. The mixture was filtered through Celite® filter agent and the filtrate was concentrated *in vacuo* to yield the title compound (3.41g, 84%).

### Example E30.3

### 4-(2-Carboxy-ethyl)-3-methyl-benzoic acid methyl ester

Trifluoroacetic acid (40ml) was added slowly to a stirred solution of 4-(2-tert-butoxycarbonyl-ethyl)-3-methyl-benzoic acid methyl ester from Example E30.2 (4.9g, 17.6mmol) in dichloromethane (80ml) at room temperature. The mixture was stirred for 2h then concentrated *in vacuo* and azeotroped with dichloromethane. The residue was recrystallised in EtOAc to yield the title compound (2.77g, 71%).

### Example E30.4

### 4-(3-Carboxy-propyl)-3-methyl-benzoic acid methyl ester

4-(2-Carboxy-ethyl)-3-methyl-benzoic acid methyl ester from Example E30.3 (500mg, 2.25mmol) was dissolved in dry dichloromethane (10ml) and a few drops of DMF. Oxalyl chloride (0.393ml, 4.5mmol) was added dropwise and the mixture was stirred for 1h at room temperature. Solvents were removed *in vacuo* and azeotroped with toluene. The residue was redissolved in acetonitrile (20ml) and cooled to 0°C. A solution of 2M (trimethylsilyl)diazomethane in hexanes (2.25ml, 4.5mmol) was added dropwise and the reaction mixture was stirred for 5h at 0°C then 20h at room temperature. Ethyl acetate then 10% citric acid solution were added and the layers were partitioned. The organic layer was washed with saturated NaHCO₃ then brine, dried and concentrated *in vacuo*. The residue was purified by flash chromatography on silica gel (eluant; 50% ethyl acetate:50% cyclohexane). The product obtained was dissolved in a mixture of acetonitrile and water. A solution of silver benzoate (103mg, 0.45mmol) in triethylamine (1.25ml, 9mmol) was added gradually while the mixture was sonicated in an ultrasound bath. After 30min at room temperature, solvents were removed *in vacuo*. Ethyl acetate and 10% aqueous citric acid solution were added and the layers were partitioned. The organic layer was washed with brine, dried and concentrated *in vacuo*. The residue was purified by flash chromatography on silica gel (eluant; 50% EtOAc:50% cyclohexane) to yield the title compound (378mg, 71%).

### Example E31

### 4-(4-Methoxycarbonyl-butyl)-3-methyl-benzoic acid

### Example E31.1

### 4-Bromo-3-methyl-benzoic acid tert-butyl ester

### 4-Bromo-3-methyl-benzoic acid (2.06g, 9.6mmol) and thionyl chloride (2.2ml, 30.2mmol) in toluene (50ml) were heated at reflux for 2h then concentrated in vacuo and azeotroped with toluene. The residue was dissolved in THF (100ml) and triethylamine (2.8ml, 20.1mmol), cooled down to 0°C and lithium tert-butoxide (1.24g, 15.5mmol) was added portionwise. The mixture was stirred for 18h at room temperature then concentrated in vacuo. The residue was dissolved in EtOAc, washed with 1M HCl, saturated NaHCO₃ then brine, dried and concentrated in vacuo. The residue was purified by flash chromatography on silica gel (eluant; 5% EtOAc:95% pet. ether) to yield the title compound (1.86g, 71%).

### Example E31.2

### 4-((1E,3E)-4-Methoxycarbonyl-buta-1,3-dienyl)-3-methylbenzoic acid tert-butyl ester

Tetrakis(triphenylphosphine)palladium (0) (852mg, 0.7mol) was added to a stirred solution of 4-bromo-3-methyl-benzoic acid tert-butyl ester from Example E31.1 (1.86g, 6.8mmol), 1-acetoxy-1,3-butadiene (7.5ml, 64.5mmol) and sodium acetate (5.69g, 69.4mmol) in DMA (75ml). The mixture was heated at 140°C for 3h, filtered through Celite® filter agent and the filtrate was concentrated *in vacuo*. The residue was purified by flash chromatography on silica gel (eluant; 20% EtOAc:80% pet. ether) to yield the title compound (1.54g, 74%).

### Example E31.3

### 4-(4-Methoxycarbonyl-butyl)-3-methyl-benzoic acid tert-butyl ester

4-((1E,3E)-4-Methoxycarbonyl-buta-1,3-dienyl)-3-methylbenzoic acid tert-butyl ester from Example E31.2 (1.54g, 5.1mmol) was dissolved in methanol (40ml) and hydrogenated over 10% Pd/C catalyst (200mg) for 5h. The mixture was filtered through Celite® filter agent and the filtrate was concentrated *in vacuo* to yield the title compound (1.43g, 92%).

### Example E31.4

### 4-(4-Methoxycarbonyl-butyl)-3-methyl-benzoic acid

Trifluoroacetic acid (20ml) was added to a solution of 4-(4-methoxycarbonyl-butyl)-3-methyl-benzoic acid tert-butyl ester from Example E31.3 (1.43g, 4.7mmol) in dichloromethane (40ml) and the mixture was stirred for 2h at room temperature. Solvents were concentrated *in vacuo* and azeotroped with dichloromethane to yield the title compound (1.07g, 92%).

### Example E32

### 4-Carboxymethyl-3-methyl-benzoic acid methyl ester

### Example E32.1

### 4-tert-Butoxycarbonylmethyl-3-methyl-benzoic acid methyl ester

Copper (II) fluoride (4.08g, 40.1mmol) and bis-[tri-(o-tolyl)phosphine]palladium dichloride (473mg, 0.6mmol) were added to a solution of methyl 4-bromo-3-methyl benzoate (4.60g, 20.1mmol) in THF (30ml). The mixture was heated at reflux before silyl ketene acetal (18.5g, 80.3mmol) was added. The mixture was heated at reflux for 2 days then diluted with Et₂O. Aqueous NH₄Cl solution (250ml) was added and the mixture was stirred for 30min at room temperature. The layers were partitioned and the aqueous layer extracted twice with Et₂O. The organics were combined, dried and concentrated *in vacuo*. The residue was purified by flash chromatography on silica gel (eluant; 5% EtOAc:95% hexane) to yield the title compound (2.84g, 53%).

### Example E32.2

### 4-Carboxymethyl-3-methyl-benzoic acid methyl ester

Trifluoroacetic acid (15ml) was added to a solution of 4-tert-butoxycarbonylmethyl-3-methyl-benzoic acid methyl ester from Example E32.1 (2.84g, 10.7mmol) in dichloromethane (15ml). The mixture was stirred for 90min at room temperature, concentrated *in vacuo* and azeotroped with toluene. The residue was recrystallised from EtOAc and hexane to yield the title compound (1.81g, 81%).

### Example E33

### 4-[1,3]Dioxolan-2-yl-piperidine

### Example E33.1

### 4-Hydroxymethyl-piperidine-1-carboxylic acid benzyl ester

4-Piperidinemethanol (5.0g, 43mmol) was dissolved in dichloromethane (100ml) and triethylamine (12ml, 86mmol) at 0°C. Benzylchloroformate (6.8ml, 47.3mmol) was added and the mixture was stirred for 20h at room temperature then solvents were removed *in vacuo*. The residue was redissolved in EtOAc, washed with 1M KHSO₄, water then brine, dried and concentrated *in vacuo* to yield the title compound (8.33g, 77%).

### Example E33.2

### 4-Formyl-piperidine-1-carboxylic acid benzyl ester

Dess-Martin reagent (17g, 39.6mmol) was added portionwise to a solution of 4-hydroxymethyl-piperidine-1-carboxylic acid benzyl ester from Example E33.1 (8.33g, 33mmol) in dichloromethane (100ml) at room temperature. The mixture was stirred for 3h under an inert atmosphere then diluted with chloroform and water and the layers were partitioned. The organic layer was washed with brine, dried and concentrated *in vacuo*. The residue was purified by flash chromatography on silica gel (eluant; 40% EtOAc:60% hexane) to yield the title compound (5.5g, 66%).

### Example E33.3

### 4-[1,3]Dioxolan-2-yl-piperidine-1-carboxylic acid benzyl ester

Ethylene glycol (5ml) and a catalytic amount of p-toluenesulfonic acid were added to 4-formyl-piperidine-1-carboxylic acid benzyl ester from Example E33.2 (5.6g, 22.6mmol) in toluene (100ml) and the mixture was heated at reflux under Dean and Stark conditions for 2.5h. Solvents were removed *in vacuo* and the residue was redissolved in EtOAc, washed with saturated NaHCO₃ then brine, dried and concentrated *in vacuo*. The residue was purified by flash chromatography on silica gel (eluant; 50% EtOAc:50% pet. ether) to yield the title compound (5.14g, 78%).

### Example E33.4

### 4-[1,3]Dioxolan-2-yl-piperidine

4-[1,3]Dioxolan-2-yl-piperidine-1-carboxylic acid benzyl ester from Example E33.3 (5.14g, 17.7mmol) was dissolved in methanol (100ml) and hydrogenated over 10% Pd/C catalyst (551mg) for 4h. The mixture was filtered through Celite® filter agent and the filtrate was concentrated *in vacuo* to yield the title compound (2.84g, 100%).

### Example E34

### 4-(3-Hydroxy-propyl)-piperazine-1-carboxylic acid tert-butyl ester

A solution of piperazine-1-carboxylic acid tert-butyl ester (26.5g, 142mmol) in acetone (300ml) at room temperature was treated with 3-bromo-propan-1-ol (14.5ml, 156.2mmol), potassium carbonate (50g, 361.8mmol) and potassium iodide (2.4g, 14.2mmol), and the mixture was heated at reflux for 18h then allowed to cool to room temperature. The suspension was filtered and the filtrate concentrated *in vacuo*. The residue was purified by flash chromatography on silica gel (eluant; 5% methanol:95% dichloromethane) to yield the title compound (27.7g, 80%).

### Example E35

### 1-Cyclopropylmethyl-imidazolidine ditrifluoroacetate

### Example E35.1

### [2-(Cyclopropylmethyl-amino)-ethyl]-carbamic acid tert-butyl ester

Potassium hydrogen carbonate (220mg, 2.2mmol) and (bromoethyl)cyclopropane (270mg, 2.0mmol) were added to a solution of tert-butyl-N-(2-aminoethyl)carbamate (320mg, 2.0mmol) in THF (10ml). The mixture was heated at 66°C for 20h and solvents were concentrated *in vacuo*. The residue was dissolved in chloroform, washed with water, dried and concentrated *in vacuo*. The residue was purified by flash chromatography on silica gel (eluant; 1% triethylamine:4% methanol:95% chloroform) to yield the title compound (130mg, 30%).

### Example E35.2

### 1-Cyclopropylmethyl-imidazolidine ditrifluoroacetate

Trifluoroacetic acid (5ml) was added to a solution of [2-(cyclopropylmethyl-amino)-ethyl]-carbamic acid tert-butyl ester from Example E35.1 (180mg, 0.84mmol) in dichloromethane (3ml) and the mixture was stirred for 75min at room temperature. Solvents were removed *in vacuo* and azeotroped with toluene. The residue was placed in water (10ml) and formaldehyde (37% w/w solution, 0.10ml, 1.36mmol) was added. The mixture was stirred for 6 days at room temperature, concentrated *in vacuo*, azeotroped with toluene then pet. ether to yield the title compound (255mg, 86%).

### Example E36

### 2-Piperazin-1-yl-1-p-tolyl-ethanone dihydrochloride

### Example E36.1

### 4-(2-oxo-2-p-tolyl-ethyl)-piperazine-1-carboxylic acid tert-butyl ester

2-Bromo-4-methylacetophenone (572mg, 2.68mmol) was added to a solution of piperazine-1-carboxylic acid tert-butyl ester (500mg, 2.68mmol) in dichloromethane (5ml) and triethylamine (0.45ml, 3.22mmol). The mixture was stirred for 3 days at room temperature and solvents were removed *in vacuo*. The residue was purified by flash chromatography on silica gel (eluant; 40% hexane:60% EtOAc) to yield the title compound (661mg, 77%).

### Example E36.2

### 2-Piperazin-1-yl-1-p-tolyl-ethanone dihydrochloride

4-(2-Oxo-2-p-tolyl-ethyl)-piperazine-1-carboxylic acid tert-butyl ester from Example E36.1 (661mg, 2.08mmol) was dissolved in 4M HCl solution in dioxan (25ml) at 0°C and the mixture was stirred for 45min at room temperature. Solvents were concentrated *in vacuo* and azeotroped with diethyl ether to yield the title compound (536mg, 88%).

### Example E37

### 2-Bromo-1-[4-(3,3-dimethyl-butyl)-piperazin-1-yl]-methanone

Bromoacetyl bromide (0.52ml, 6.0mmol) was added to a solution of 1-(3,3-dimethyl-butyl)-piperazine hydrochloride from Example E4 (1.5g, 5.7mmol) in dichloromethane (20ml) and triethylamine (3.57ml, 25.6mmol) at 0°C. The mixture was stirred for 20h at room temperature, washed with saturated NaHCO₃, dried and concentrated *in vacuo.* The residue was purified by flash chromatography on silica gel (eluant; 5% methanol:95% dichloromethane) to yield the title compound (220mg, 13%).

### Example E38

### Cyclopropyl-piperidin-4-ylmethyl-carbamic acid tert-butyl ester

### Example E38.1

### 4-Cyclopropylaminomethyl-piperidine-1-carboxylic acid benzyl ester

Cyclopropylamine (1.4g, 24.4mmol) and acetic acid (0.5ml) were added to a solution of 4-formylpiperidine-1-carboxylic acid benzyl ester from Example E33.2 (5.5g, 22.2mmol) in methanol (49.5ml) and the mixture was stirred for 1h at room temperature. Sodium cyanoborohydride (1.84g, 28.9mmol) was added and the mixture was stirred for 20h at room temperature under an inert atmosphere. Solvents were removed *in vacuo* and azeotroped with toluene. The residue was dissolved in EtOAC, washed with saturated NaHCO₃ then brine, dried and concentrated *in vacuo*. The residue was purified by flash chromatography on silica gel (eluant; 10% methanol:90% dichloromethane) to yield the title compound (3.0g, 47%).

### Example E38.2

### 4-[(tert-Butoxycarbonyl-cyclopropyl-amino)-methyl]-piperidine-1-carboxylic acid benzyl ester

DMAP (127mg, 1.04mmol) and di-tert-butyl dicarbonate (3.4g, 12.5mmol) were added to a solution of 4-cyclopropylaminomethyl-piperidine-1-carboxylic acid benzyl ester from Example E38.1 (3.0g, 10.4mmol) in dichloromethane (50ml) and triethylamine (to pH9). The mixture was stirred for 20h at room temperature then concentrated *in vacuo*. The residue was dissolved in EtOAC, washed with saturated NaHCO₃ then brine, dried and concentrated *in vacuo*. The residue was purified by flash chromatography on silica gel (eluant; 40% EtOAc:60% hexane) to yield the title compound (3.67g, 92%).

### Example E38.3

Cyclopropyl-piperidin-4-ylmethyl-carbamic acid tert-butyl ester

4-[(tert-Butoxycarbonyl-cyclopropyl-amino)-methyl]-piperidine-1-carboxylic acid benzyl ester from Example E38.2 (3.67g, 9.4mmol) was dissolved in methanol (100ml) and hydrogenated over 10% Pd/C catalyst (3g) for 1h. The mixture was filtered through Celite® filter agent and the filtrate was concentrated *in vacuo* to yield the title compound (2.07g, 87%).

### Example E39

### 4-[3-(4-Carboxy-2-fluoro-phenoxy)-propyl]-piperazine-1-carboxylic acid tert-butyl ester

### Example E39.1

### 4-[3-(4-Ethoxycarbonyl-2-fluoro-phenoxy)-propyl]-piperazine-1-carboxylic acid tert-butyl ester

Triphenylphosphine polystyrene (loading 1.0 mmol/g, 11g, 11.0mmol) and DEAD (2.45g, 11.0mmol) were added to a solution of 3-fluoro-4-hydroxy-benzoic acid ethyl ester from Example E27 (1.3g, 7.4mmol) and 4-(3-hydroxypropyl)-piperazine-1-carboxylic acid tert-butyl ester from Example E34 (1.8g, 7.4mmol) in tetrahydrofuran (100ml) at 0°C. The suspension was allowed to warm to room temperature and stirred for 18h. The mixture was filtered and the filtrate concentrated *in vacuo*. The residue was purified by flash chromatography on silica gel (eluant; EtOAC) to yield the title compound (1.7g, 57%).

### Example E39.2

### 4-[3-(4-Carboxy-2-fluoro-phenoxy)-propyl]-piperazine-1-carboxylic acid tert-butyl ester

A solution of 4-[3-(4-ethoxycarbonyl-2-fluoro-phenoxy)-propyl]-piperazine-1-carboxylic acid tert-butyl ester from Example E39.1 (1.7g, 4.1 mmol) in dioxan (25ml) was treated with 2N NaOH (3ml) and the mixture stirred at 50°C for 18h. A further aliquot of 2N NaOH was added (2ml), and stirring continued at 50°C for 3h. Solvents were removed *in vacuo* and azeotroped with toluene. The residue was purified by flash chromatography on silica gel (eluant; 1% acetic acid:9% methanol:90% chloroform) to yield the title compound (1.45g, 92%).

### Example E40

### 4-{2-[4-(3,3-Dimethyl-butyl)-piperazin-1-yl]-ethoxy}-3-methyl-benzoic acid

### Example E40.1

### 4-[2-(4-Methoxycarbonyl-2-methyl-phenoxy)-ethyl]-piperazine-1-carboxylic acid tert-butyl ester

4-(2-Hydroxy-ethyl)-piperazine-1-carboxylic acid tert-butyl ester (500mg, 2.2mmol) was dissolved in THF (30ml) and cooled down to 0°C. Polymer-supported triphenylphosphine (2.2g, 2.2mmol), DEAD (378mg, 2.2mmol) and 4-hydroxy-3-methyl-benzoic acid methyl ester from Example E29 (361mg, 2.2mmol) were added and the mixture was stirred for 20h at room temperature. The resin was filtered off and the filtrate concentrated *in vacuo*. The residue was purified by flash chromatography on silica gel (eluant; EtOAc) to yield the title compound (495mg, 60%).

### Example E40.2

### 3-Methyl-4-(2-piperazin-1-yl-ethoxy)-benzoic acid methyl ester dihydrochloride

4-[2-(4-Methoxycarbonyl-2-methyl-phenoxy)-ethyl]-piperazine-1-carboxylic acid tert-butyl ester from Example E40.1 (200mg, 0.53mmol) was dissolved in 4M HCl solution in dioxan (5ml). The mixture was stirred for 30min at room temperature, concentrated *in vacuo* and azeotroped with toluene to yield the title compound (185mg, 100%).

### Example E40.3

### 4-{2-[4-(3,3-Dimethyl-butyl)-piperazin-l-yl]-ethoxy}-3-methyl-benzoic acid methyl ester

3,3-Dimethylbutyraldehyde (54mg, 0.53mmol) was added to a solution of 3-methyl-4-(2-piperazin-1-yl-ethoxy)-benzoic acid methyl ester dihydrochloride from Example E40.2 (146mg, 0.42mmol) in methanol/acetic acid (99:1, v/v, 10ml) and the mixture was stirred at room temperature for 1h. Sodium cyanoborohydride (42mg, 0.69mmol) was added, and the mixture was stirred at room temperature for 18h then concentrated *in vacuo*. The residue was dissolved in EtOAc, washed with saturated NaHCO₃, water then brine, dried and concentrated *in vacuo*. The residue was purified by flash chromatography on silica gel through an isolute (eluant; EtOAc) to yield the title compound (64mg, 34%).

### Example E40.4

### 4-{2-[4-(3,3-Dimethyl-butyl)-piperazin-1-yl]-ethoxy}-3-methyl-benzoic acid

4-{2-[4-(3,3-Dimethyl-butyl)-piperazin-1-yl]-ethoxy}-3-methyl-benzoic acid methyl ester from Example E40.3 (42mg, 0.12mmol) was dissolved in dioxan (5ml) and 1M NaOH (1ml) was added. The mixture was heated at reflux for 2h then solvents were concentrated *in vacuo*. The residue was dissolved in EtOAc, washed with saturated NaHCO₃, water then brine, dried and concentrated *in vacuo* to yield the title compound (40mg, 100%).

### Example E41

### 4-{2-[4-(3,3-Dimethyl-butyl)-piperazin-1-yl]-2-oxo-ethoxy}-3-methyl-benzoic acid

### Example E41.1

### 4-tert-Butoxycarbonylmethoxy-3-methyl-benzoic acid methyl ester

Potassium carbonate (4.6g, 33mmol), potassium iodide (0.25g, 1.5mmol) and tert-butylbromoacetate (2.5ml, 16.5mmol) were added to a solution of 4-hydroxy-3-methyl-benzoic acid methyl ester from Example E29 (2.5g, 15mmol) in acetone (150ml) and the mixture was heated at reflux for 20h. The solid was filtered off and the filtrate concentrated *in vacuo*. The residue was dissolved in EtOAc, washed with 1N KHSO₄, water then brine, dried and concentrated *in vacuo*. The residue was purified by flash chromatography on silica gel (eluant; 15% EtOAc:85% hexane) to yield the title compound (3.8g, 90%).

### Example E41.2

### 4-Carboxymethoxy-3-methyl-benzoic acid methyl ester

Trifluoroacetic acid (20ml) was added to a solution of 4-tert-butoxycarbonylmethoxy-3-methyl-benzoic acid methyl ester from Example E41.1 (3.8g, 13.6mmol) in dichloromethane (40ml) and the mixture was stirred for 1h at room temperature. Volatiles were removed *in vacuo* and azeotroped with dichloromethane to yield the title compound (3.04g, 100%).

### Example E41.3

### 4-{2-[4-(3,3-Dimethyl-butyl)-piperazin-1-yl]-2-oxo-ethoxy}-3-methyl-benzoic acid methyl ester

WSCD (5.14g, 27.2mmol) and DMAP (1.64g, 13.6mmol) were added to a solution of 4-carboxymethoxy-3-methylbenzoic acid methyl ester from Example E41.2 (3.04g, 13.6mmol) in dichloromethane (100ml) and triethylamine (5ml). The mixture was stirred for 1h at room temperature then 1-(3,3-dimethyl-butyl)-piperazine dihydrochloride from Example E4(3.67g, 14.9mmol) was added. The mixture was stirred for 20h at room temperature then solvents were removed *in vacuo*. The residue was dissolved in EtOAc, washed with saturated NaHCO₃ then brine, dried and concentrated *in vacuo*. The residue was purified by flash chromatography on silica gel (eluant; EtOAc) to yield the title compound (2.8g, 55%).

### Example E41.4

### 4-{2-[4-(3,3-Dimethyl-butyl)-piperazin-1-yl]-2-oxo-ethoxy}-3-methyl-benzoic acid

1M Boron tribromide solution (2.66ml, 2.66mmol) was added dropwise to a solution of 4-{2-[4-(3,3-dimethyl-butyl)-piperazin-1-yl]-2-oxo-ethoxy}-3-methyl-benzoic acid methyl ester from Example E41.3 (500mg, 1.33mmol) in dichloromethane (20ml) at 0°C under an inert atmosphere. The mixture was stirred for 20h at room temperature then cooled down to 0°C. 1M Boron tribromide solution (1.33ml, 1.33mmol) was added and the mixture was stirred for 2h at room temperature. Solvents were removed *in vacuo* and azeotroped with toluene. The residue was purified by flash chromatography on silica gel (eluant; 1% acetic acid:5% methanol:94% chloroform) to yield the title compound (350mg, 72%).

### Example E41a

### 4-(2-Bromo-acetyl)-piperazine-1-carboxylic acid tert-butyl ester

A solution of bromoacetyl bromide (8.5ml, 97mmol) in DCM (250ml) at 0°C was treated dropwise with a solution of 1-Boc-piperazine (15.9, 85.3mmol) and triethylamine (18.0ml, 130mmol) in DCM (150ml). After addition was complete, the mixture was stirred at room temperature for 4 h. The solution was washed with ice-cold 1M HCl, sat. aq. NaHCO3, dried and concentrated in vacuo. The residue was purified by flash chromatography on silica gel (eluant; 20% EtOAc:80% cyclohexane to 50% EtOAc:50% cyclohexane) to yield the title compound (15.7g, 60.0%).

### Example E41b

### 2-Chloro-4-hydroxy-benzoic acid methyl ester

### Example E41b.1

### 4-Amino-2-chloro-benzoic acid methyl ester

A solution of 4-Amino-2-chloro-benzoic acid (5.3g, 31mmol) in methanol (100 ml) was treated with acetyl chloride (5ml) and then heated at reflux for 16 hours. The solvents were evaporated in vacuo. The residue was dissolved in EtOAc, washed with saturated sodium hydrogen carbonate, dried and concentrated in vacuo to yield the title compound as a purple solid, 5.45g, 95%.

### Example E41b.2

### 2-Chloro-4-hydroxy-benzoic acid

4-Amino-2-chloro-benzoic acid methyl ester from Example E41b.1 (5.45g, 29.4mmol) was treated with a 35% solution of sulphuric acid (120ml) and the mixture was stirred and heated until dissolution then cooled to 0°C. Sodium nitrite (4.30g, 62.5mmol) in water (25ml) was added dropwise and the mixture was stirred for 15min at 0°C. Urea was added to destroy the excess nitrite. Copper nitrate (200mg, 0.83mmol) was added and heated to 90 °C. The reaction mixture was cooled to room temperature, extracted into ethyl acetate ×3, dried and concentrated in vacuo. The residue was purified by flash chromatography on silica gel (eluant; 30% EtOAc:70% hexane to 50% EtOAc:49% hexane):1% AcOH) to yield the title compound (3.7g, 73%).

### Example E41b.3

### 2-Chloro-4-hydroxy-benzoic acid methyl ester

A solution of 2-chloro-4-hydroxy-benzoic acid (3.70 g, 21.4 mmol)) in methanol (50 ml) was treated with thionyl chloride (2.4 ml, 32 mmol) dropwise and stirred for 24 hours and the solvents were removed in vacuo. The residue was dissolved in ethyl acetate and washed with sat. sodium hydrogen carbonate, dried and solvents were removed in vacuo. The residue was purified by column chromatography on silica gel (eluant with 30% EtOAc:70% cyclohexane) to yield the desired product as a yellow solid, 3.68 g, 92%.

### Example E41c

### 4-[2-(4-Carboxy-3-chloro-phenoxy)-acetyl]-piperazine-1-carboxylic acid tert-butyl ester

### Example E41c.1

### 4-[2-(3-Chloro-4-methoxycarbonyl-phenoxy)-acetyl]-piperazine-1-carboxylic acid tert-butyl ester

2-Chloro-4-hydroxy-benzoic acid methyl ester from example E41b (2.0g, 10.9mmol) and 4-(2-Bromo-acetyl)-piperazine-1-carboxylic acid tert-butyl ester from Example E41a (3.68g, 12.0mmol) in acetonitrile (30ml) were treated with potassium carbonate (1.6g, 11.5mmol), and the mixrure heated at reflux 18 h. before the solvents were removed in vacuo. The residue was adsorbed onto silica gel and purified by flash chromatography on silica gel (eluant; 20% EtOAc:80% cyclohexane to 50% EtOAc:50% cyclohexane to 70% EtOAc:30% cyclohexane) to yield the title compound (4.5g, 100%).

### Example E41c.2

### 4-[2-(4-Carboxy-3-chloro-phenoxy)-acetyl]-piperazine-1-carboxylic acid tert-butyl ester

A solution of 4-[2-(3-Chloro-4-methoxycarbonylphenoxy)-acetyl]-piperazine-1-carboxylic acid tert-butyl ester from Example E41c.1 (4.5g, 10.8mmol) in THF at 0°C was treated with potassium trimethylsilanolate (1.6g, 10.9mmol), and the mixture stirred at room temperature for 18 h. A further 1.6g (10.9mmol) potassium silanolate was added, and stirring continued at room temperatuire for 2h. A further 1.6g (10.9mmol) potassium silanolate was added, and the mixture was stirred at 45°C for 2h. The mixture was then diluted with water and THF was removed in vacuo. The residue was washed with ether, cooled in an ice-bath and adjusted to pH5 with solid KHSO₄ whereupon it was extracted with CHCl₃ and CHCl₃/isopropanol (90:10 v/v). The combined organics were dried and solvents were removed in vacuo to yield the title compound (3.7g, 85%).

### Example E42

### 4-(3-tert-Butoxycarbonyl-propoxy)-3-methyl-benzoic acid

### Example E42.1

### 4-Bromo-butyric acid tert-butyl ester

1M Boron tribromide (39ml, 39mmol) was added to a solution of butyrolactone (3ml, 39mmol) in dichloromethane (30ml). The mixture was stirred for 20h at room temperature then quenched with an excess of tert-butylalcohol. The mixture was stirred for 2h at room temperature, diluted with dichloromethane, washed with saturated NaHCO₃, saturated Na₂S₂O₃ then brine, dried and concentrated *in vacuo*. The residue was purified by flash chromatography on silica gel through an isolute (eluant; 5% EtOAc:95% hexane) to yield the title compound (3g, 34%).

### Example E42.2

### 4-(3-tert-Butoxycarbonyl-propoxy)-3-methyl-benzoic acid methyl ester

Potassium carbonate (580mg, 4.20mmol) and potassium iodide (72mg, 0.43mmol) were added to a solution of 4-hydroxy-3-methyl-benzoic acid methyl ester from Example E29 (293mg, 1.76mmol) and 4-bromo-butyric acid tert-butyl ester from Example E42.1 (397mg, 1.78mmol) in acetone (50ml). The mixture was heated at reflux for 18h then the solid was filtered off and the filtrate concentrated *in vacuo*. The residue was dissolved in EtOAc, washed with water then brine, dried and concentrated *in vacuo*. The residue was purified by flash chromatography on silica gel (eluant; 20% EtOAc:80% pet. ether) to yield the title compound (414mg, 76%).

### Example E42.3

### 4-(3-tert-Butoxycarbonyl-propoxy)-3-methyl-benzoic acid

Lithium hydroxide monohydrate (137mg, 3.26mmol) was added to a solution of 4-(3-tert-butoxycarbonylpropoxy)-3-methyl-benzoic acid methyl ester from Example E42.2 (414mg, 1.34mmol) in THF (10ml) and water (5ml). The mixture was stirred for 48h at room temperature and concentrated *in vacuo*. The residue was dissolved in chloroform, acidified with 1M HCl then washed with brine, dried and concentrated *in vacuo* to yield the title compound (275mg, 70%).

### Example E43

### 4-{4-[4-(3,3-Dimethyl-butyl)-piperazin-1-yl]-4-oxobutyl}-3-methyl-benzoic acid

### Example E43.1

### 4-{4-[4-(3,3-Dimethyl-butyl)-piperazin-1-yl]-4-oxobutyl}-3-methyl-benzoic acid methyl ester

HBTU (910mg, 2.4mmol) was added to a solution of 4-(3-carboxy-propyl)-3-methyl-benzoic acid methyl ester from Example E30 (378mg, 1.6mmol) and 1-(3,3-dimethyl-butyl)-piperazine hydrochloride from Example E4 (467mg, 1.9mmol) in dichloromethane (15ml) and DIEA (0.836ml, 4.8mmol). The mixture was stirred for 20h at room temperature, washed with saturated NaHCO₃ then brine, dried and concentrated *in vacuo*. The residue was purified by flash chromatography on silica gel through an isolute (eluant; 5% methanol:95% dichloromethane) to yield the title compound (582mg, 94%).

### Example E43.2

### 4-{4-[4-(3,3-Dimethyl-butyl)-piperazin-1-yl]-4-oxobutyl}-3-methyl-benzoic acid

Lithium hydroxide monohydrate (108mg, 2.6mmol) was added to a solution of 4-{4-[4-(3,3-dimethyl-butyl)-piperazin-1-yl]-4-oxo-butyl}-3-methyl-benzoic acid methyl ester from Example E43.1 (500mg, 1.3mmol) in THF (12ml) and water (6ml). The mixture was stirred for 20h at room temperature then solvents were removed *in vacuo*. The residue was purified by flash chromatography on silica gel (eluant; 2% acetic acid:4% methanol:94% dichloromethane) then recrystallised from chloroform and pet. ether to yield the title compound (439mg, 91%).

### Example E44

### 4-{3-[4-(3,3-Dimethyl-butyl)-piperazin-1-yl]-3-oxopropyl}-3-methyl-benzole acid

### Example E44.1

### 4-{3-[4-(3,3-Dimethyl-butyl)-piperazin-1-yl]-3-oxopropyl}-3-methyl-benzoic acid methyl ester

Oxalyl chloride (2.6ml, 30mmol) was added slowly to a solution of 4-(2-carboxy-ethyl)-3-methyl-benzoic acid methyl ester from Example E30.3 (5.33g, 24mmol) in dichloromethane (60ml) and few drops of DMF at 0°C. The mixture was stirred for 2h at room temperature then concentrated *in vacuo* and azeotroped with toluene. The residue was dissolved in dichloromethane (30ml) and added to a solution of 1-(3,3-dimethyl-butyl)-piperazine hydrochloride from Example E4 (6.3g, 26mmol) in dichloromethane (45ml) and DIEA (17ml, 96mmol) at 0°C. The mixture was stirred for 1h at room temperature then concentrated *in vacuo*. The residue was purified by flash chromatography on silica gel (eluant; 5% methanol:95% dichloromethane) to yield the title compound (8.5g, 96%).

### Example E44.2

### 4-{3-[4-(3,3-Dimethyl-butyl)-piperazin-1-yl]-3-oxopropyl}-3-methyl-benzoic acid

Lithium hydroxide monohydrate (2.4g, 56.7mmol) was added to a solution of 4-{3-[4-(3,3-dimethyl-butyl)-piperazin-1-yl]-3-oxo-propyl}-3-methyl-benzoic acid methyl ester from Example E44.1 (8.5g, 22.7mmol) in THF (200ml) and water (100ml). The mixture was stirred for 24h at room temperature then solvents were removed *in vacuo*. The residue was purified by flash chromatography on silica gel (eluant; 2% acetic acid:4% methanol:94% dichloromethane) to yield the title compound (8.1g, 99%).

### Example E88

### 1-Methyl-5-(3-methyl-2-nitro-phenylamino)-1H-pyrazole-4-carboxylic acid ethyl ester

Cesium carbonate (35.2g, 108mmol), 4,5-bis(diphenylphosphino)-9,9-dimethylxanthene (0.4g, 0.7mmol) and tris(dibenzylideneacetone)dipalladium(0) (0.2g, 0.2mmol) were added to a solution of ethyl 5-amino-1-methylpyrazole-4-carboxylate (15.3g, 90mmol) in dioxan (50ml) under an inert atmosphere. A solution of 3-bromo-2-nitrotoluene (16.2g, 75mmol) in dioxane (10ml) was added and the mixture was stirred for 30min at room temperature then heated at reflux for 72h. The suspension was filtered through Celite® filter agent, washed with EtOAc and the filtrate was concentrated *in vacuo*. The residue was purified by flash chromatography on silica gel (eluant; 50% EtOAc:50% pet. ether) then recrystallised from EtOAc/pet. ether to yield the title compound (12.5g, 45%).

### Example E89

### 5-Amino-1-ethyl-1H-pyrazole-4-carboxylic acid ethyl ester

Triethylamine (19.6ml, 144mmol) was added cautiously to a solution of ethyl (ethoxymethylene)cyanoacetate (10.1g, 60mmol) and ethyl hydrazine oxalate (9.9g, 66mmol) in ethanol (200ml). The mixture was heated at reflux for 24h then concentrated *in vacuo*. The residue was redissolved in EtOAc, washed with 5% KHCO₃ solution then brine, dried and concentrated *in vacuo.* The residue was purified by flash chromatography on silica gel (eluant; 25% pet. ether:75% EtOAc) to yield the title compound (8.8g, 80%).

### Example A

### In vitro Testing

Compounds were assayed to determine their ability to inhibit the cellular consequences of AVP stimulation on intact cells. In the assay, the compounds of the invention cause significant inhibition of cellular activation at concentrations of 30 µM or less. Preferred compounds cause significant inhibition at concentrations of 300 nM.

### Example B

### Tablet for Oral Administration

Tablets containing 100 mg of the compound of Example E8 as the active agent may be prepared from the following:

| | |
|---|---|
| Compound of Example E8 | 200.0 g |
| Corn starch | 71.0 g |
| Hydroxypropylcellulose | 18.0 g |
| Carboxymethylcellulose calcium | 13.0 g |
| Magnesium stearate | 3.0 g |
| Lactose | 195.0 g |
| *Total* | *500.0 g* |

The materials are blended and then pressed to give 2000 tablets of 250 mg, each containing 100 mg of the compound of Example E8.

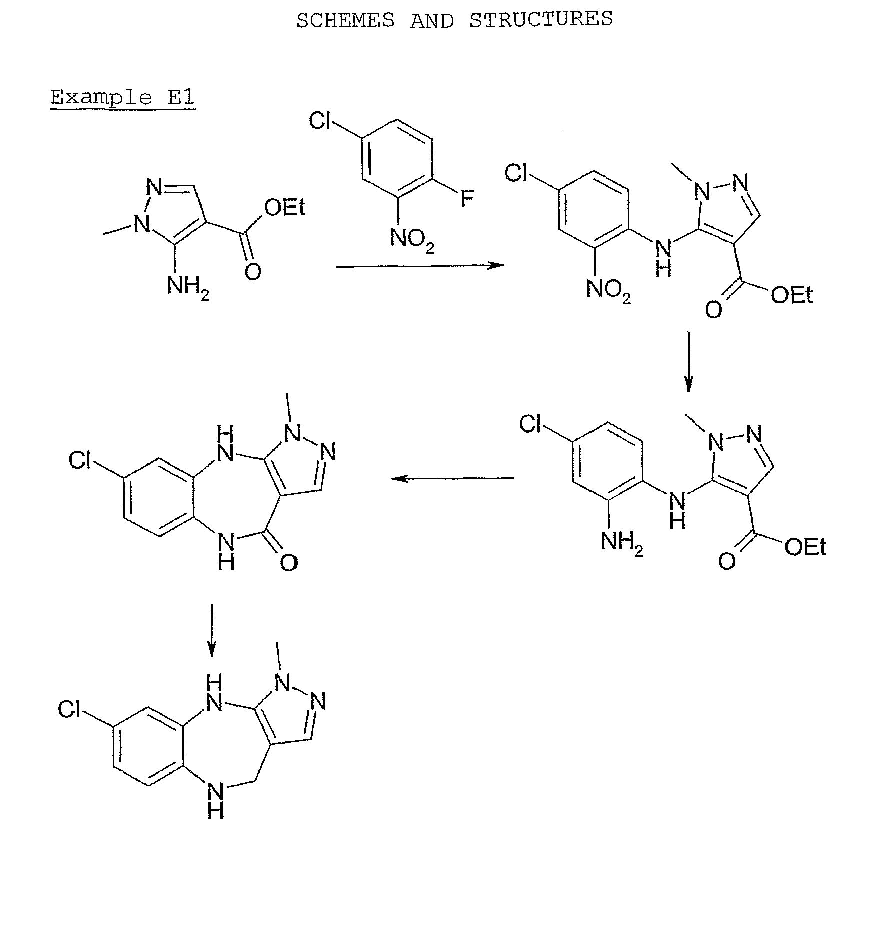

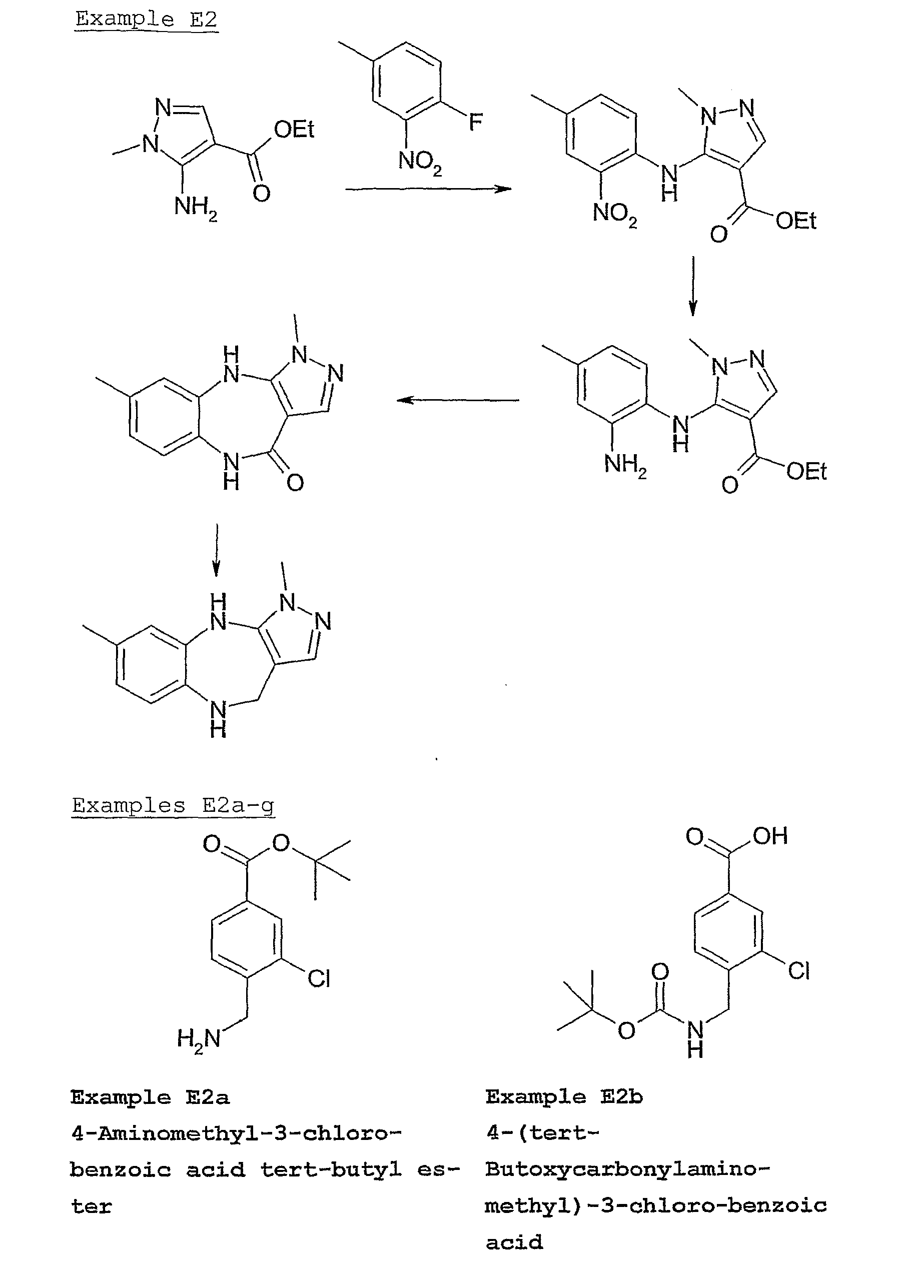

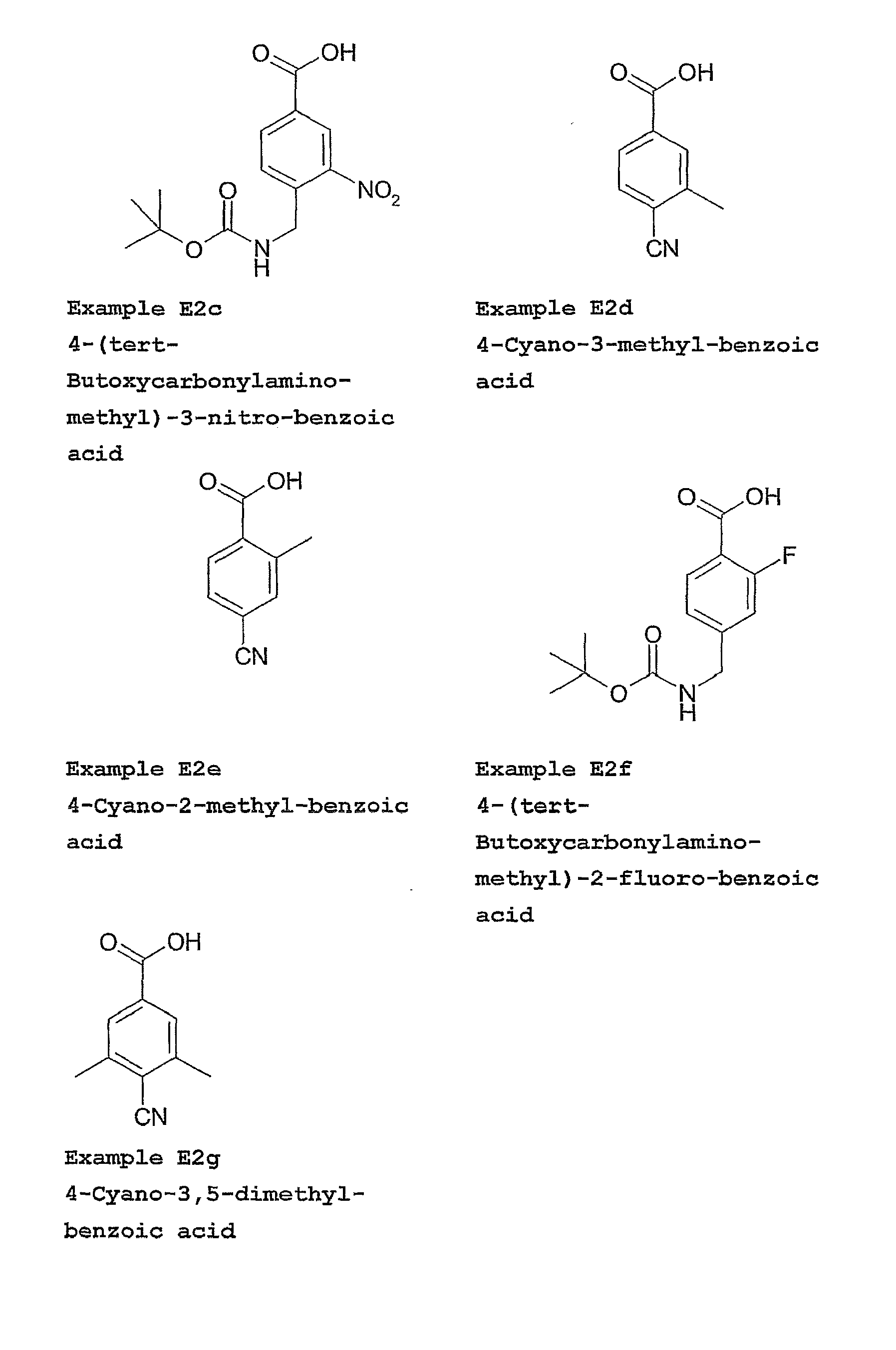

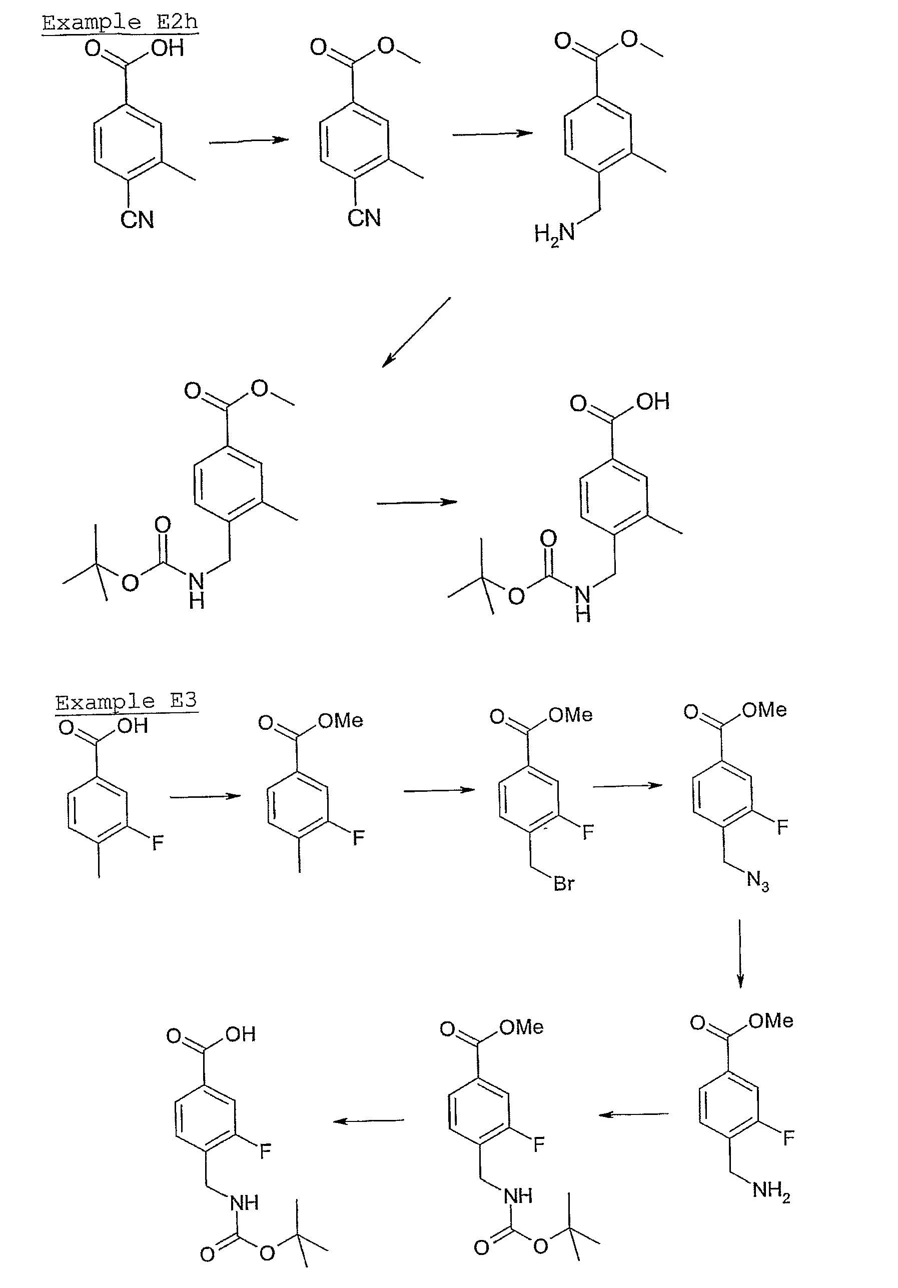

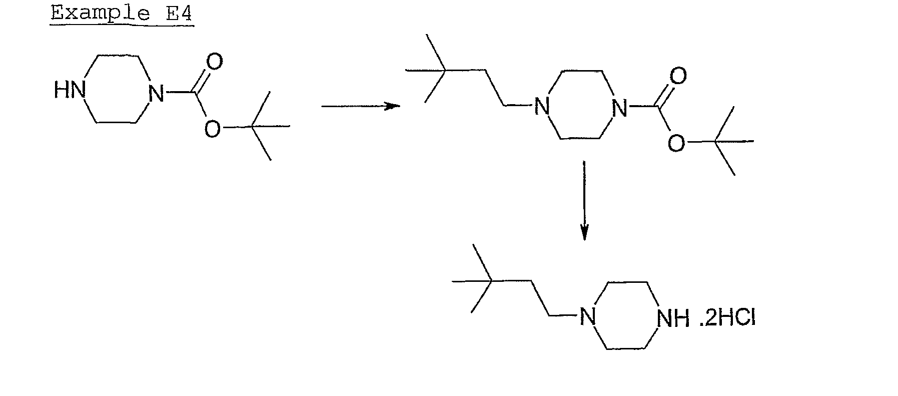

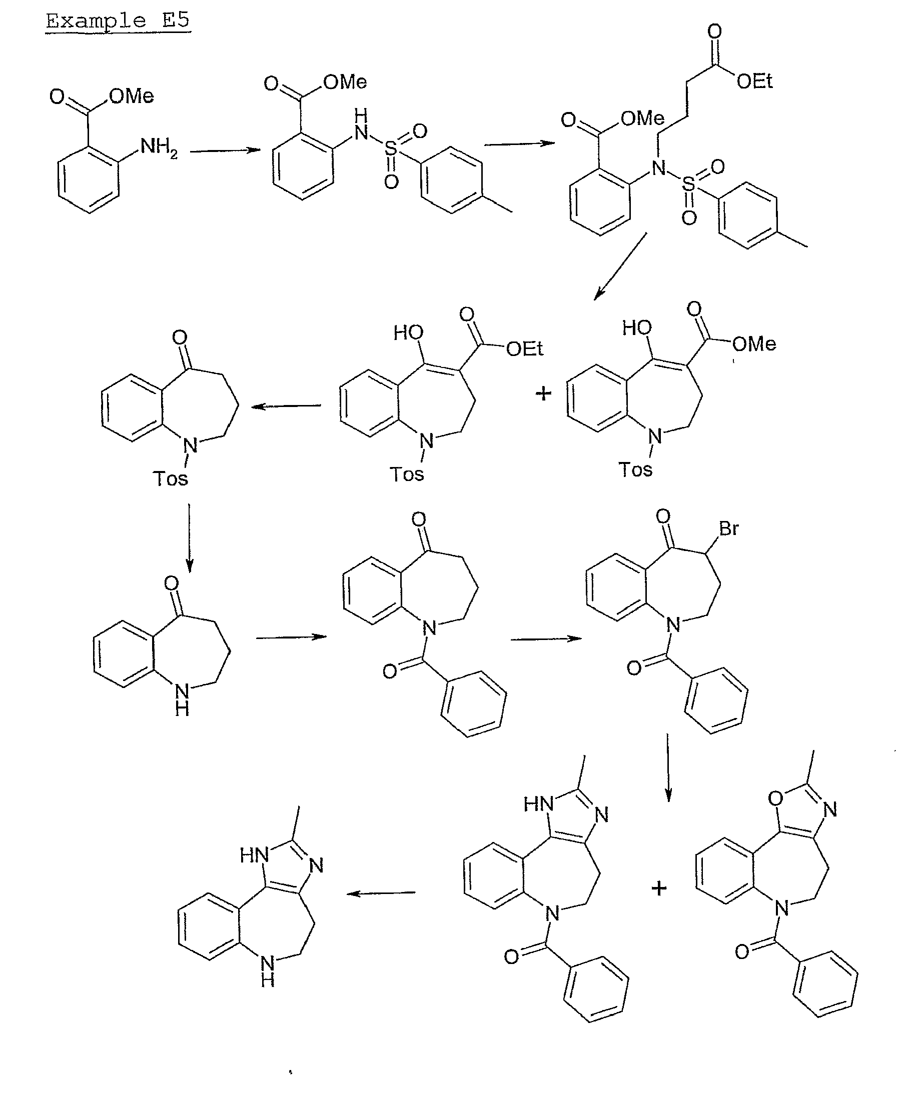

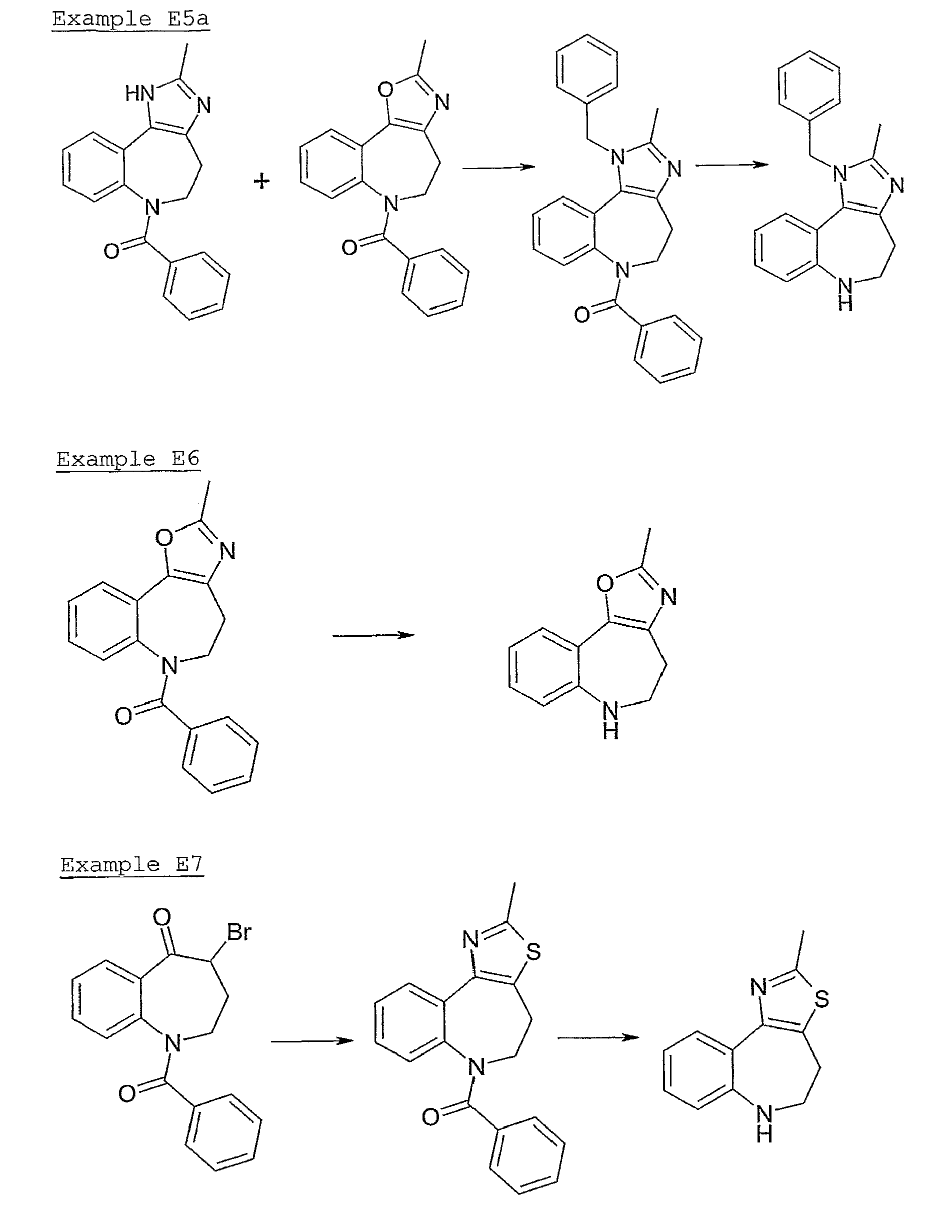

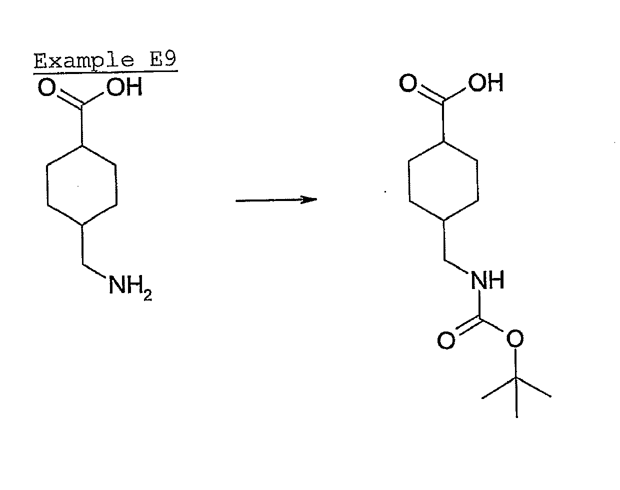

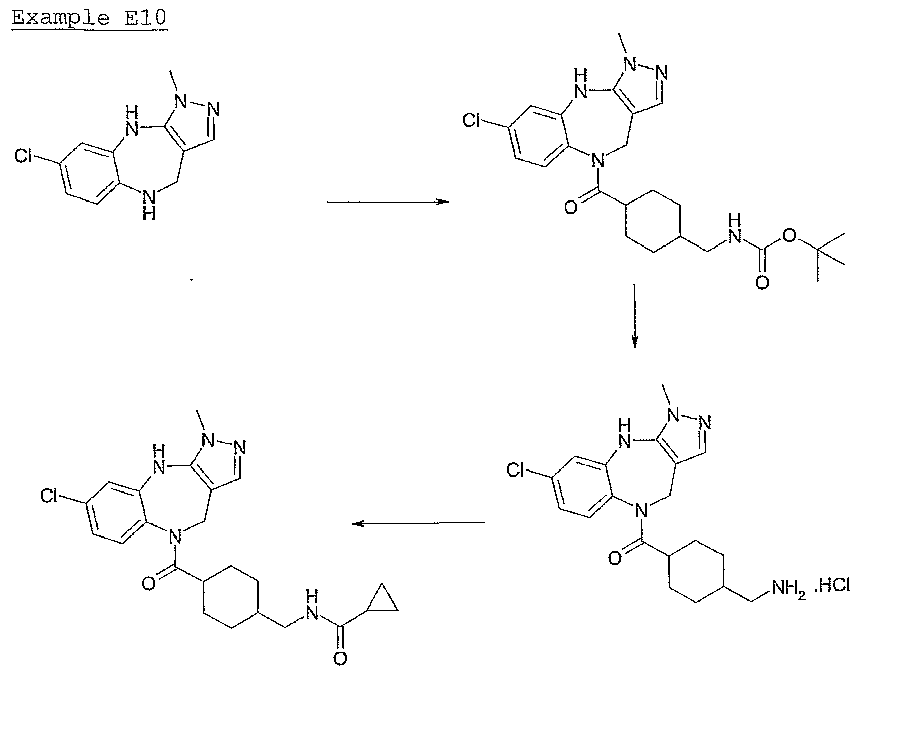

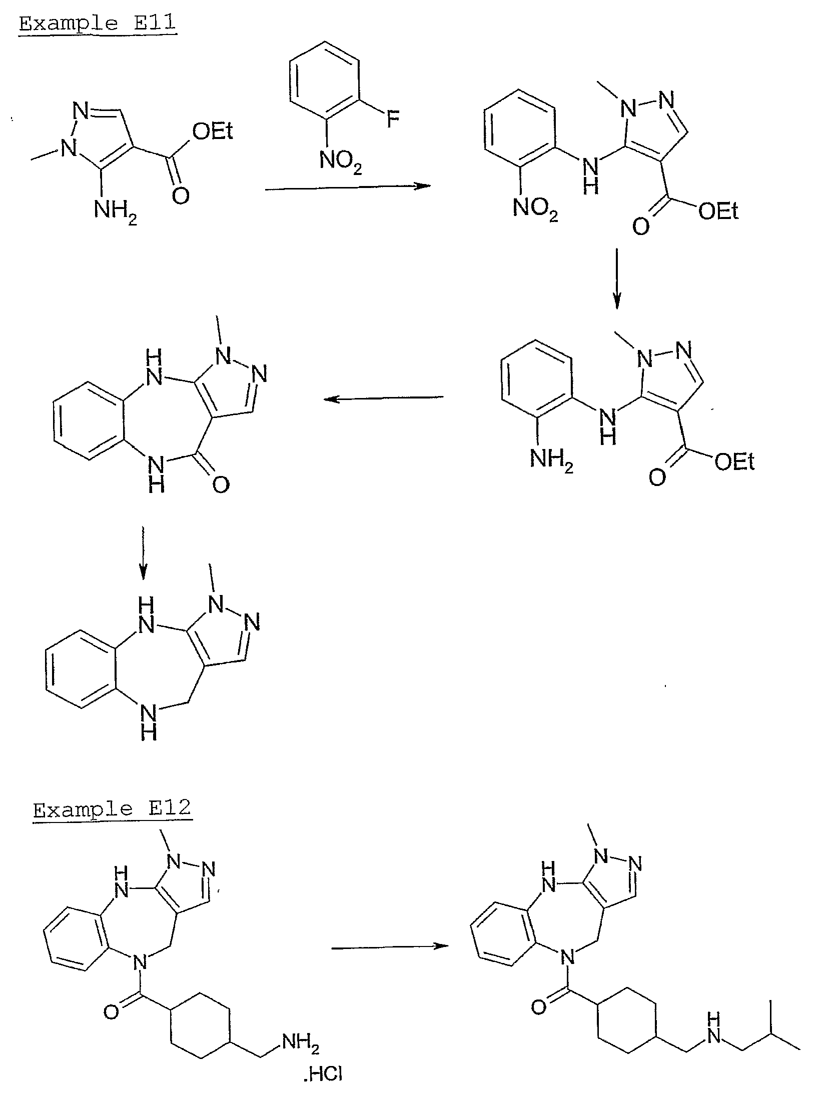

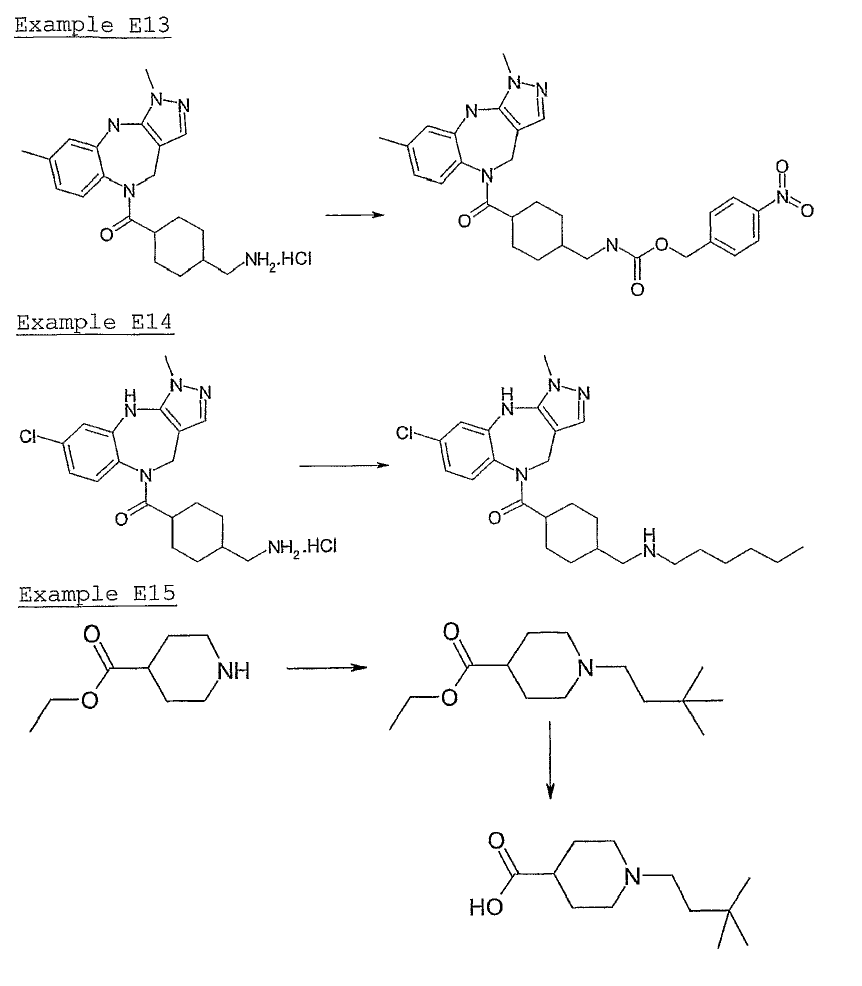

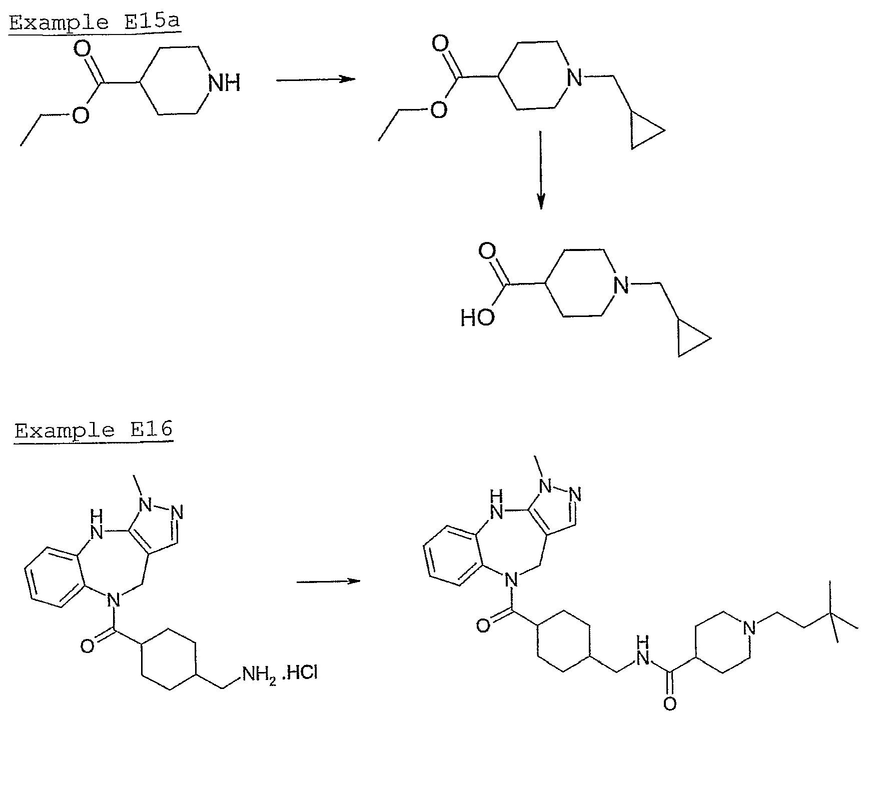

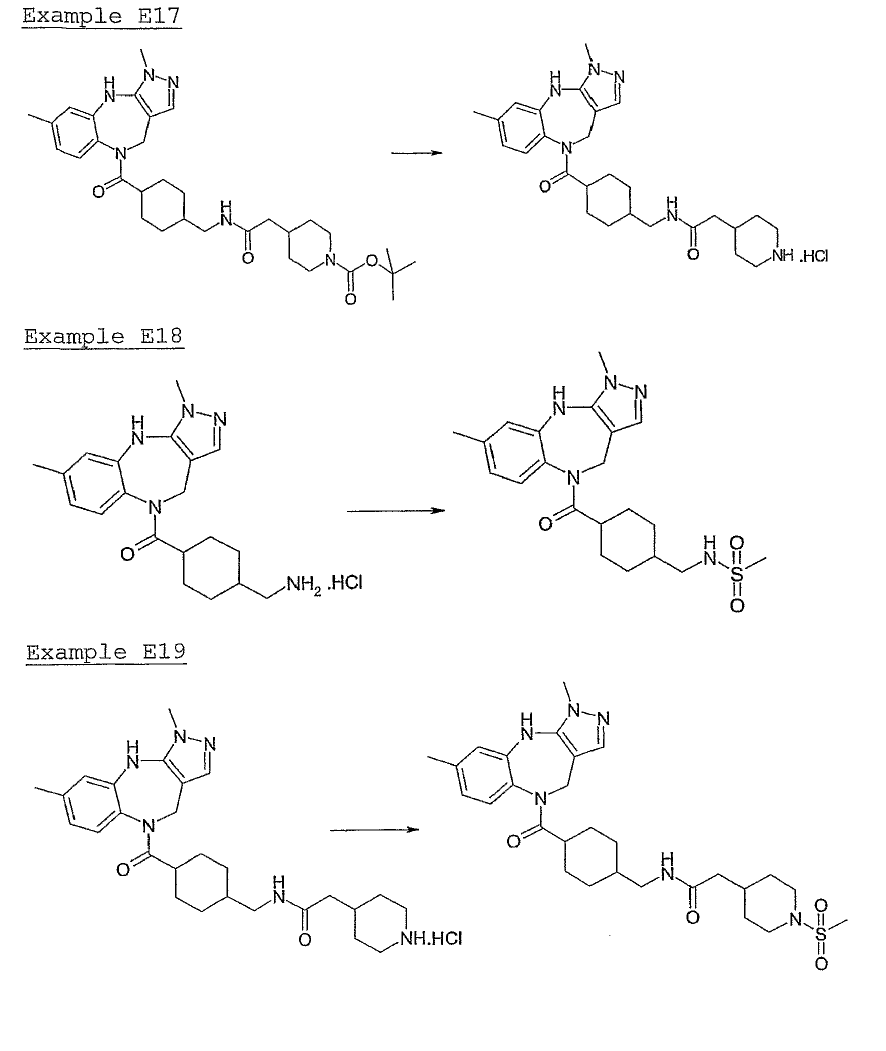

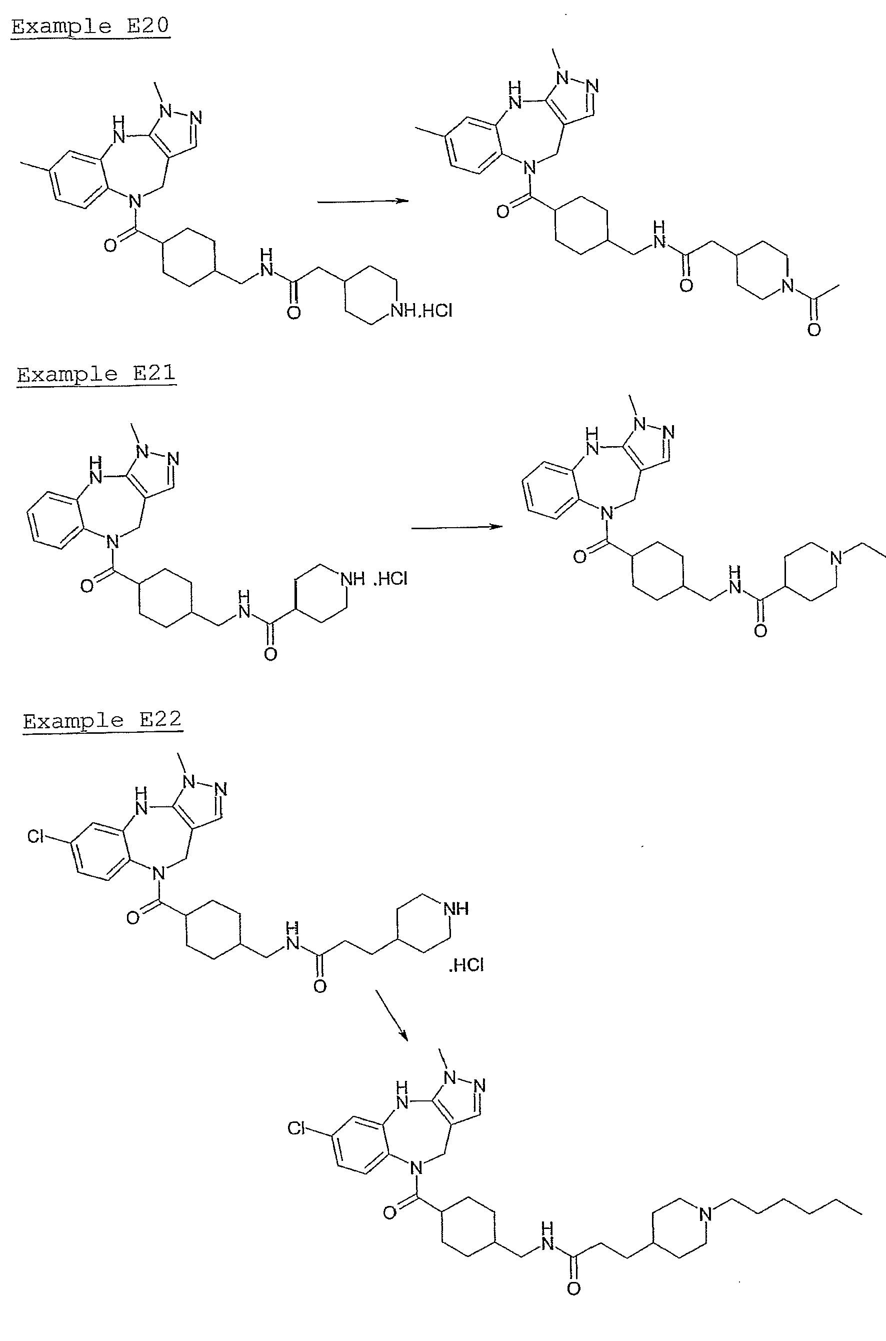

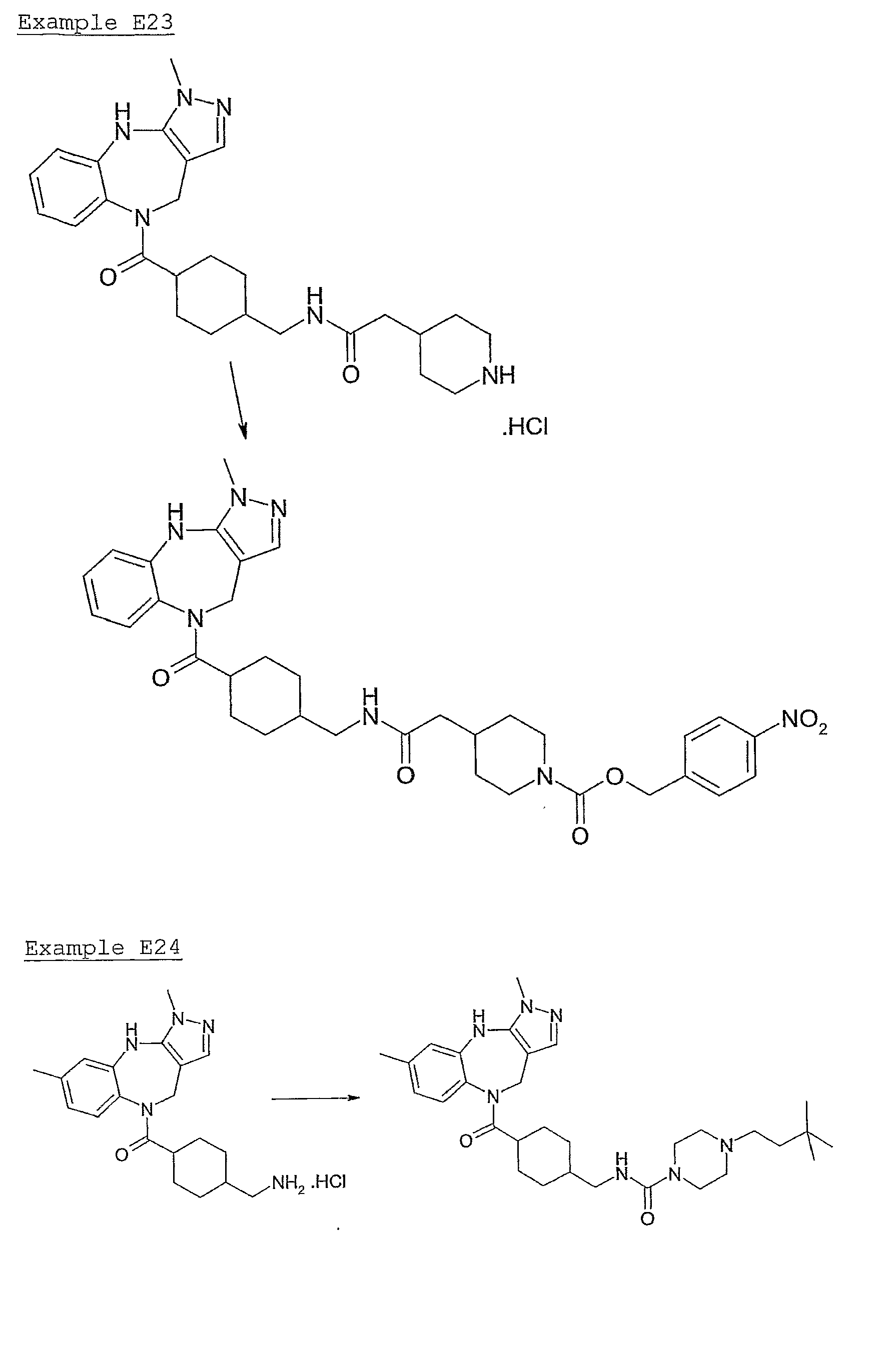

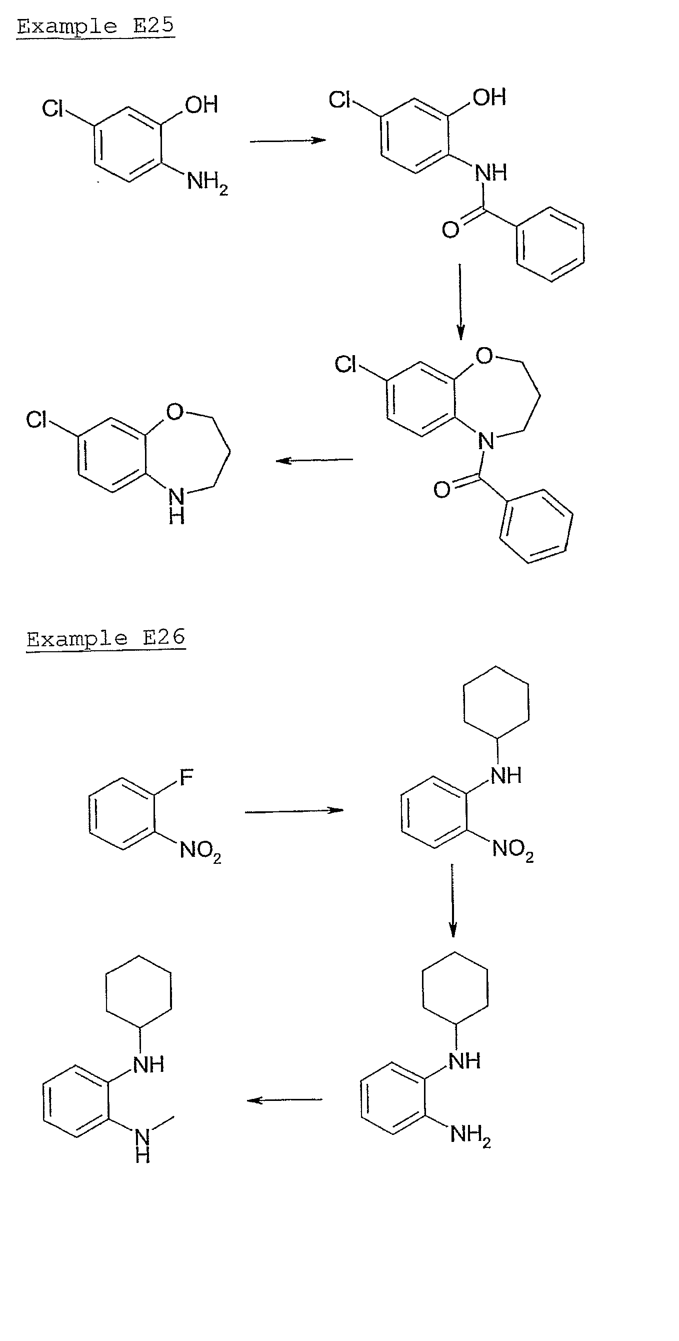

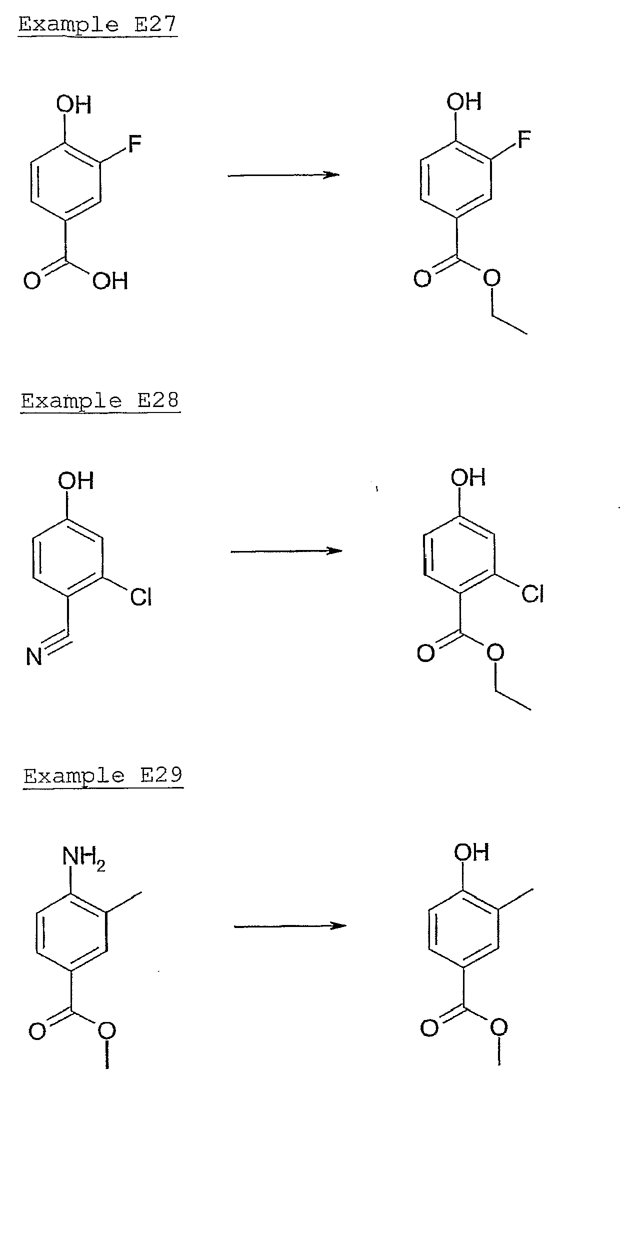

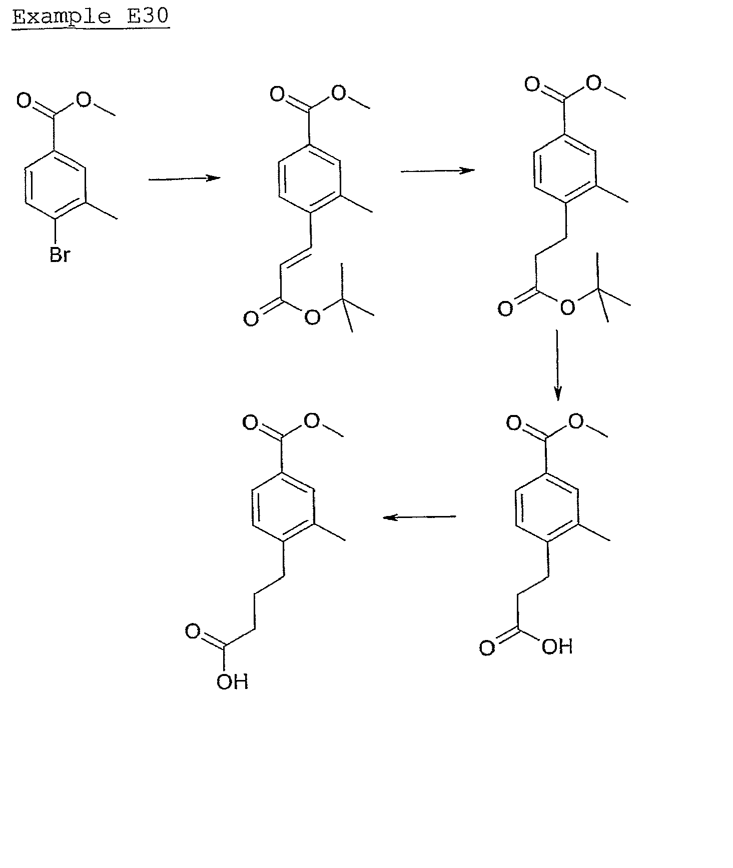

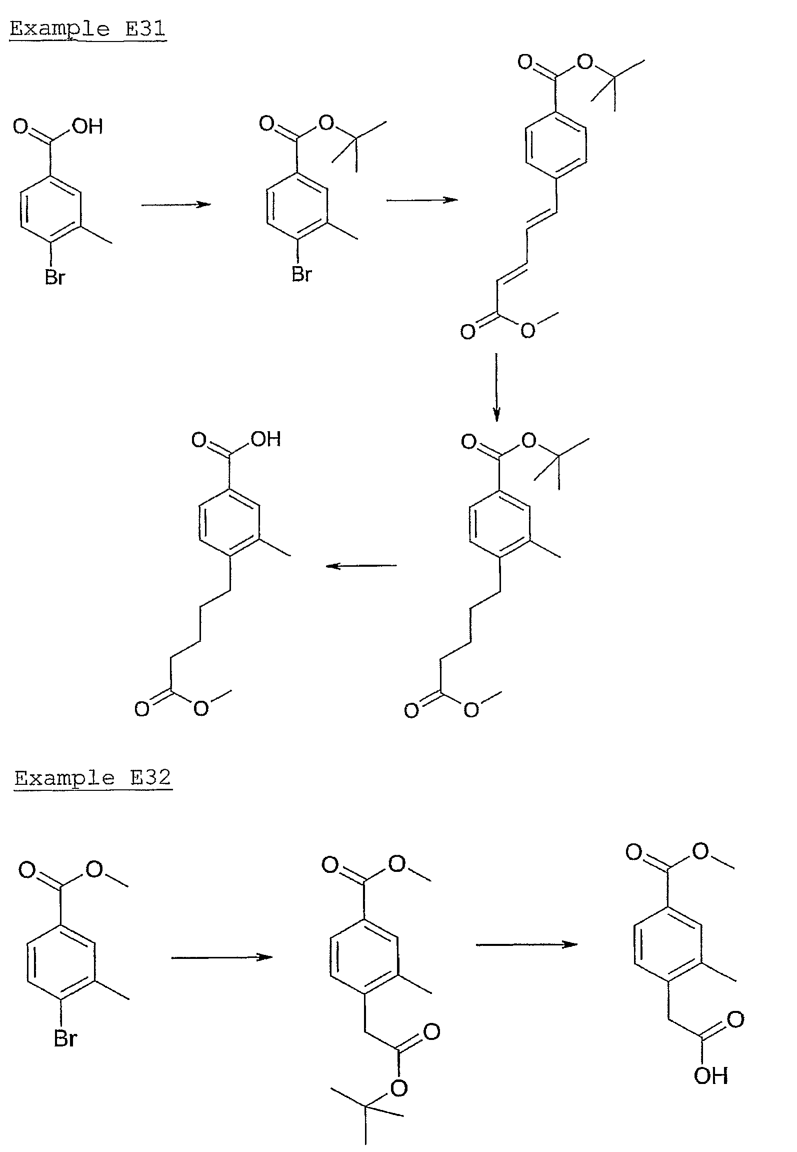

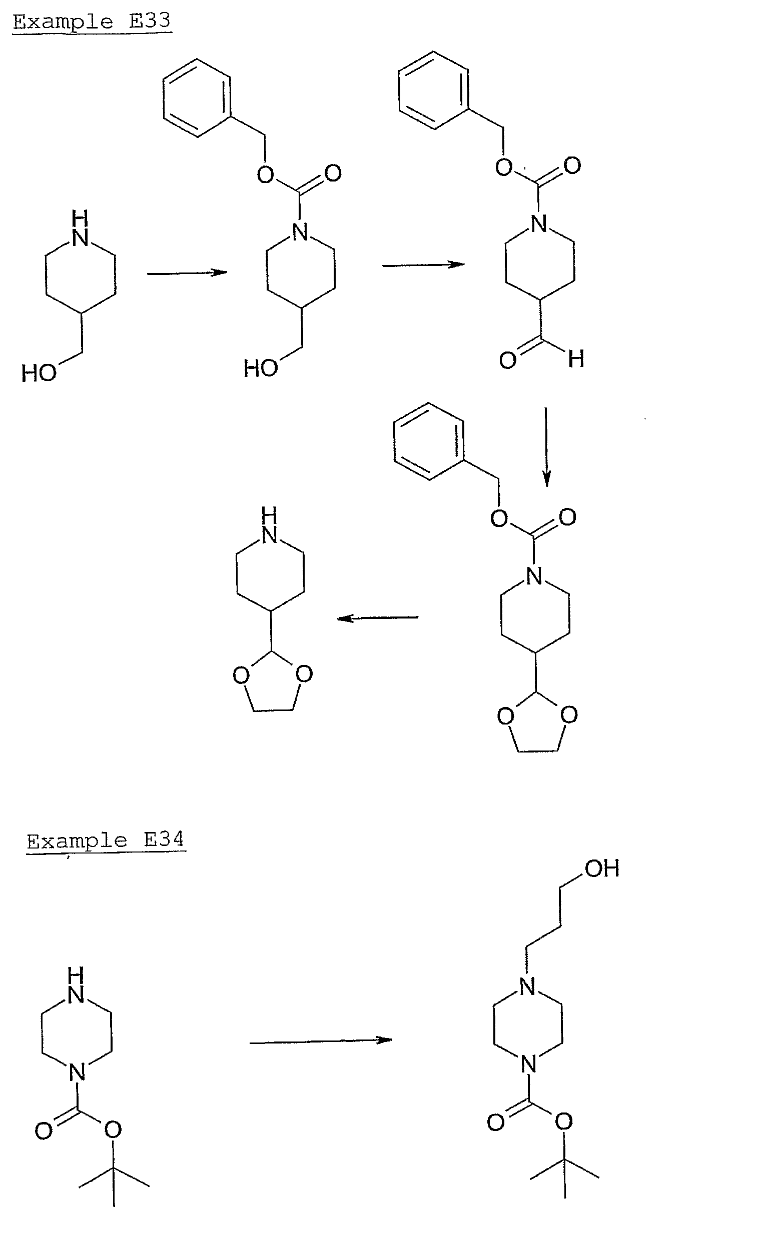

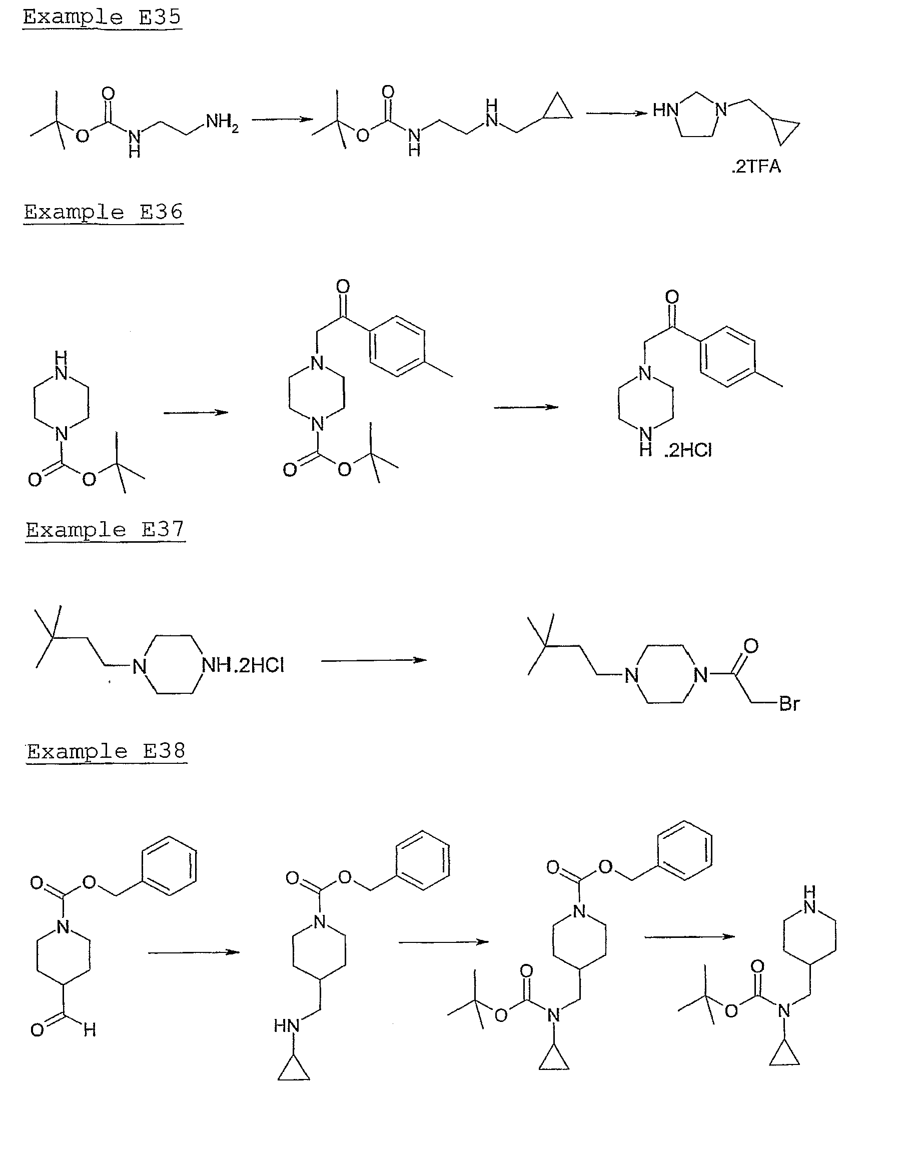

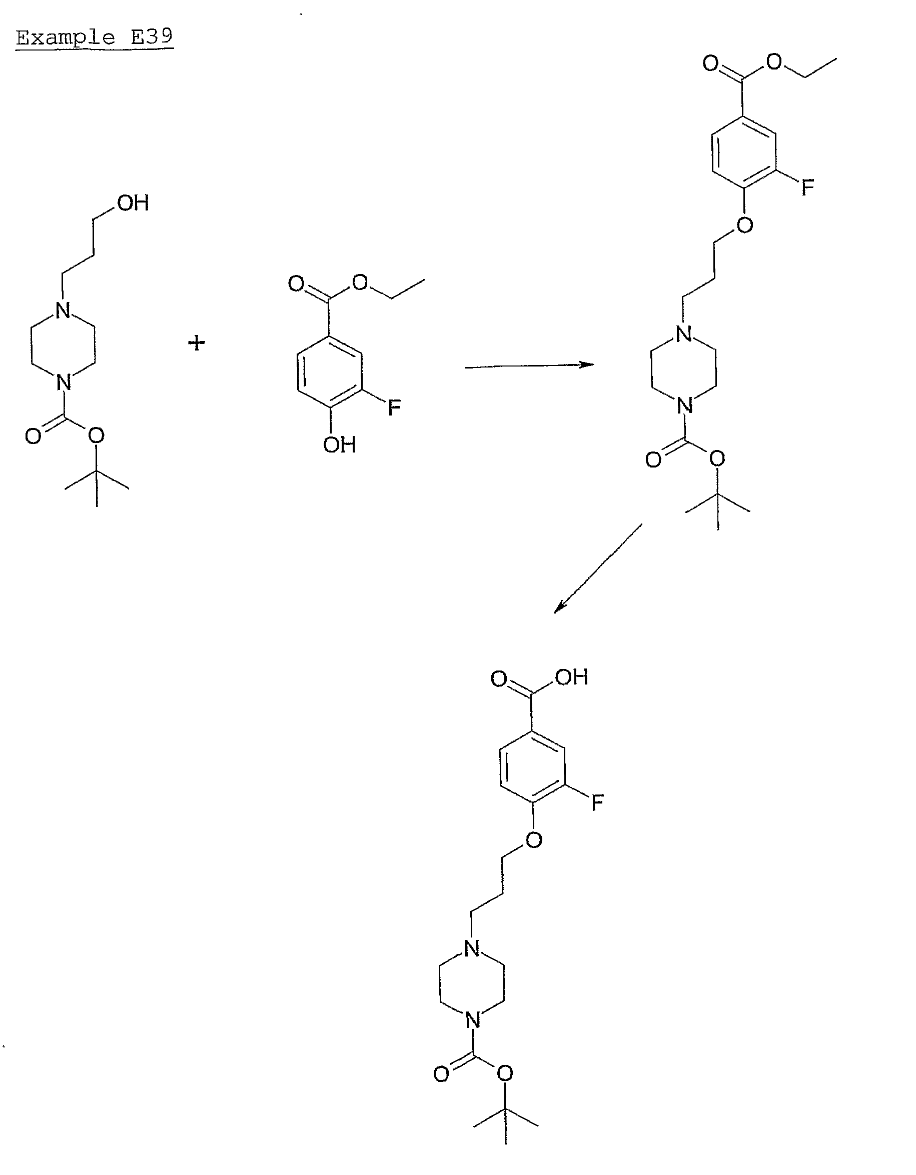

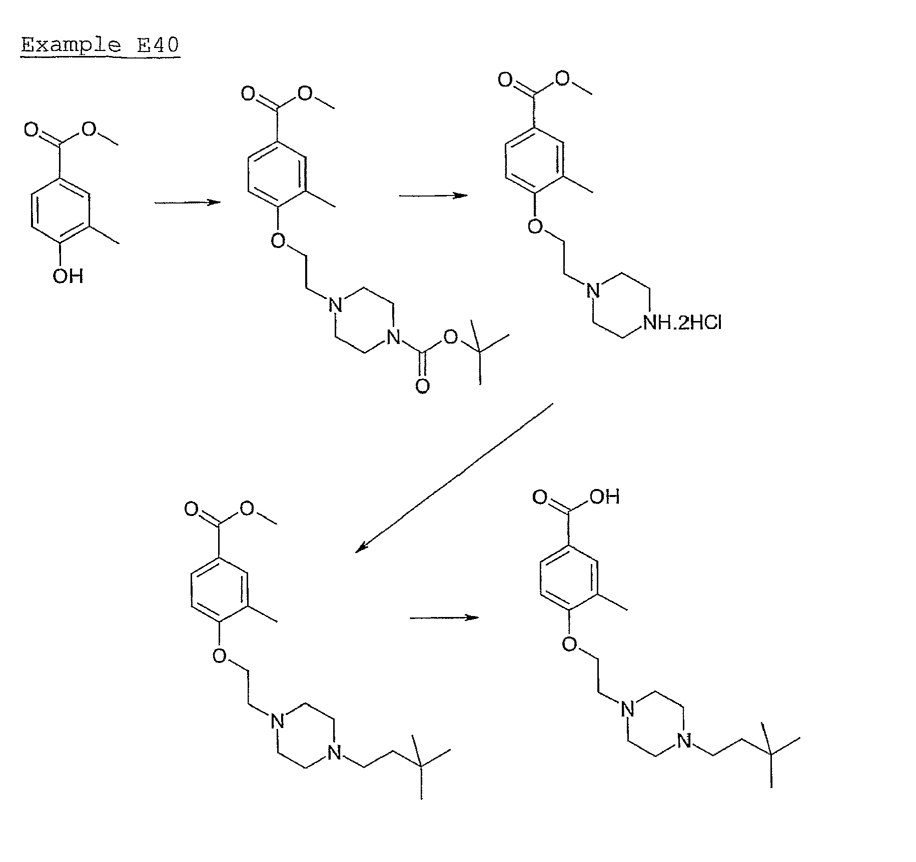

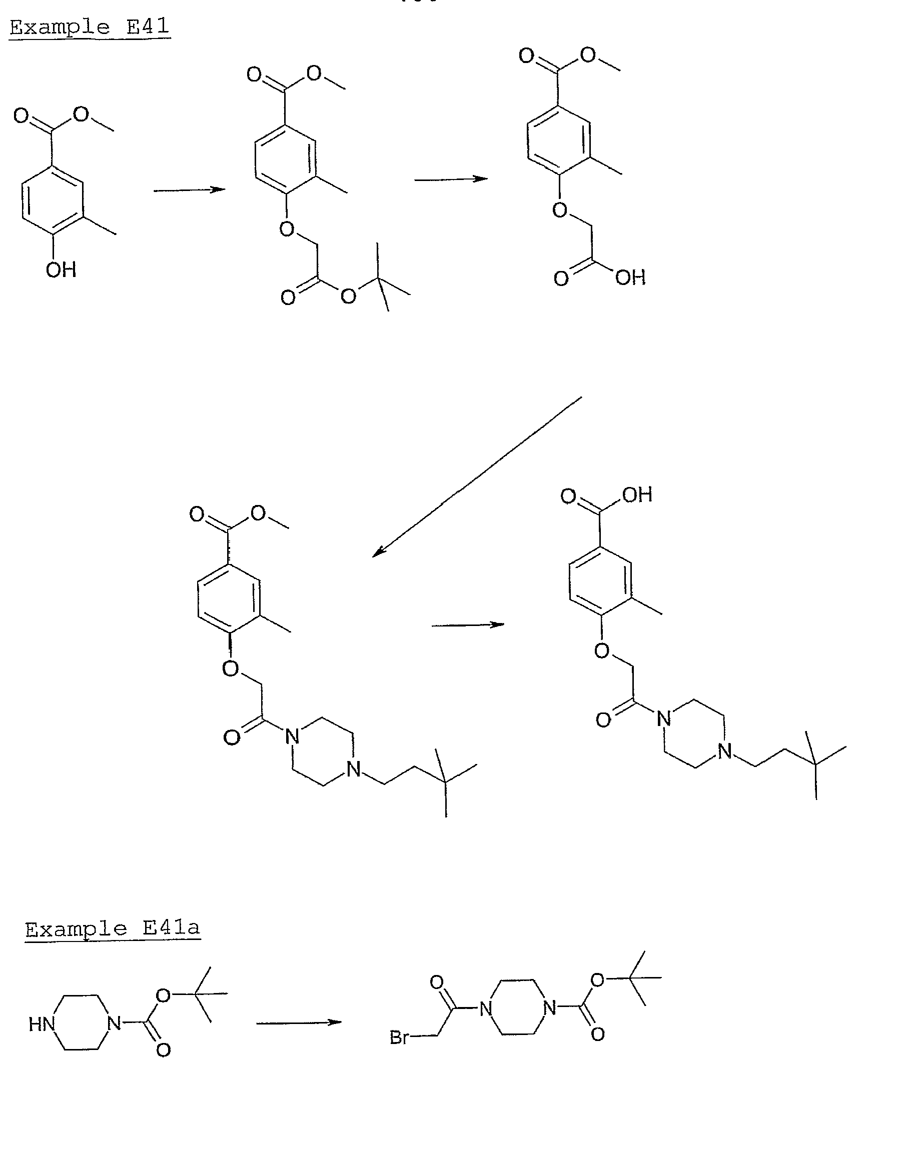

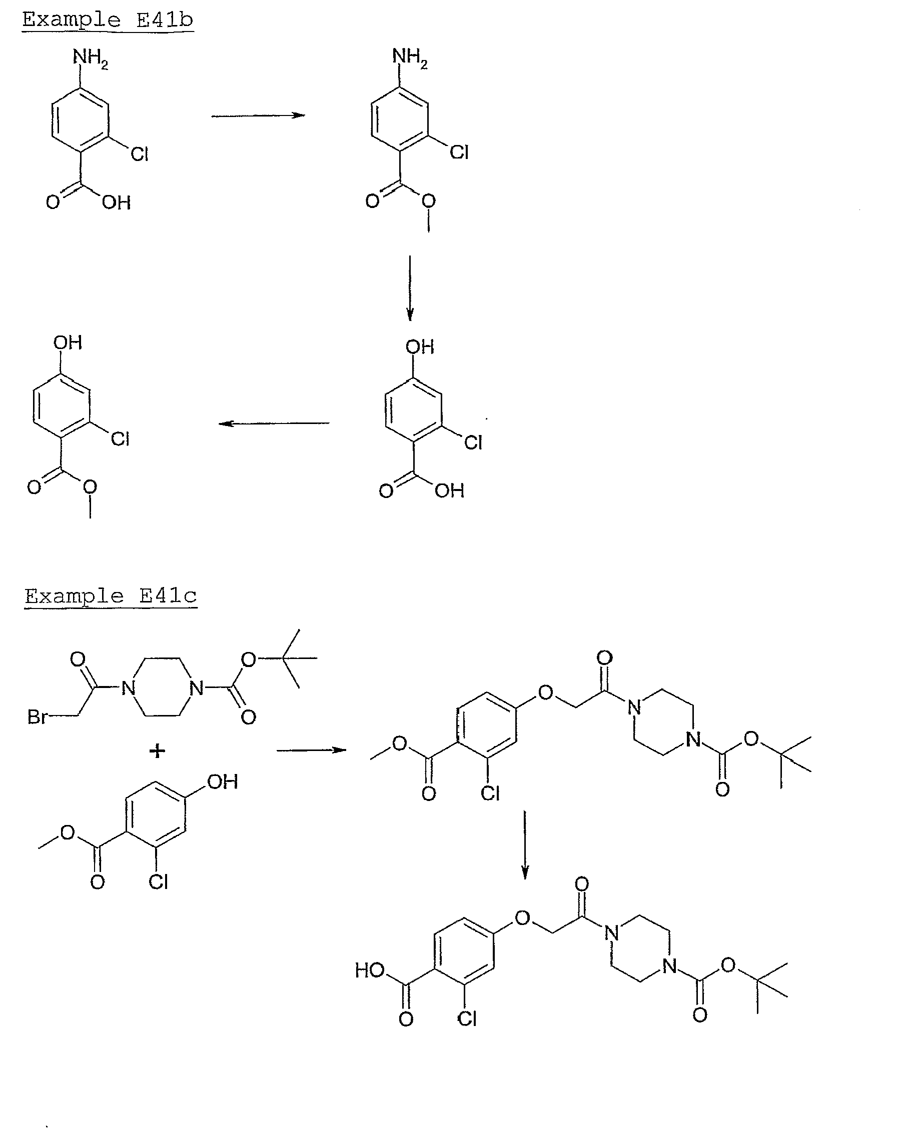

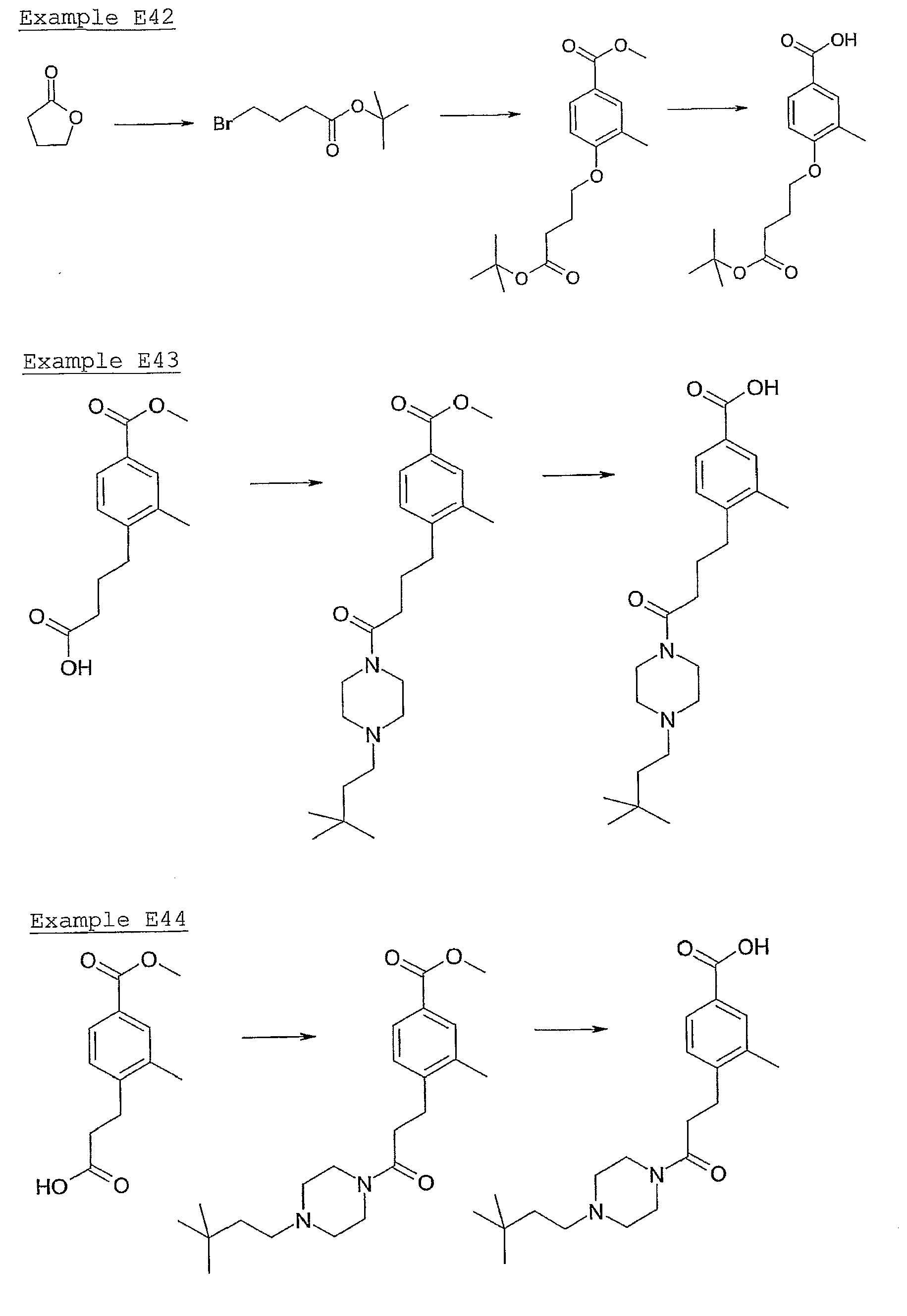

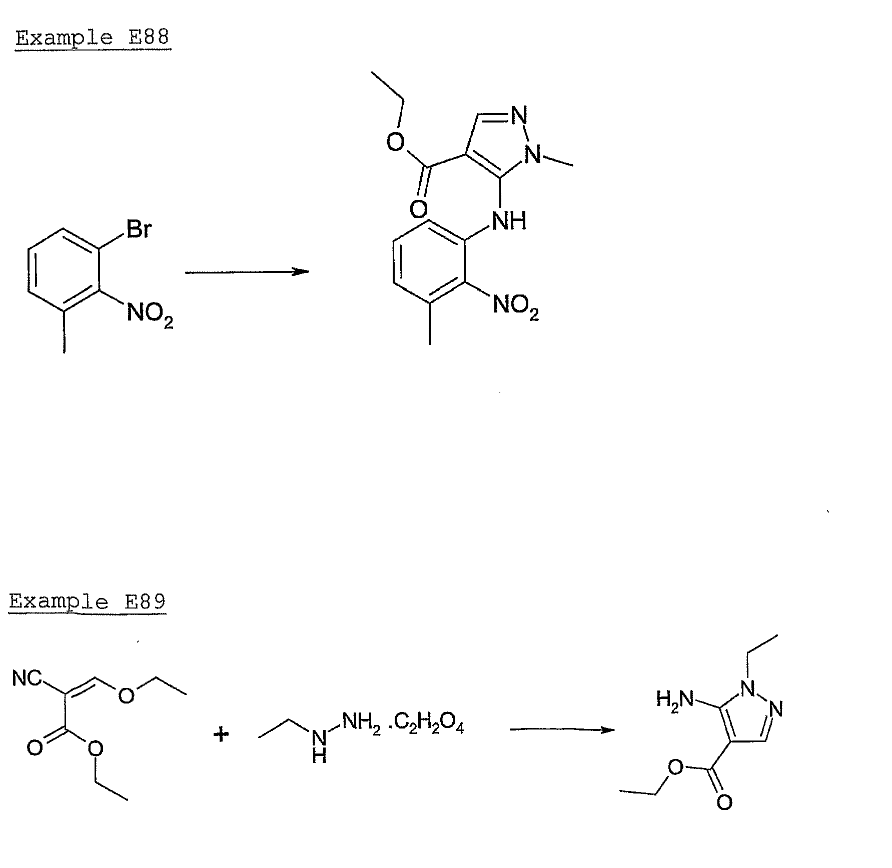

### EXAMPLE TABLES

The following compounds were prepared using methods analogous to those described above.

Example E5 and Compound number 2 have particular utility as intermediates.

| | | | |
|---|---|---|---|
| 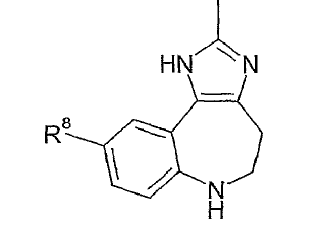 | | | |

| **Compound number** | **R⁸** | **MS** **(ESI)+: [M+H]+ =** | **¹H NMR:** δ **(ppm)** |
|---|---|---|---|
| 1 E5 | H | | |
| 2 | Cl | 234.0, 236.0 | 2.43 (3H, s), 2.98 (2H, d, J=5.2Hz), 3.34 (2H, t, J=5.2 Hz), 6.59 (2H, d, J=8.4Hz), 6.90-6.95 (1H, m), 7.80-8.10 (1H, m) |

| | | | | |
|---|---|---|---|---|
| 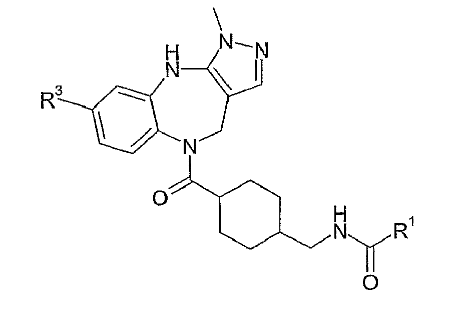 | | | | |

| **Compound number** | R³ | **R¹** | **¹H NMR: δ (ppm)** | **MS** |
|---|---|---|---|---|
| 109 E10 | Cl | 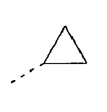 | 0.42-0.90 (6H, m), 1.28 (6H, m), 1.78-1.80 (2H, m), 2.08 (1H, m), 2.95-3.01 (2H, m), 3.66 (3H, s), 3.76 (1H,d, J=14.6Hz), 5.55 (1H, d, J=14.6Hz), 5.95 (1H, m), 6.97 (1H, dd, J=8.4, 2.1 Hz), 7.03 (1H, s), 7.07 (1H, d, J=8.4Hz), 7.09 (1H, d, J=2.1 Hz), 7.14 (1H, s) | (ESI)+:[M+H]+=4 42.3, 444.3 |
| 110 | Me | 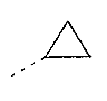 | 0.58-0.90 (6H, m), 1.23-2.13 (9H, m), 2.33 (3H, s), 2.95-3.03 (2H, m), 3.68 (3H, s), 3.76 (1H, d, J=14.6Hz), 5.59 (1H, d, J=14.6Hz), 5.80 (1H, m), 6.15 (1H, s), 6.81-6.83 (2H, m), 6.97-7.00 (1H, m), 7.14 (1H, s) | (ESI)+:[M+H]+=4 22.4 |
| 111 | H | 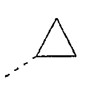 | 0.58-0.81 (6H, m), 1.31-1.74 (6H, m), 1.69-1.80 (2H, m), 2.09-2.16 (1H, m), 2.89-2.98 (2H, m), 3.64 (3H, s), 3.80 (1H, d, J=14.6Hz), 5.58 (1H, d, J=14.6Hz), 6.49 (1H, m), 6.95-7.23 (5H, m), 7.39 (1H, s) | (APCI)+: [M+H]+= 408.3 |
| 112 | Me | CH₃ | | |
| 113 | Me | 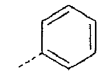 | | (ESI)+:[M+H]+=4 58.7 |
| 114 | Me | 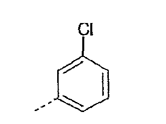 | | (ESI)+:[M+H]+=4 92.2, 494.2 |
| 115 | Me | 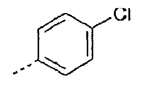 | | (ESI)+:[M+H]+=4 92.6, 494.2 |
| 116 | Me | 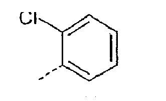 | | (ESI)+:[M+H]+=4 92.2, 494.2 |
| 117 | Me | 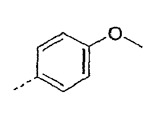 | | (ESI)+:[M+H]+=4 88.7 |
| 118 | Me | 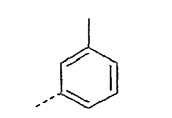 | | (ESI)+:[M+H]+=4 72.7 |
| 119 | Me | 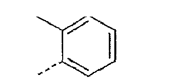 | | (ESI)+:[M+H]+=4 72.7 |
| 120 | Me | 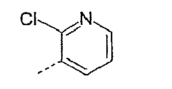 | | (ESI)+:[M+H]+=4 93.2 |
| 121 | Me | 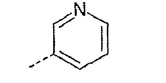 | | (ESI)+:[M+H]+=4 59.2 |
| 122 | Me | 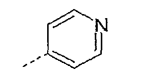 | | (ESI)+:[M+H]+=4 59.2 |
| 123 | Me | 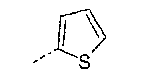 | | (ESI)+:[M+H]+=4 64.6 |
| 124 | Me | 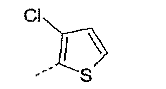 | | (ESI)+:[M+H]+=4 98.1 |
| 125 | Me | 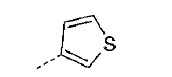 | | (ESI)+:[M+H]+=4 64.2 |
| 126 | Me | 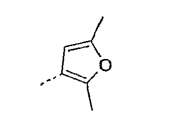 | | (ESI)+:[M+H]+=4 76.6 |
| 127 | Me | 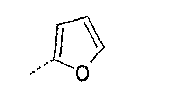 | | (ESI)+:[M+H]+=4 48.6 |
| 128 | Me | 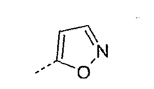 | | (ESI)+:[M+H]+=4 49.6 |
| 129 | Me | 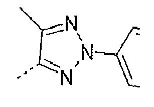 | | (ESI)+:[M+H]+=5 39.3 |
| 130 | Me | CH₂CH₃ | | (ESI)+:[M+H]+=4 10.7 |
| 131 | Me | CH₂CH₂CH₃ | | (ESI)+:[M+H]+=4 24.7 |
| 132 | Me | C(CH₃)₃ | | (ESI)+:[M+H]+=4 38.3 |
| 133 | Me | 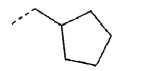 | | (ESI)+:[M+H]+=4 64.7 |
| 134 | Me | CH₂(CH₂)₃C H₃ | | (ESI)+:[M+H]+=4 52.8 |
| 135 | Me | CH(CH₂CH₃) CH₂CH₂CH₂ CH₃ | | (ESI)+:[M+H]+= (ESI)+:[M+H]+=4 80.8 |
| 136 | Me | 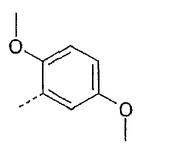 | | (ESI)+:[M+H]+=5 32.3 |
| 137 | Me | 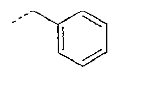 | | (ESI)+:[M+H]+=4 72.7 |
| 138 | Me | 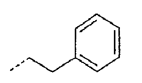 | | (ESI)+:[M+H]+=4 86.7 |
| 139 | Me | 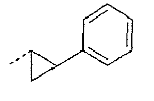 | | (ESI)+:[M+H]+=4 98.7 |
| 140 | Me | 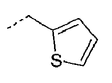 | | (ESI)+:[M+H]+=4 78.6 |
| 141 | Me | 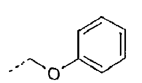 | | (ESI)+:[M+H]+=4 88.7 |
| 142 | Me | 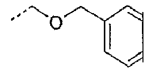 | | (ESI)+:[M+H]+=5 02.7 |
| 143 | Me | CH₂CO₂CH₂ CH₃ | | (ESI)+:[M+H]+=4 68.2 |
| 144 | Me | CH₂CH₂CO₂ CH₃ | | (ESI)+:[M+H]+=4 68.2 |
| 145 | Me | CH₂(CH₂)₂C O₂CH₂CH₃ | | (ESI)+:[M+H]+=4 96.7 |

| | | | | | |
|---|---|---|---|---|---|
| 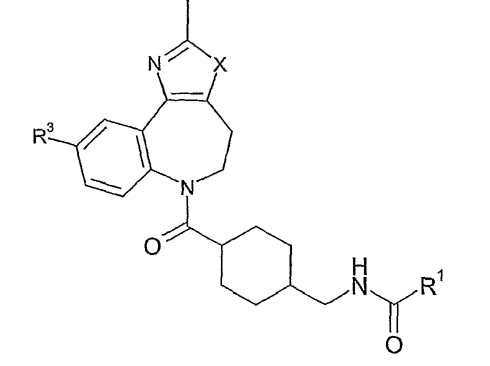 | | | | | |

| **Compound number** | **R¹** | **R³** | **X** | **MS (ESI)+: [M+H] + =** | **¹H NMR: δ (ppm)** |
|---|---|---|---|---|---|
| 146 | 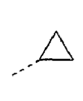 | Cl | s | 458.4, 460.4 | 0.51-0.98 (6H, m), 1.11-1.99 (7H, m), 2.30-2.41 (1H, m), 2.67 (3H, s), 2.83-3.08 (4H, m), 3.42-3.62 (1H, m), 5.55 (1H, s), 7.10 (1H, d, J=8.4Hz), 7.25 (1H, d d, J=2.5, 8.4Hz), 8.38 (1H, d, J=2.5Hz) |
| 147 | 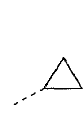 | Me | s | 438.2 | 0.55-0.62 (1H, m), 0.62-0.72 (2H, m), 0.72-0.85 (1H, m), 0.85-1.00 (2H, m), 1.10-1.45 (4H, m), 1.45-1.95 (4H, m), 2.35-2.50 (1H, m), 2.43 (3H, s), 2.68 (3H, s), 2.85-3.10 (4H, m), 3.45-3.65 (1H, m), 4.75-4.85 (1H, m), 5.45-5.65 (1H, m), 7.00-7.15 (2H, m), 8.14(1H, s) |

| | | |
|---|---|---|
| 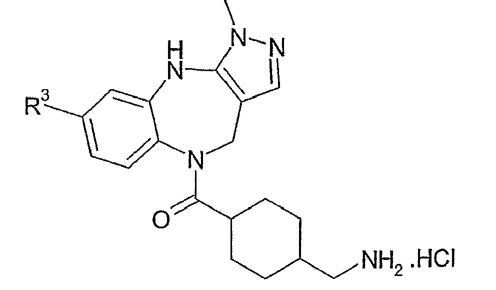 | | |

| **Compound number** | **R³** | **MS** **(ESI)+: [M+H]+=** |
|---|---|---|
| 148 E10.2 | Cl | |
| 149 | Me | 354.2 |
| 150 | H | 340.2 |

| | | |
|---|---|---|
| 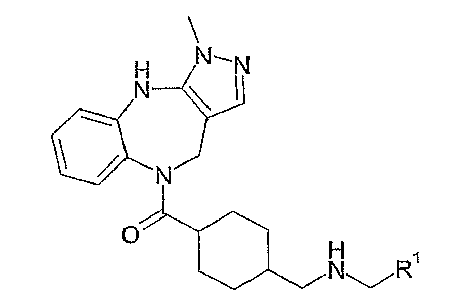 | | |

| **Compound number** | **R⁸** | **MS** **(ESI)+: [M+H]+=** |
|---|---|---|
| 151 E12 | CH(CH₃)₂ | 396.4 |
| 152 | CH₂CH₃ | 396.4 |
| 153 | 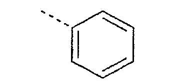 | 430.4 |
| 154 | 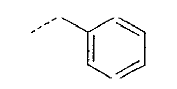 | 444.4 |
| 155 | 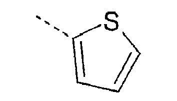 | 436.1 |
| 156 | 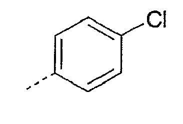 | 464.2, 466.2 |
| 157 | 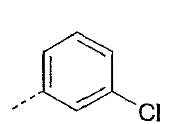 | 464.3, 466.3 |
| 158 | CH₂CH₂CH₂CH₃ | 410.4 |
| 159 | 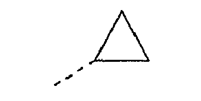 | 394.4 |
| 160 | 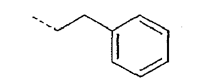 | 458.4 |
| 161 | CH₂CH(CH₃)₂ | 410.4 |
| 162 | 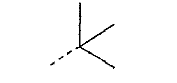 | 410.3 |
| 163 | 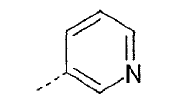 | 431.1 |
| 164 | 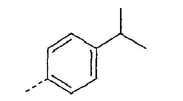 | 472.4 |
| 165 | 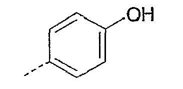 | 446.4 |
| 166 | 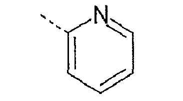 | 431.4 |
| 167 | 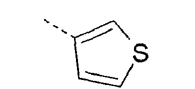 | 436.3 |
| 168 | CH₂CF₃ | 436.3 |
| 169 | 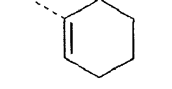 | 434.4 |
| 170 | 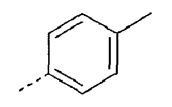 | 444.4 |
| 171 | 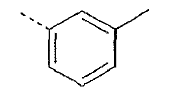 | 444.3 |
| 172 | 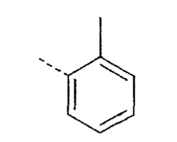 | 444.2 |
| 173 | 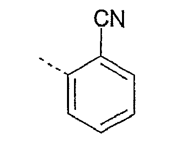 | 454.2 |
| 174 | 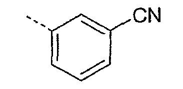 | 454.4 |
| 175 | 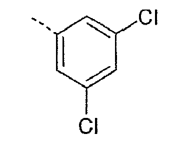 | 498.3 |
| 176 | 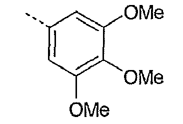 | 520.4 |
| 177 | 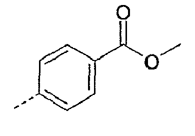 | 488.4 |

| | | |
|---|---|---|
| 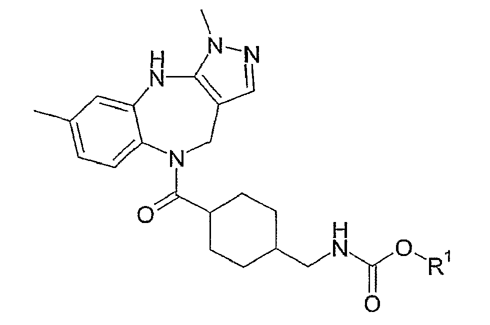 | | |

| **Compound number** | **R¹** | **MS** **(ESI)+: [M+H]+=** |
|---|---|---|
| 178 E13 | 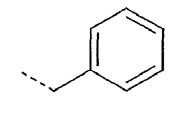 | 533.3 |
| 179 | 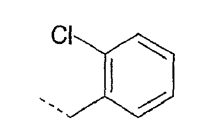 | 522.2, 524.2 |
| 180 | 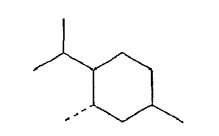 | 536.4 |
| 181 | 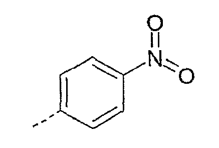 | 519.7 |
| 182 | 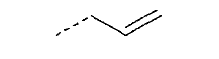 | 438.2 |
| 183 | 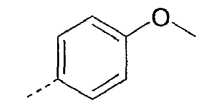 | 504.7 |
| 184 | 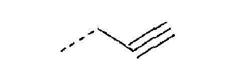 | 436.7 |
| 185 | 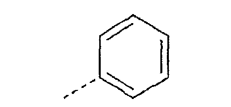 | 474.7 |
| 186 | 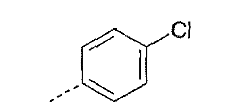 | 508.6 |
| 187 | 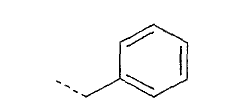 | 488.7 |
| 188 | 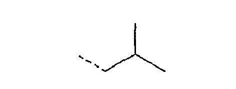 | 454.7 |
| 189 | 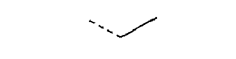 | 426.2 |
| 190 | 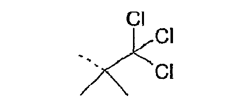 | 556.2, 558.2 |
| 191 | | 412.2 |
| 192 | 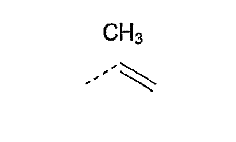 | 424.2 |
| 193 | 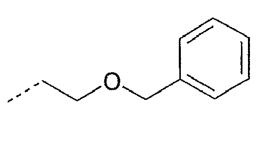 | 532.3 |

| | | |
|---|---|---|
| 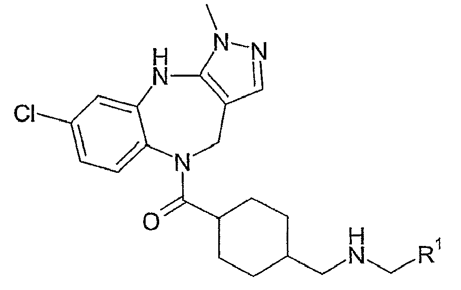 | | |

| **Compound number** | **R¹** | **MS** **(ESI)+: [M+H]+=** |
|---|---|---|
| 194 E14 | CH₂CH₂CH₂CH₂CH₃ | 458.4 |
| 195 | CH₂CH₃ | 416.4, 418.4 |
| 196 | (CH₂)₂CN | 441.5, 443.5 |
| 197 | 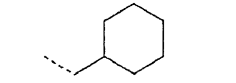 | 484.4, 486.4 |
| 198 | 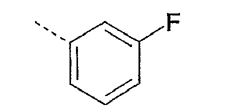 | 482.4, 484.4 |
| 199 | 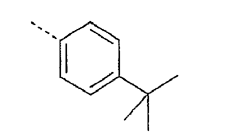 | 520.6, 522.6 |
| 200 | 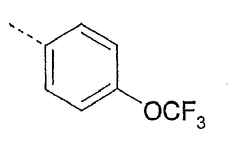 | 548.5, 550.5 |
| 201 | 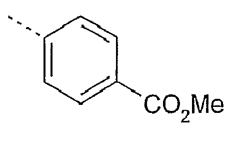 | 522.6, 524.6 |
| 202 | 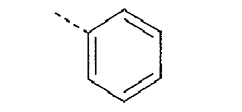 | 464.5, 466.5 |
| 203 | 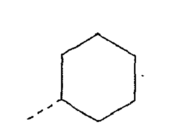 | 470.5, 472.5 |
| 204 | CH₂OH | 418.3, 420.3 |
| 205 | CO₂CH(CH₃)₂ | 474.4, 476.4 |
| 206 | 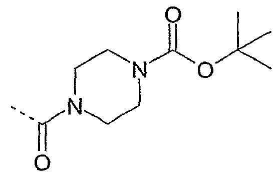 | 600.6, 602.6 |
| 207 | CH₂CH(CH₃)₂ | 444.5, 446.5 |
| 208 | CH₂CH₂OH | 432.5 |
| 209 | CN | 413.4, 415.5 |
| 210 | 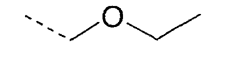 | 446.5, 448.5 |
| 211 | 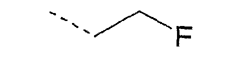 | 434.3, 436.3 |
| 212 | 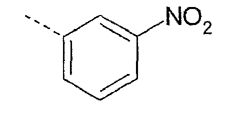 | 509.5, 511.5 |
| 213 | 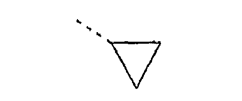 | 428.4, 430.4 |
| 214 | 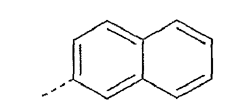 | 514.6, 516.6 |
| 215 | 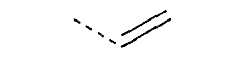 | 414.4, 416.5 |
| 216 | 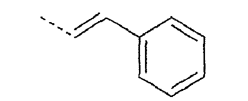 | 490.6, 492.6 |
| 217 | 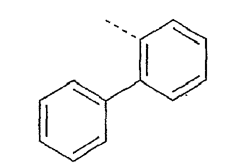 | 540.6, 542.6 |
| 218 | 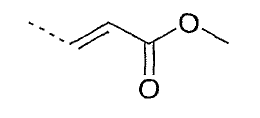 | 472.3, 474.4 |
| 219 | 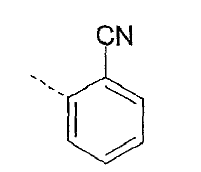 | 489.4, 491.4 |
| 220 | 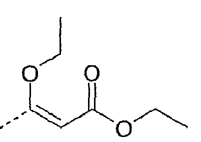 | 530.5, 532.5 |
| 221 | COC(CH₃)₃ | 472.5, 474.5 |
| 222 | CONH₂ | 431.5, 433.5 |
| 223 | 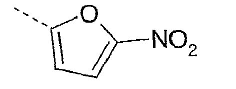 | 499.5, 501.5 |
| 224 | 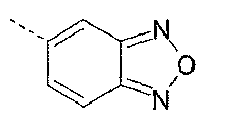 | 506.6, 508.6 |

| | | | | | |
|---|---|---|---|---|---|
| 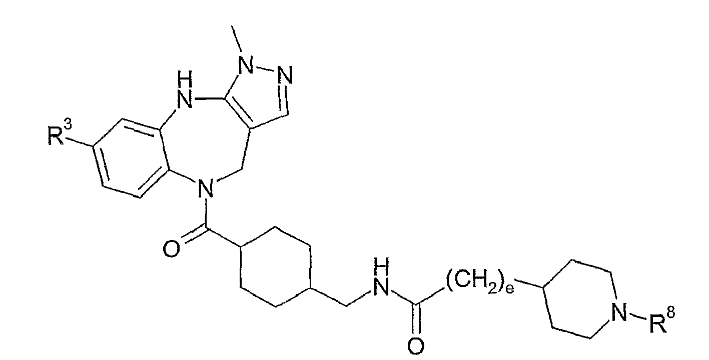 | | | | | |

| **Compound number** | **R³** | **e** | **R⁸** | **MS** | **¹H NMR:** δ **(ppm)** |
|---|---|---|---|---|---|
| 225 E16 | H | 0 | CH₂CH₂C(CH₃)₃ | (ESI)+:[ M+H]+= 535.5 | 0.45-0.80 (2H, m), 0.88 (9H, s), 1.20-1.62 (7H, m), 1.62-2.19 (10H, m), 2.33-2.46 (2H, m), 3.72 (3H, s), 3.79 (1H, d, J=14.6Hz), 5.61 (1H, d, J=14.6Hz), 5.63-5.70 (1H, m), 6.10 (1H, s), 7.02 (2H, t, J=8.4Hz), 7.10-7.19 (3H, m), 7.26-7.28 (2H, m) |
| 226 | Cl | 2 | CO₂C(CH₃)₃ | (ESI)+:[ M+H]+= 613.4, 615.4 | 0.43-0.62 (1H, m), 0.64-0.83 (1H, m), 0.91-1.14 (2H, m), 1.22-1.81 (11H, m), 1.44 (9H, s), 1.98-2.07 (2H, m), 2.16 (2H, t, J=7.7Hz), 2.61 (2H, brs), 2.98 (2H, d, J=5.4Hz), 3.70 (3H, s), 3.75 (1H, d, J=14.6Hz), 4.02 (2H, d, J=12.4Hz), 5.55 (1H, d, J=14.6Hz), 5.95 (1H, t, J=5.2Hz), 6.96 (1H, dd, J=2.0, 8.4Hz), 7.04 (1H, d, J=8.4Hz), 7.09-7.13 (2H, m), 7.35 (1H, s) |
| 227 | H | 0 | CO₂C(CH₃)₃ | | d4-MeOH δ 0.46-0.80 (2H, m), 1.13-1.84 (11H, m), 1.44 (9H, s), 2.13-2.37 (2H, m), 2.74 (2H, brs), 2.89 (2H, t, J=6.2Hz), 3.72 (3H, s), 3.80 (1H, d, J=14.6Hz), 4.07 (2H, d, J=13.4Hz), 5.47 (1H, d, J=14.6Hz), 7.01-7.09 (1H, m), 7.14 (1H, s), 7.16-7.22 (1H, m), 7.26-7.37 (2H, m), 7.82-7.92 (2H, m) |
| 228 | M e | 1 | CO₂C(CH₃)₃ | | 0.48-0.61 (1H, m), 0.63-1.81 (1H, m), 0.92-1.14 (2H, m), 1.16-1.30 (2H, m), 1.42 (9H, s), 1.50-2.21 (10H, m), 2.31 (3H, s), 2.53-2.72 (2H, m), 2.95 (2H, t, J=5.9Hz), 3.31-3.53 (1H, m), 3.67 (3H, s), 3.75 (1H, d, J=14.6Hz), 3.98-4.03 (2H, m), 5.53 (1H, d, J=14.6Hz), 6.10 (1H, brt), 6.71 (1H, s), 6.80 (1H, d, J=7.9Hz), 6.86 (1H, s), 6.97 (1H, d, J=7.9Hz), 7.10 (1H, s) |
| 229 | H | 0 | 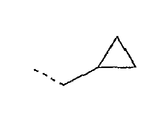 | (ESI)+:[ M+H]+= 505.5 | 0.19 (2H, d, J=4.7Hz), 0.41-0.80 (4H, m), 0.80-0.99 (2H, m), 1.06-1.60 (6H, m), 1.61-1.93 (4H, m), 2.18-2.30 (2H, m), 2.30-2.45 (2H, m), 2.48 (2H, d, J=6.7Hz), 2.91 (2H, t, J=6.4Hz), 3.16-3.30 (2H, m), 3.68 (3H, s), 3.78 (1H, d, J=14.6Hz), 5.54 (1H, d, J=14.6Hz), 6.42-6.74 (1H, m), 6.95-7.18 (4H, m), 7.20-7.30 (2H, m) |
| 230 | Cl | 0 | CH₂CH₂C(CH₃)₃ | (APCI)+ [M+H]+ =569.5, 571.5 | 0.42-0.82 (2H, m), 0.86 (9H, s), 1.19-2.14 (17H, m), 2.28-2.34 (2H, m), 2.86-3.00 (4H, m), 3.65 (3H, s), 3.76 (1H, d, J=14.6Hz), 5.52 (1H, d, J=14.6Hz), 6.01-6.03 (1H, m), 6.94 (1H, dd, J=2.2, 8.4Hz), 7.03 (1H, d, J=8.4Hz), 7.10-7.11 (2H, m), 7.72 (1H, s) |
| 231 | Cl | 0 | 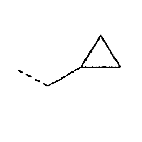 | (APCI)+ :[M+H]+ =539.4, 541.4 | d₄-MeOH: 0.37-0.43 (2H, m), 0.57-0.80 (4H, m), 1.05-2.01 (12H, m), 2.15-2.24 (1H, m), 2.44-2.52 (1H, m), 2.91-3.04 (6H, m), 3.61-3.69 (2H, m), 3.71 (3H, s), 3.80 (1H, d, J=14.6Hz), 5.43 (1H, d, J=14.6Hz), 7.03 (1H, dd, J=2.2, 8.4Hz), 7.15 (1H, s), 7.20 (1H, d, J=8.4Hz), 7.33 (1H, d, J=2.2Hz) |
| 232 | M e | 0 | CH₂CH₂C(CH₃)₃ | (APCI)+ :[M+H]+ =549.5 | 0.51-0.82 (2H, m), 0.86 (9H, s), 1.12-1.84 (13H, m), 1.91-2.11 (4H, m), 2.20-2.36 (5H, m), 2.94-3.02 (4H, m), 3.66 (3H, s), 3.75 (1H, d, J=14.6Hz), 5.55 (1H, d, J=14.6Hz), 6.53 (1H, s), 6.80 (1H, d, J=7.9Hz), 6.85 (1H, s), 6.97 (1H, d, J=7.9Hz), 7.12 (1H, s) |
| 233 | M e | 0 | 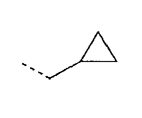 | (APCI)+ :[M+H]+ =519.4 | d₄-MeOH: 0.39-0.76 (6H, m), 1.05-2.02 (16H, m), 2.20-2.26 (1H, m), 2.34 (3H, s), 2.44-2.50 (1H, m), 2.88-3.00 (4H, m), 3.69 (3H, s), 3.75 (1H, d, J=14.6Hz), 5.43 (1H, d, J=14.6Hz), 6.86-6.89 (1H, m), 7.03-7.13 (3H, m), 7.79 (1H, t, J=5.9Hz), 7.85 (1H, s) |

| | | | | | |
|---|---|---|---|---|---|
| 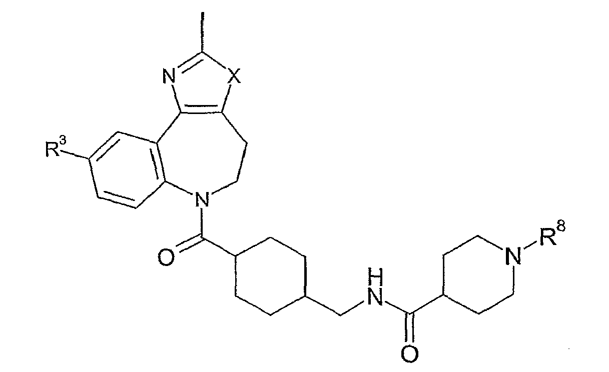 | | | | | |

| **Compound number** | **R³** | **R⁸** | **X** | **MS** | ¹**H NMR:** δ **(ppm)** |
|---|---|---|---|---|---|
| 234 | Cl | CH₂CH₂C(CH₃)₃ | S | 585.5, 587.6 | 0.50-0.56 (1H, m), 0.88 (9H, s), 1.11-1.19 (2H, m), 1.35-1.49 (4H, m), 1.56-2.10 (9H, m), 2.28-2.34 (3H, m), 2.67 (3H, s), 2.83-3.12 (6H, m), 3.42-3.60 (2H, m), 4.76-4.83 (1H, m), 5.39-5.44 (1H, m), 7.10 (1H, d, J=8.4Hz), 7.26 (1H, dd, J=2.5, 8.4Hz), 8.38 (1H, d, J=2.5Hz) |
| 235 | Me | CH₂CH₂C(CH₃)₃ | S | 565.4 | 0.42-0.62 (1H, m), 0.72-0.84 (1H, m), 0.87 (9H, s), 1.08-1.20 (2H, m), 1.30-1.50 (4H, m), 1.56-1.75 (6H, m), 1.75-1.95 (5H, m), 1.96-2.10 (1H, m), 2.25-2.35 (1H, m), 2.43 (3H, s), 2.69 (3H, s), 2.85-3.05 (6H, m), 3.40-3.65 (1H, m), 4.75-4.85 (1H, m), 5.35-5.45 (1H, m), 7.00-7.15 (2H, m), 8.15 (1H, s) |

| | | | | |
|---|---|---|---|---|
| 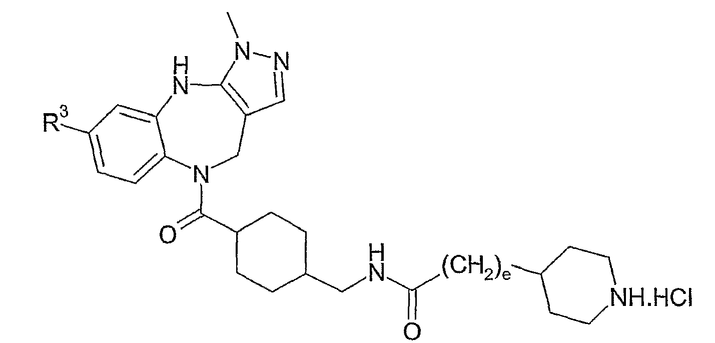 | | | | |

| **Compound number** | **R³** | **e** | **MS** | **¹H NMR:** δ **(ppm)** |
|---|---|---|---|---|
| 236 E17 | Me | 1 | (APCI) +:[M+ H]+= 579.5 | 0.44-0.68 (1H, m), 0.69-0.87 (1H, m), 1.19-2.32 (15H, m), 2.40 (3H, s), 2.88-3.03 (4H, m), 3.28-3.43 (4H, m), 3.52-3.75 (1H, m), 3.84 (1H, d, J=14.6Hz), 3.96 (3H, s), 5.50 (1H, d, J=14.6Hz), 7.07 (1H, d, J=7.9Hz), 7.22 (1H, d, J=7.9Hz), 7.28 (1H, s), 7.96 (1H, s) |
| 237 | Cl | 2 | (ESI)+ [M+H] +=513 .4, 515.4 | CD₃OD δ 0.54-0.70 (1H, m), 0.72-0.89 (1H, m), 1.12-1.98 (14H, m), 2.20-2.29 (3H, m), 2.89-2.98 (4H, m), 3.30-3.39 (2H, m), 3.87 (1H, d, J=14.6Hz), 3.98 (3H, s), 5.51 (1H, d, J=14.6Hz), 7.24 (1H, dd, J=2.2, 8.4Hz), 7.37 (1H, d, J=8.4Hz), 7.56 (1H, d, J=2.2Hz), 7.91 (1H, s), 8.00 (1H, s) |
| 238 | H | 0 | (ESI)+ [M+H] += 451.3 | CD₃OD δ 0.44-0.63 (1H, m), 0.66-0.83 (1H, m), 1.10-2.03 (11H, m), 2.21-2.31 (1H, m), 2.47-2.61 (1H, m), 2.89-2.92 (2H, m), 2.98-3.14 (2H, m), 3.34-3.42 (2H, m), 3.87 (1H, d, J=14.6Hz), 3.99 (3H, s), 5.53 (1H, d, J=14.6Hz), 7.24 (1H, dd, J=2.0, 8.4Hz), 7.32 (1H, d, J=8.4Hz), 7.38-7.53 (2H, m), 7.98 (1H, s) |

| | | | | |
|---|---|---|---|---|
| 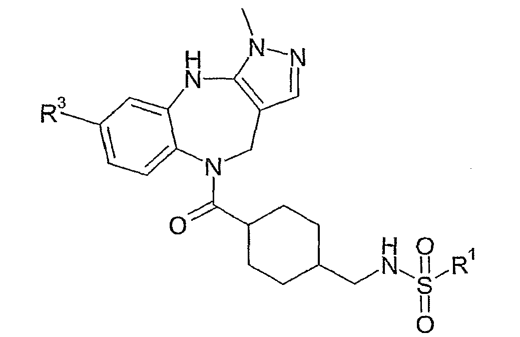 | | | | |

| **Compound number** | **R¹** | **R³** | **MS (ESI)+: [M+H]+=** | **¹H NMR:** δ **(ppm)** |
|---|---|---|---|---|
| 239 E18 | Me | Me | 432.1 | |
| 240 | CH(CH₃)₂ | Me | 460.2 | |
| 241 | 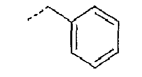 | Me | 508.2 | |
| 242 | 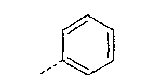 | Me | (494.2 | |
| 243 | 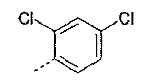 | Me | 562.2 | |
| 244 | 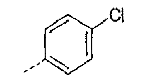 | Me | 528.2, 530.2 | |
| 245 | 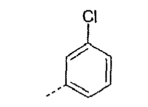 | Me | 528.2, 530.2 | |
| 246 | 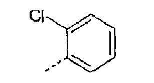 | Me | 528.2, 530.2 | |
| 247 | 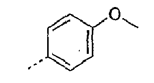 | Me | 524.2 | |
| 248 | 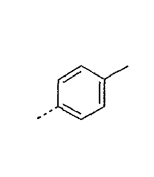 | Me | 508.7 | 0.50-0.80 (2H, m), 1.20-1.80 (7H, m), 2.05-2.20 (1H, m), 2.35 (3H, s), 2.40 (3H, s), 2.67 (2H, t, J=6.4Hz), 3.71 (3H, s), 3.75 (1H, d, J=14.5Hz), 4.29 (1H, t, J=6.5Hz), 6.59 (1H, d, J=14.5Hz), 5.71 (1H, s), 6.79 (1H, s), 6.79-6.89 (1H, m), 6.99 (1H, d, J=7.9Hz), 7.23-7.31 (2H, m), 7.66 (2H, d, J=8.2Hz) |
| 249 | 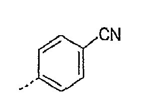 | Me | 519.7 | |
| 250 | 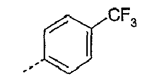 | Me | 562.2 | |
| 251 | 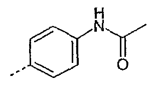 | Me | 551.3 | |
| 252 | 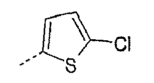 | Me | 534.1, 536.1 | |
| 253 | 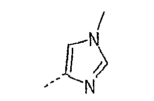 | Me | 498.2 | |
| 254 | | Me | 500.2 | |
| 255 | 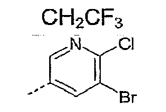 | Me | 607.1, 609.1 | |
| 256 | 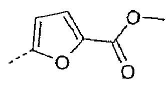 | Me | 542.2 | |
| 257 | CH₂CH₃ | Me | 446.1 | 0.55-0.90 (2H, m), 1.20-1.90 (10H, m) 2.08-2.20 (1H, m), 2.36 (3H, s), 2.86 (2H, tJ=6.0Hz)2.95-3.15 (2H, m), 3.71 (3H, s), 3.75 (1H, d, J=14.6Hz), 4.12-4.16 (1H, m), 5.60 (1H, d, J=14.6Hz), 5.77 (1H, s), 6.80 (1H, s), 6.83 (1H, d, J=7.9Hz), 7.01 (1H, d, J=7.9Hz), 7.17 (1H, s) |
| 258 | CH₂CH₂CH₃ | Me | 460.6 | |
| 259 | CH₂CH₂CH₂CH₃ | Me | 474.2 | |
| 260 | CH₂CH₂CH₂CH₃ | Cl | 494.2, 496.2 | 0.55-0.85 (2H, m), 0.92 (3H, t, J=7.3Hz), 1.25-1.85 (11H, m), 2.05-2.20 (1H, m), 2.80-3.00 (4H, m), 3.72 (3H, s), 3.76 (1H, d, J=14.6Hz), 4.10-4.20 (1H, m), 5.59 (1H, d, J=14.6Hz), 5.91 (1H, s), 7.00-7.10 (3H, m), 7.19(1H, s) |
| 261 | 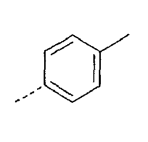 | Cl | 528.1, 530.1 | 0.50-0.80 (2H, m), 1.20-1.80 (7H, m), 2.00-2.15 (1H, m), 2.40 (3H, s), 2.60-2.75 (2H, m), 3.71 (3H, s), 3.75 (1H, d, J=14.6Hz), 4.64 (1H, t, J=6.5Hz), 5.57 (1H, d, J=14.6Hz), 6.29 (1H, s), 6.95-7.10 (3H, m), 7.16 (1H, s), 7.25-7.35 (2H, m), 7.71 (2H, d, J=8.2Hz) |
| 262 | 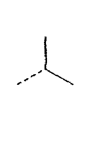 | H | 446.2 | 0.50-0.80 (1H, m), 1.30 (6H, d, J=6.7Hz), 1.32-1.48 (4H, m), 1.50-1.90 (5H, m), 2.10-2.20 (1H, m)2.75-3.00 (2H, m), 3.72 (3H, s), 3.81 (1H, d, J=14.6Hz), 4.25-4.45 (1H, m), 5.62 (1H, d, J=14.6Hz), 6.21 (1H, s), 6.95-7.10 (2H, m), 7.10-7.20 (2H, m), 7.25-7.30 (1H, m) |
| 263 | 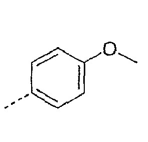 | H | 510.1 | 0.45-0.75 (1H, m), 1.20-1.85 (8H, m), 2.00-2.15 (1H, m), 2.60-2.70 (2H, m), 3.73 (3H, s), 3.79 (1H, d, J=14.6Hz), 3.84 (3H, s), 4.30-4.40 (1H, m), 5.60 (1H, d, J=14.6Hz), 5.90 (1H, s), 6.90-7.10 (4H, m), 7.10-7.20 (2H, m), 7.25-7.30 (1H, m), 7.70-7.80 (2H, m) |

| | | |
|---|---|---|
| 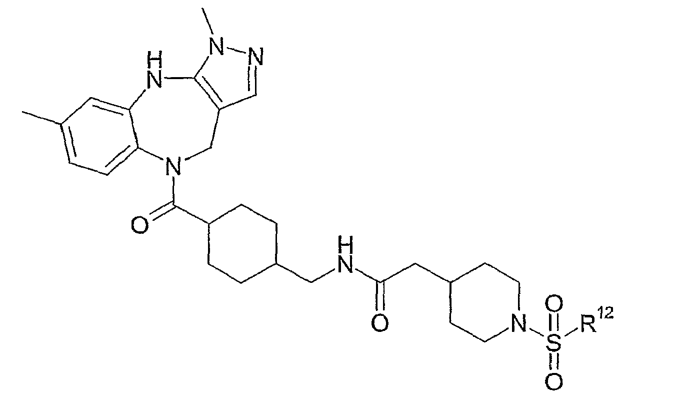 | | |

| **Compound number** | **R¹²** | **MS** |
|---|---|---|
| 264 E19 | Me | (ESI)+: [M+H]+= 557.4 |
| 265 | CH(CH₃)₂ | (ESI)+: [M+H]+= 585.4 |
| 266 | 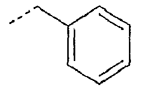 | (ESI)+: [M+H]+= 633.6 |
| 267 | 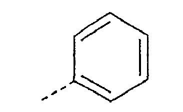 | (ESI)+: [M+H]+= 619.5 |
| 268 | 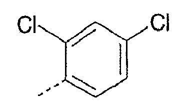 | (ESI)+: [M+H]+= 687.4, 689.4 |
| 269 | 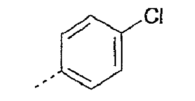 | (ESI)+: [M+H]+= 653.4, 655.4 |
| 270 | 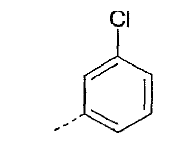 | (ESI)+: [M+H]+= 653.5, 655.5 |
| 271 | 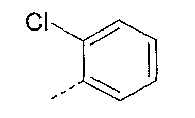 | (ESI)+: [M+H]+= 653.5, 655,5 |
| 272 | 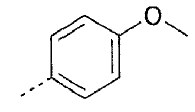 | (ESI)+: [M+H]+= 649.6 |
| 273 | 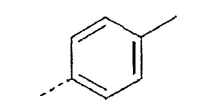 | (ESI)+: [M+H]+= 633.5 |
| 274 | 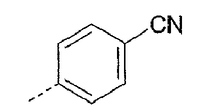 | (ESI)+: [M+H]+= 644.5 |
| 275 | 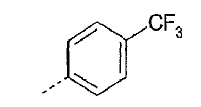 | (ESI)+: [M+H]+= 687.5 |
| 276 | 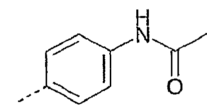 | (ESI)+: [M+H]+= 676.5 |
| 277 | 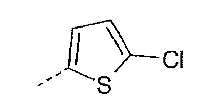 | (ESI)+: [M+H]+= 659.4, 661.4 |
| 278 | 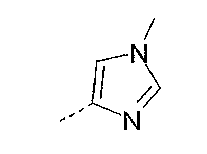 | (ESI)+: [M+H]+= 623.5 |
| 279 | CH₂CF₃ | (ESI)+: [M+H]+= 625.5 |
| 280 | 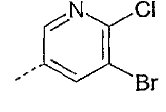 | (ESI)+: [M+H]+= 732.4, 734.4 |
| 281 | 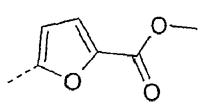 | (ESI)+: [M+H]+= 667.5 |
| 282 | CH₂CH₃ | (ESI)+: [M+H]+= 571.4 |
| 283 | CH₂CH₂CH₃ | (ESI)+: [M+H]+= 585.5 |
| 284 | CH₂CH₂CH₂CH₃ | (ESI)+: [M+H]+= 599.5 |

| | | |
|---|---|---|
| 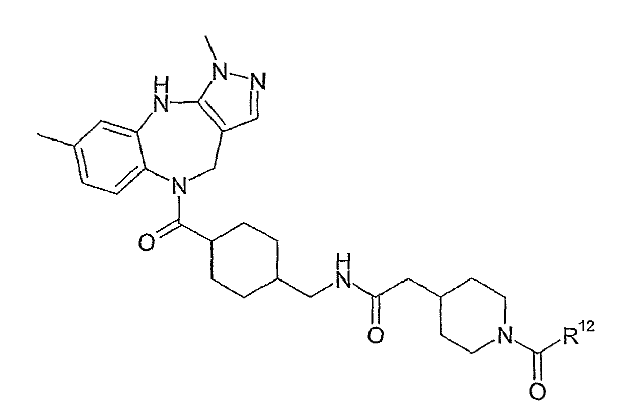 | | |

| **Compound number** | **R¹²** | **MS** |
|---|---|---|
| 285 E20 | Me | (ESI)+: [M+H]+= 521.5 |
| 286 | 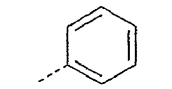 | (ESI)+: [M+H]+= 583.4 |
| 287 | 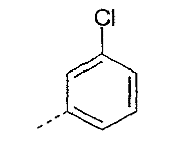 | (ESI)+: [M+H]+= 617.5, 619.5 |
| 288 | 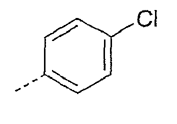 | (ESI)+: [M+H]+= 617.5, 619.5 |
| 289 | 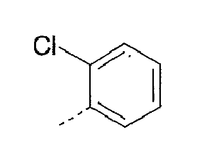 | (ESI)+: [M+H]+= 617.3, 619.3 |
| 290 | 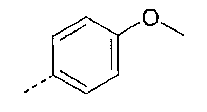 | (ESI)+: [M+H]+= 613.5 |
| 291 | 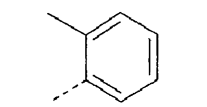 | (ESI)+: [M+H]+= 597.5 |
| 292 | 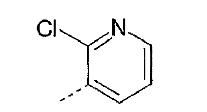 | (ESI)+: [M+H]+= 618.5, 620.5 |
| 293 | 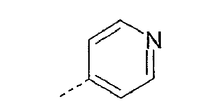 | (ESI)+: [M+H]+= 584.4 |
| 294 | 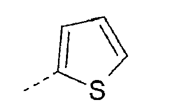 | (ESI)+: [M+H]+= 589.3 |
| 295 | 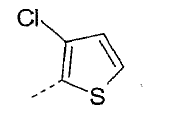 | (ESI)+: [M+H]+= 623.5, 625.5 |
| 296 | 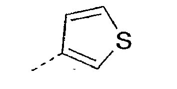 | (ESI)+: [M+H]+= 589.2 |
| 297 | 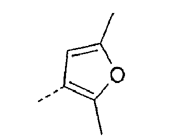 | (ESI)+: [M+H]+= 601.5 |
| 298 | 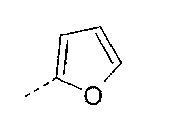 | (ESI)+: [M+H]+= 573.4 |
| 299 | 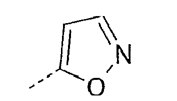 | (ESI)+: [M+H]+= 574.4 |
| 300 | 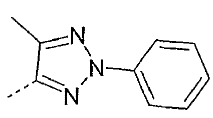 | (ESI)+: [M+H]+= 664.6 |
| 301 | CH₂CH₃ | (ESI)+: [M+H]+= 535.3 |
| 302 | CH₂CH₂CH₃ | (ESI)+: [M+H]+= 549.4 |
| 303 | 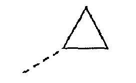 | (ESI)+: [M+H]+= 547.4 |
| 304 | | (ESI)+: [M+H]+= 563.5 |
| 305 | 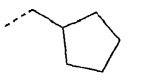 | (ESI)+: [M+H]+= 589.5 |
| 306 | CH₂(CH₂)₃CH₃ | (ESI)+: [M+H]+= 577.5 |
| 307 | CH(CH₂CH₃)CH₂CH₂C H₂CH₃ | (ESI)+: [M+H]+= 605.6 |
| 308 | 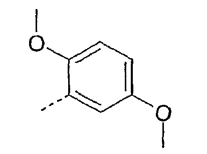 | (ESI)+: [M+H]+= 657.6 |
| 309 | 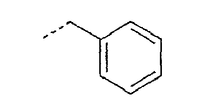 | (ESI)+: [M+H]+= 597.4 |
| 310 | 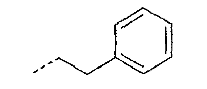 | (ESI)+: [M+H]+= 611.5 |
| 311 | 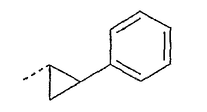 | (ESI)+: [M+H]+= 623.6 |
| 312 | 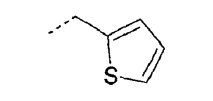 | (ESI)+: [M+H]+= 603.5 |
| 313 | 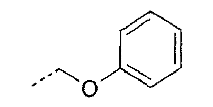 | (ESI)+: [M+H]+= 613.5 |
| 314 | 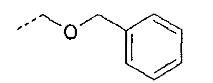 | (ESI)+: [M+H]+= 627.6 |
| 315 | CH₂CH₂CO₂CH₃ | (ESI)+: [M+H]+= 593.4 |
| 316 | CH₂(CH₂)₂CO₂CH₂CH₃ | (ESI)+: [M+H]+= 621.6 |

| | | |
|---|---|---|
| 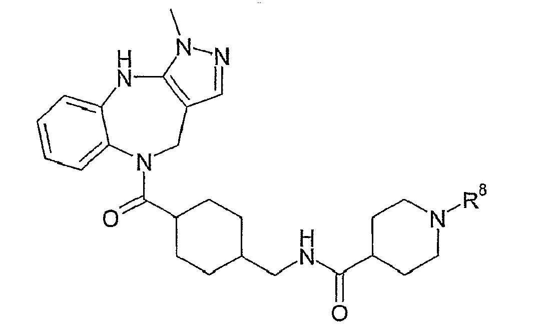 | | |

| **Compound number** | **R⁸** | **MS** |
|---|---|---|
| 317 E21 | | (ESI)+: [M+H]+= 507.4 |
| 318 | CH₂CH₂CH₃ | (ESI)+: [M+H]+= 493.5 |
| 319 | 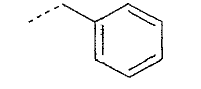 | (ESI)+: [M+H]+= 541.5 |
| 320 | 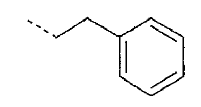 | (ESI)+: [M+H]+= 555.5 |
| 321 | 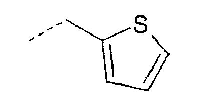 | (ESI)+: [M+H]+= 547.4 |
| 322 | CH₂CH₂CH₂CH₂CH₃ | (ESI)+: [M+H]+= 521.5 |
| 323 | 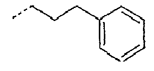 | (ESI)+: [M+H]+= 569.4 |
| 324 | CH₂CH₂CH(CH₃)₂ | (ESI)+: [M+H]+= 521.5 |
| 325 | 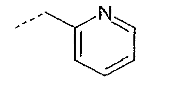 | (ESI)+: [M+H]+= 542.4 |
| 326 | 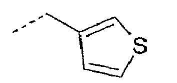 | (ESI)+: [M+H]+= 547.4 |
| 327 | CH₂CH₂CF₃ | (ESI)+: [M+H]+= 547.3 |
| 328 | 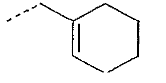 | (ESI)+: [M+H]+= 545.5 |
| 329 | CH₂CH₂OH | (ESI)+: [M+H]+= 495.6 |
| 330 | 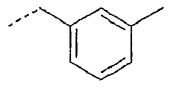 | (ESI)+: [M+H]+= 555.5 |
| 331 | 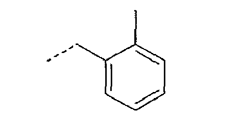 | (ESI)+: [M+H]+= 555.3 |
| 332 | 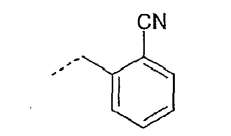 | (ESI)+: [M+H]+= 566.4 |
| 333 | 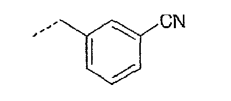 | (ESI)+: [M+H]+= 566.4 |
| 334 | 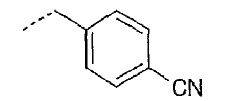 | (ESI)+: [M+H]+= 566.5 |
| 335 | 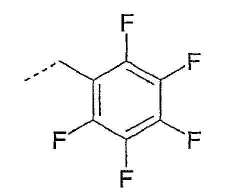 | (ESI)+: [M+H]+= 631.5 |
| 336 | 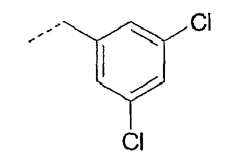 | (ESI)+: [M+H]+= 609.5 |
| 337 | 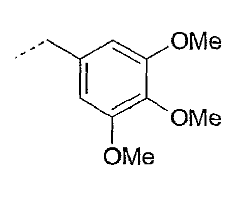 | (ESI)+: [M+H]+= 631.6 |

| | | |
|---|---|---|
| 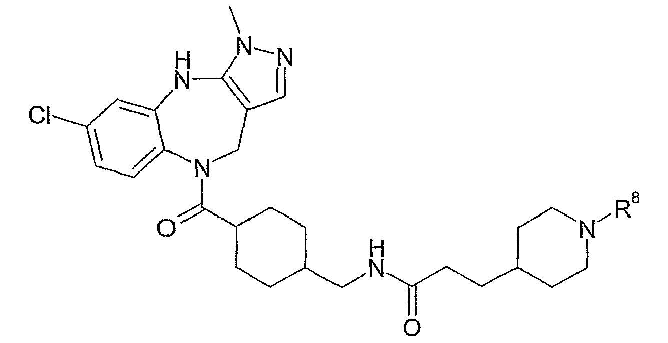 | | |

| **Compound number** | **R⁸** | **MS** |
|---|---|---|
| 338 E22 | | (ESI)+: [M+H]+= 597.6, 599.6 |
| 339 | CH₂CH₂CH₃ | (ESI)+: [M+H]+= 555.6, 557.6 |
| 340 | CH₂(CH₂)₂CN | (ESI)+: [M+H]+= 580.7, 582.7 |
| 341 | 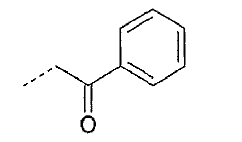 | (ESI)+: [M+H]+= 631.8, 633.8 |
| 342 | 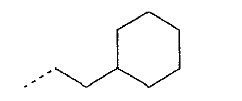 | (ESI)+: [M+H]+= 623.6, 625.6 |
| 343 | 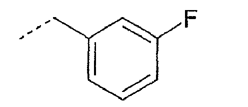 | (ESI)+: [M+H]+= 621.6, 623.6 |
| 344 | 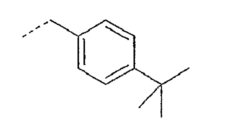 | (ESI)+: [M+H]+= 659.8, 661.8 |
| 345 | 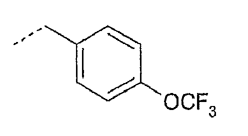 | (ESI)+: [M+H]+= 687.7, 689.7 |
| 346 | 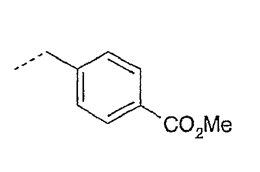 | (ESI)+: [M+H]+= 661.8, 663.8 |
| 347 | 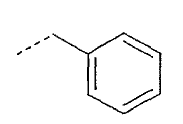 | (ESI)+: [M+H]+= 603.7, 605.7 |
| 348 | CH₂CH₂OH | (ESI)+: [M+H]+= 557.5, 559.5 |
| 349 | CH₂CO₂CH(CH₃)₂ | (ESI)+: [M+H]+= 613.6, 615.6 |
| 350 | CH₂CH₂CH(CH₃)₂ | (ESI)+: [M+H]+= 583.6, 585.6 |
| 351 | CH₂CH₂CH₂OH | (ESI)+: [M+H]+= 571.6, 573.6 |
| 352 | CH₂CN | (ESI)+: [M+H]+= 552.6, 554.6 |
| 353 | 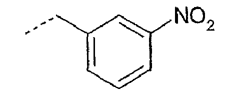 | (ESI)+: [M+H]+= 648.7, 650.7 |
| 354 | 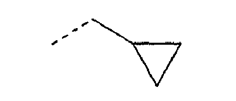 | (ESI)+: [M+H]+= 567.7, 569.6 |
| 355 | 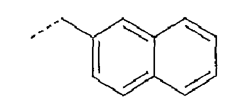 | (ESI)+: [M+H]+= 653.8, 655.8 |
| 356 | 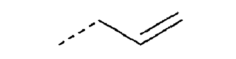 | (ESI)+: [M+H]+= 553.6, 555.6 |
| 357 | 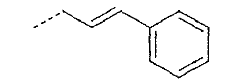 | (ESI)+: [M+H]+= 629.8, 631.8 |
| 358 | 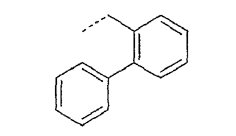 | (ESI)+: [M+H]+= 679.8, 681.8 |
| 359 | 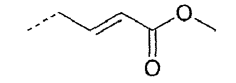 | (ESI)+: [M+H]+= 611.6, 613.6 |
| 360 | 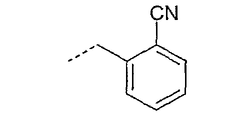 | (ESI)+: [M+H]+= 628.7, 630.7 |
| 361 | CH₂COC(CH₃)₃ | (ESI)+: [M+H]+= 611.7, 613.7 |
| 362 | CH₂CONH₂ | (ESI)+: [M+H]+= 570.6, 572.6 |
| 363 | 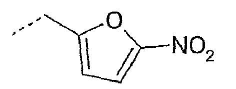 | (ESI)+: [M+H]+= 638.8, 640.8 |
| 364 | 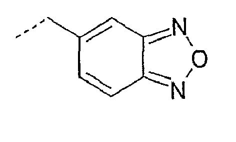 | (ESI)+: [M+H]+= 645.8, 647.8 |

| | | |
|---|---|---|
| 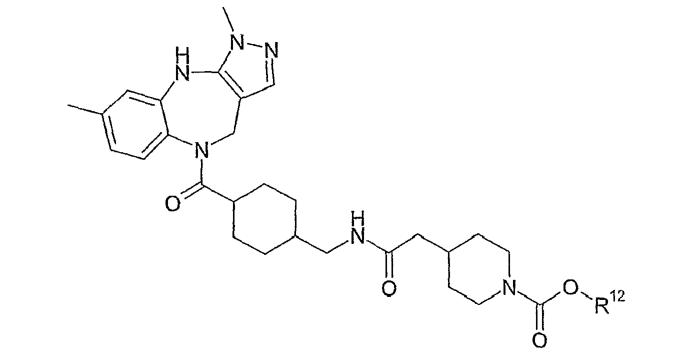 | | |

| **Compound number** | **R¹²** | **MS** |
|---|---|---|
| 365 E23 | 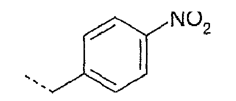 | (ESI)+: [M+H]+= 658.5 |
| 366 | 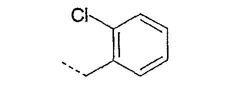 | (ESI)+: [M+H]+ = 647.5 |
| 367 | 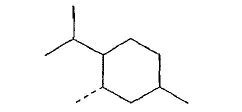 | (ESI)+: [M+H]+ = 661.7 |
| 368 | 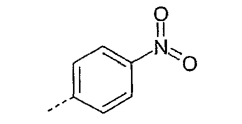 | (ESI)+: [M+H]+ = 644.5 |
| 369 | 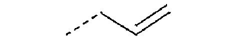 | (ESI)+: [M+H]+ = 563.5 |
| 370 | 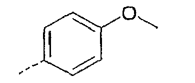 | (ESI)+: [M+H]+ = 629.6 |
| 371 | 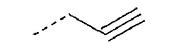 | (ESI)+: [M+H]+ = 561.4 |
| 372 | 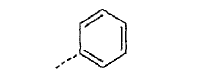 | (ESI)+: [M+H]+ = 599.4 |
| 373 | 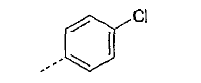 | (ESI)+: [M+H]+ = 633.5 |
| 374 | 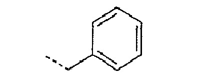 | (ESI)+: [M+H]+ = 613.5 |
| 375 | 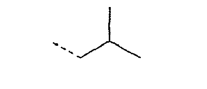 | (ESI)+: [M+H]+ = 579.5 |
| 376 | 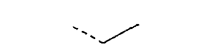 | (ESI)+: [M+H]+ = 551.5 |
| 377 | 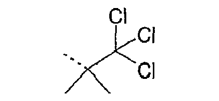 | (ESI)+: [M+H]+ = 683.5 |
| 378 | CH₃ | (ESI)+: [M+H]+ = 537.4 |
| 379 | 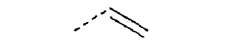 | (ESI)+: [M+H]+ = 549.4 |
| 380 | 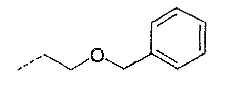 | (ESI)+: [M+H]+ = 657.6 |

| | | | | |
|---|---|---|---|---|
| 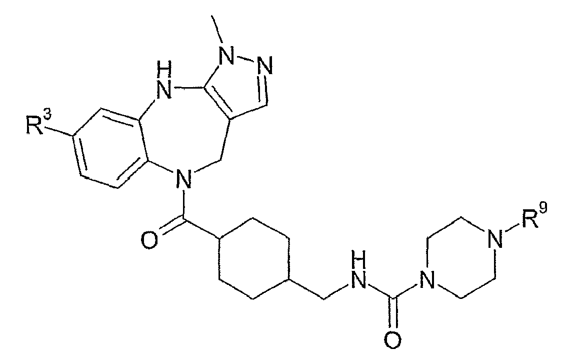 | | | | |

| **Compound number** | **R³** | **R⁹** | **MS** | **¹H NMR:** δ **(ppm)** |
|---|---|---|---|---|
| 381 E24 | Me | CH₂CH₂C(CH₃)₃ | (APCI)+:[ M+H]+= 550.4 | 0.41-0.82 (2H, m), 0.86 (9H, s), 1.33-1.50 (7H, m), 1.73 (2H, m), 2.12 (1H, m), 2.27-2.39 (6H, m), 2.29 (3H, s), 2.90-2.93 (2H, m), 3.31-3.35 (4H, m), 3.64 (3H, s), 3.74 (1H, d, J=14.6Hz), 4.78 (1H, m), 5.54 (1H, d, J=14.6Hz), 6.77 (1H, dd, J=1.0, 8.0Hz), 6.88 (1H, s), 6.95 (1H, d, J=8.0Hz), 7.03 (1H, d, J=1.0Hz), 7.11 (1H, s) |
| 382 | Me | CH₂CH₂CH₂SCH₃ | (ESI)+: [M+H]+= 554.6 | - |
| 383 | Me | CH₂CH₂OCH₃ | (ESI)+: [M+H]+= 524.7 | - |
| 384 | Me | CH₂CH₂CH₂CF₃ | (ESI)+: [M+H]+= 576.6 | - |
| 385 | Me | CH₂CH₂N(CH₂CH₃)₂ | (ESI)+: [M+H]+= 565.6 | - |
| 386 | Me | CH₂CH₂CH₂F | (ESI)+: [M+H]+= 526.6 | - |
| 387 | Me | CH₂CH₂CH₂OH | (ESI)+: [M+H]+= 524.6 | - |
| 388 | Me | CH₂C(CH₃)₃ | (ESI)+: [M+H]+= 536.5 | 0.55-0.84 (2H, m), 0.84 (9H, s), 1.18-1.75 (7H, m), 2.04-2.16 (3H, m), 2.34 (3H, s), 2.44-2.48 (4H, m), 2.93-2.97 (2H, m), 3.26-3.29 (4H, m), 3.70 (3H, s), 3.75 (1H, d, J=14.8Hz), 4.35-4.39 (1H, bs), 5.58 (1H, d, J=14.6Hz), 5.84 (1H, s), 6.79-6.83 (2H, m), 6.98 (1H, m), 7.16 (1H, s) |
| 389 | Me | 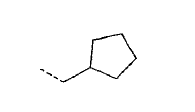 | (APCI)+: [M+H]+= 548.5 | 0.46-0.71 (2H, m), 1.11-1.27 (4H, m), 1.37-1.59 (7H, m), 1.69-1.78 (4H, m), 1.95-2.17 (2H, m), 2.21 (2H, d, J=7.4Hz), 2.30 (3H, s), 2.32-2.36 (4H, m), 2.85-2.98 (2H, m), 3.29-3.32 (4H, m), 3.64 (3H, s), 3.74 (1H, d, J=14.6Hz), 4.67-4.71 (1H, m), 5.55 (1H, d, J=14.6Hz), 6.78 (1H, d, J=8.0Hz), 6.87-6.88 (2H, m), 6.95 (1H, d, J=8.0Hz), 7.11 (1H, s) |
| 390 | Me | CH₂CH₂CH(CH₃)₂ | (APCI)+: [M+H]+= 536.5 | 0.48-0.72 (2H, m), 0.86 (6H, t, J=6.4Hz), 1.18-1.38 (5H, m), 1.46-1.60 (3H, m), 1.68-1.79 (2H, m), 2.07-2.18 (1H, m), 2.28-2.38 (9H, m), 2.88-2.96 (2H, m), 3.31-3.34 (4H, m), 3.65 (3H, s), 3.75 (1H, d, J=14.6Hz), 4.71-4.75 (1H, m), 5.55 (1H, d, J=14.8Hz), 6.78 (1H, d, J=7,9Hz), 6.88 (1H, s), 6.96 (1H, d, J=7.9Hz), 7.04 (1H, dd, J=1.2, 7.9Hz), 7.12 (1H, s) |
| 391 | Me | CH₂CH(CH₃)₂ | (APCI)+: [M+H]+= 522.4 | 0.46-0.71 (2H, m), 0.85 (6H, t, J=6.4Hz), 1.19-1.27 (2H, m), 1.37-1.54 (3H, m), 1.68-1.78 (3H, m), 2.04 (2H, d, J=7.4Hz), 2.05-2.17 (1H, m) 2.29 (3H, s), 2.29-2.33 (4H, m), 2.87-2.98 (2H, m), 3.28-3.32 (4H, m), 3.64 (3H, s), 3.74 (1H, d, J=14.6Hz), 4.68-4.72 (1H, m), 5.55 (1H, d, J=14.6Hz), 6.78 (1H, d, J=8.0Hz), 6.88 (1H, s), 6.93 (1H, s), 6.95 (1H, d, J=8.0Hz), 7.11 (1H, s) |
| 392 | Me | CH₂CH₂CH₃ | (APCI)+: [M+H]+= 508.4 | 0.47-0.89 (2H, m), 0.87 (3H, t, J=7.2Hz), 1.18-1.27 (2H, m), 1.37-1.54 (5H, m), 1.68-1.78 (2H, m), 2.07-2.17 (1H, m), 2.23-2.26 (2H, m), 2.29 (3H, s), 2.34-2.38 (4H, m), 2.87-2.97 (2H, m), 3.30-3.34 (4H, m), 3.64 (3H, s), 3.74 (1H, d, J=14.6Hz), 4.72-4.76 (1H, m), 5.54 (1H, d, J=14.6Hz), 6.78 (1H, d, J=8.0Hz) 6.87-6.88 (2H, m), 6.95 (1H, d, J=8.0Hz), 7.11 (1H, s) |
| 393 | Cl | 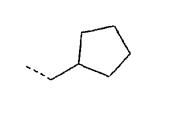 | (APCI)+: [M+H]+= 568.4, 570.4 | 0.41-0.56 (1H, m). 0.60-0.75 (1H, m), 0.78 (1H, d, J=6.4Hz), 0.94-1.14 (2H, m), 1.14-1.55 (9H, m), 1.57-1.72 (4H, m), 1.83-2.00 (2H, m), 2.18 (2H, d, J=6.9Hz), 2.29 (4H, t, J=4.9Hz), 2.70-2.90 (2H, m), 3.22 (4H, t, J=4.9Hz), 3.58 (3H, s), 3.66 (1H, d, J=14.6Hz), 5.17 (1H, t, J=5.4Hz), 5.38 (1H, d, J=14.6Hz), 6.86 (1H, d, J=2.2Hz), 6.91 (1H, s), 7.03 (1H, s), 7.12 (1H, d, J=2.2Hz) |
| 394 | Cl | CH₂CH₂CH(CH₃)₂ | (APCI)+: [M+H]+= 556.4, 558.4 | 0.40-0.59 (1H, m), 0.60-0.80 (1H, m), 0.86 (6H, d, J=6.7Hz), 1.09-1.65 (9H, m), 1.65-1.81 (2H, m), 2.01-2.17 (1H, m), 2.30-2.41 (2H, m), 2.41 (4H, t, J=4.9Hz) 2.92 (2H, t, J=5.9Hz), 3.38 (4H, t, J=4.9Hz), 3.70 (3H, s), 3.77 (1H, d, J=14.6Hz), 5.07 (1H, t, J=5.4Hz) 5.52 (1H, d, J=14.6Hz), 6.94 (1H, d, J=2.2Hz), 7.02 (1H, s), 7.12 (1H, s), 7.32 (1H, d, J=2.2Hz) |
| 395 | Cl | CH₂CH(CH₃)₂ | (APCI)+: [M+H]+= 542.4, 544.4 | 0.38-0.60 (1H, m), 0.60-0.78 (1H, m), 0.82 (6H, d, J=6.7Hz), 1.10-1.40 (2H, m), 1.40-1.56 (2H, m), 1.63-1.80 (4H, m), 2.03 (2H, d, J=7.4Hz), 2.12 (2H, s), 2.31 (4H, t, J=4.9Hz), 2.85-2.94 (2H, m), 3.30 (4H, t, J=4.9Hz), 3.64 (3H, s), 3.72 (1H, d, J=14.6Hz), 4.75 (1H, t, J=5.7Hz), 5.49 (1H, d, J=14.6Hz), 6.93 (1H, d, J=2.2Hz), 6.98 (1H, s), 7.11 (1H, s), 7.15 (1H, d, J=2.2Hz) |
| 396 | Cl | CH₂CH₂CH₃ | (APCI)+: [M+H]+= 528.3, 530.3 | 0.39-0.60 (1H, m), 0.60-0.83 (1H, m), 0.88 (3H, t, J=7.4Hz), 1.17-1.63 (9H, m), 1.64-1.83 (2H, m), 2.00-2.15 (1H, m), 2.25-2.35 (2H, m), 2.40 (4H, t, J=4.9Hz), 2.90-3.00 (2H, m), 3.35 (4H, t, J=4.9Hz), 3.72 (3H, s), 4.59 (1H, t, J=5.7Hz), 5.54 (1H, d, J=14.6Hz), 6.90-7.04 (2H, m), 7.10-7.19 (2H, m) |
| 397 | Cl | 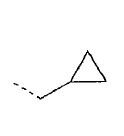 | (APCI)+: [M+H]+= 540.3, 542.3 | d₆-DMSO: 0.02-0.04 (2H, m), 0.41-0.47 (2H, m), 0.80-1.75 (10H, m), 2.03 (1H, m), 2.14 (2H, d, J=6.5Hz), 2.30-2.34 (4H, m), 2.74 (2H, m), 3.20-3.28 (4H, m), 3.67 (3H, s), 3.70 (1H, d, J=14.6Hz), 5.34 (1H, d, J=14.6Hz), 6.29 (1H, m), 7.02 (1H, dd, J=2.5, 8.0Hz), 7.08 (1H, s), 7.25 (1H, d, J=8.0Hz), 7.42 (1H, d, J=2.5Hz) |
| 398 | Me | 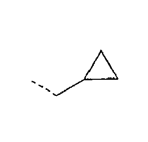 | (APCI)+: [M+H]+= 520.4 | 0.06-0.08 (2H, m), 0.46-0.53 (2H, m), 0.81 (1H, m), 1.13-1.79 (9H, m), 2.09 (1H, m), 2.23 (2H, m), 2.30 (3H, s), 2.45-2.49 (4H, m), 2.92 (2H, m), 3.33-3.37 (4H, m), 3.65 (3H, s), 3.74 (1H, d, J=14.6Hz), 4.69 (1H, m), 5.54 (1H, d, J=14.6Hz), 6.67 (1H, s), 6.78 (1H, dd, J=1.0, 8.0Hz), 6.86 (1H, d, J=1.0Hz), 6.96 (1H, d, J=8.0Hz), 7.11 (1H, s) |
| 399 | Cl | CH₂CH₂C(CH₃)₃ | (APCI)+: [M+H]+= 570.3, 572.3 | 0.42-0.81 (2H, m), 0.87 (9H, s), 1.22-1.40 (7H, m), 1.74-1.78 (2H, m), 2.08 (1H, m), 2.28-2.40 (6H, m), 2.87-2.92 (2H, m), 3.33- 3.37 (4H, m), 3.63 (3H, s), 3.74 (1H, d, J=14.6Hz), 4.89 (1H, m), 5.51 (1H, d, J=14.6Hz), 6.93 (1H, dd, J=2.2, 8.4Hz), 7.03 (1H, d, J=8.4Hz), 7.10 (1H, s), 7.20 (1H, d, J=2.2Hz), 8.10 (1H, s) |
| 400 | H | CH₂CH₂C(CH₃)₃ | (APCI)+: [M+H]+= 536.4 | 0.40-0.46 (1H, m), 0.63-0.68 (1H, m), 0.82 (9H, s), 1.26-1.67 (7H, m), 1.73-1.79 (2H, m), 2.03-2.08 (1H, m), 2.34-2.37 (6H, m), 2.82-2.94 (2H, m), 3.32 (4H, m), 3.64 (3H, s), 3.77 (1H, d, J=14.6Hz), 4.80 (1H, t, J=5.4Hz), 5.56 (1H, d, J=14.6Hz), 6.95 (1H, m), 7.05-7.12 (3H, m), 7.19 (1H, m), 7.32 (1H, s) |
| 401 | H | 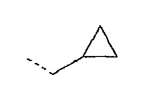 | (ESI)+: [M+H]+= 506.3 | 0.04-0.11 (2H, m), 0.46-0.53 (2H, m), 0.61-0.72 (1H, m), 0.72-0.89 (1H, m), 1.20-1.59 (6H, m), 1.69-1.81 (2H, m), 2.04-2.17 (1H, m), 2.23 (2H, d, J=6.4Hz), 2.46 (4H, t, J=4.9Hz), 2.81-3.05 (2H, m), 3.35 (4H, t, J=4.9Hz), 3.69 (3H, s), 3.79 (1H, d, J=14_{.}6Hz), 4.56 (1H, t, J=5.6Hz), 5.60 (1H, d, J=14.6Hz), 6.67 (1H, s), 6.96-7.26 (4H, m), 7.15 (1H, s) |
| 402 | H | 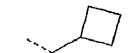 | (ESI)+: [M+H]+= 520.3 | - |

| | | | | | |
|---|---|---|---|---|---|
| 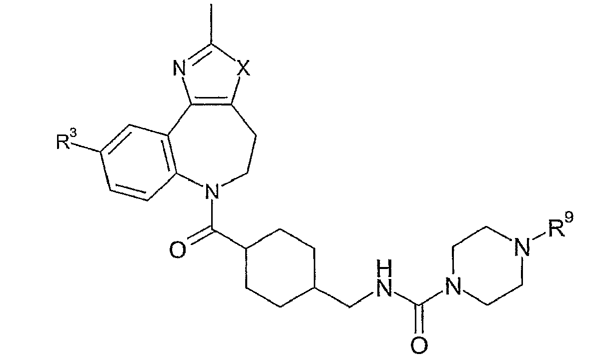 | | | | | |

| **Compound number** | **R³** | **R⁹** | **X** | **MS** | **¹H NMR:** δ **(ppm)** |
|---|---|---|---|---|---|
| 403 | Cl | CH₂CH₂C(CH₃)₃ | S | (ESI)⁺: [M+H]⁺ = 586.6, 588.6 | 0.49-0.60 (1H, m), 0.88 (9H, s), 1.11-1.24 (2H, m), 1.34-1.41 (4H, m), 1.47-1.87 (5H, m), 2.28-2.41 (5H, m), 2.67 (3H, s), 2.82-3.18 (4H, m), 3.29-3.33 (4H, m), 3.37-3.59 (1H, m), 4.34-4.38 (1H, m), 4.76-4.83 (1H, m), 7.10 (1H, dJ, =8.4Hz), 7.25 (1H, dd, J=2.5, 8.4Hz), 8.38 (1H, d, J=2.5Hz) |
| 404 | Me | 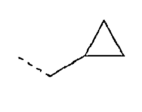 | S | (ESI)+: [M+H]+ = 536.3 | 0.05-0.15 (2H, m), 0.40-0.60 (3H, m), 0.70-0.90 (2H, m), 1.10-1.20 (2H, m), 1.45-1.95 (5H, m), 2.24 (2H, d, J=6.7Hz), 2.35-2.55 (8H, m), 2.69 (3H, s), 2.85-3.05 (4H, m), 3.30-3.40 (4H, m), 3.46-3.65 (1H, m), 4.32-4.38 (1H, m), 4.74-4.86 (1H, m), 7.00-7.15 (2H, m), 8.14(1H, s) |

| | | | | | |
|---|---|---|---|---|---|
| 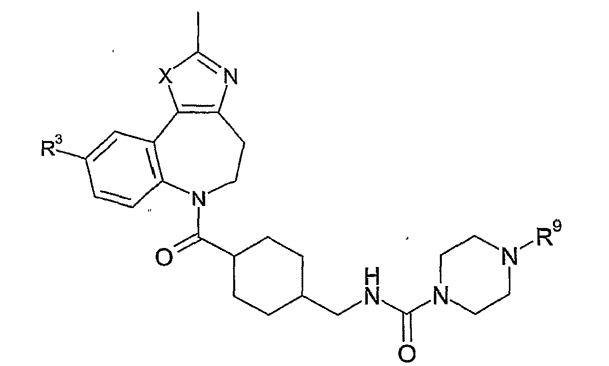 | | | | | |

| **Compound number** | **R³** | **R⁹** | **X** | **MS** | **¹H NMR:** δ **(ppm)** |
|---|---|---|---|---|---|
| 405 | Me | CH₂CH₂C(CH₃)₃ | NH | | 0.45-0.55 (1H, m), 0.75-0.90 (10H, m), 1.10-1.20 (1H, m), 1.20-1.90 (8H, m), 2.30-2.45 (13H, m), 2.65-3.00 (4H, m), 3.30-3.40 (5H, m), 4.35-4.45 (1H, m), 4.70-4.80 (1H, m), 6.95-7.15 (2H, m), 7.95-8.05 (1H, m) |

## Claims

1. A compound according to general formula 1c, or a compound which is a tautomer or a pharmaceutically acceptable salt thereof, 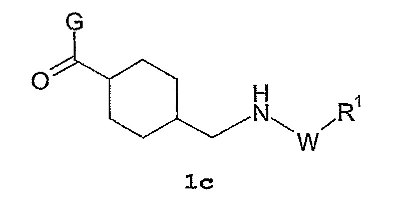 wherein G is a group selected among general formula 2c, 3c, 4c and 5c, 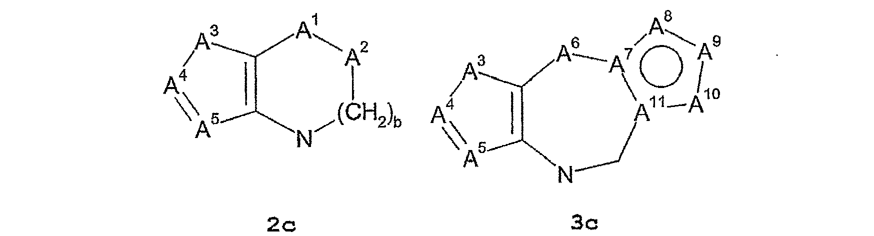 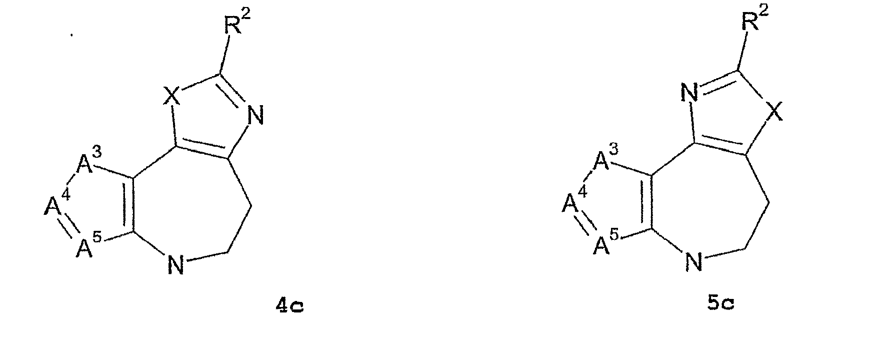 and wherein
A¹ is selected among CH₂, CH(OH), NH, N-alkyl, O and S;
A² is selected among CH₂, CH(OH), C(=O) and NH;
A³ is selected among S, NH, N-alkyl, -C(R³)=CH-, -C(R³)=N-, -N=C(R³)- and -CH=C(R³)-;
A⁴ is selected among C(R⁴) and N;
A⁵ is selected among C(R⁵) and N;
A⁶ is selected among CH₂, NH, N-alkyl and O;
A⁷ and A¹¹ are independently selected among C and N;
A⁸ and A⁹ are independently selected among CH, N, NH, N(CH₂)_{c}R⁶ and S; A¹⁰ is selected among -CH=CH-, CH, N, NH, N-(CH₂)_{c}-R⁶ and S;
wherein the ring constituted by A⁷, A⁸, A⁹, A¹⁰ and A¹¹ is aromatic; and
R¹ is selected among H, alkyl, alkenyl, alkyl-aryl, alkenyl-aryl, aryl, heteroaryl, -(CH₂)_{d}-R⁷, 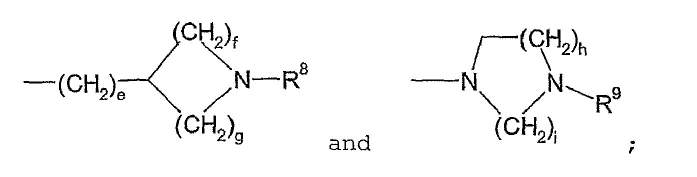 R² is selected among H, alkyl, aryl, heteroaryl and -(CH₂)ⱼ-R¹⁰;
R³, R⁴ and R⁵ are independently selected among H, alkyl, alkoxy, F, Cl, Br, CN, NH₂, NO₂, NH(alkyl) and N(alkyl)₂;
R⁶ and R⁷ are independently selected among H, alkyl, aryl, heteroaryl, F, OH, O-alkyl, S-alkyl, O-acyl, O-aryl, O-alkyl-aryl, NH₂, NH-alkyl, N(alkyl)₂, NH-acyl, N(alkyl)-acyl, CO₂H, CO₂-alkyl, CONH₂, CONH-alkyl, CON(alkyl)₂, CN and CF₃;
R⁸ and R⁹ are independently selected among H, alkyl, alkenyl, aryl, heteroaryl, - (CH₂)ₖ-R¹¹ and Y-R¹²;
R¹⁰ and R¹¹ are independently selected among H, alkyl, alkenyl, aryl, heteroaryl, F, OH, O-alkyl, S-alkyl, O-acyl, NH₂, NH-alkyl, N(alkyl)₂, NH-acyl, N(alkyl)-acyl, CO₂H, CO-alkyl, CO-aryl, CO₂-alkyl, CONH₂, CONH-alkyl, CON(alkyl)₂, alkenyl-CO₂-alkyl, alkenyl-aryl, CN and CF₃;
R¹² is selected among H, alkyl, aryl, heteroaryl and -(CH₂)₁-R¹³;
R¹³ is selected among H, alkyl, aryl, heteroaryl, F, OH, O-alkyl, S-alkyl, O-acyl, NH₂, NH-alkyl, N(alkyl)₂, NH-acyl, N(alkyl)-acyl, CO₂H, CO₂-alkyl, CONH₂, CONH-alkyl, CON(alkyl)₂, CN and CF₃;
W is selected among CH₂, C(=O), C(=O)-O, and S (=O)ₘ;
X is selected among O, S, NH and N-alkyl;
Y is selected among C(=O), C(=O)-O and -S(=O)ₙ;
b is selected among 1 and 2;
c and d are independently selected among 0, 1, 2 and 3;
e is selected among 0, 1 and 2;
f, g, h and i are independently selected among 1 and 2;
j, k and 1 are independently selected among 1, 2 and 3;
m and n are independently selected among 1 and 2.

2. The compound according to claim 1, wherein G is selected among: 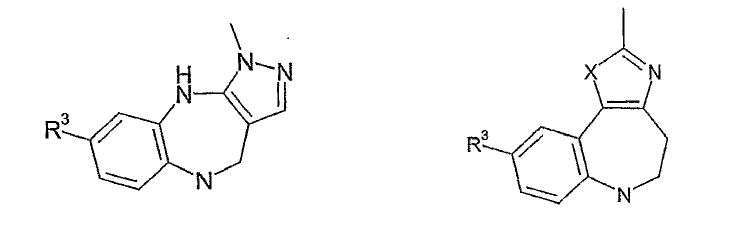 For example:

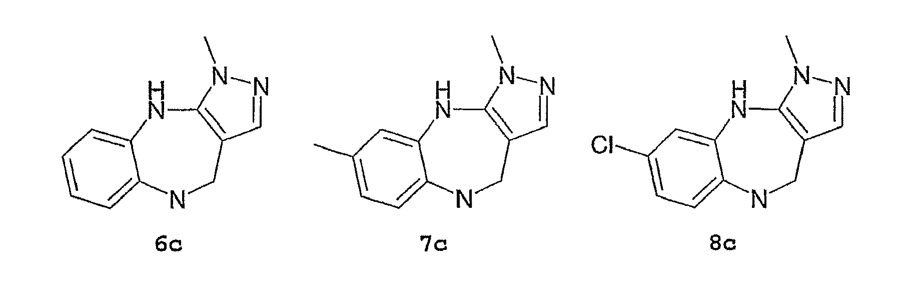

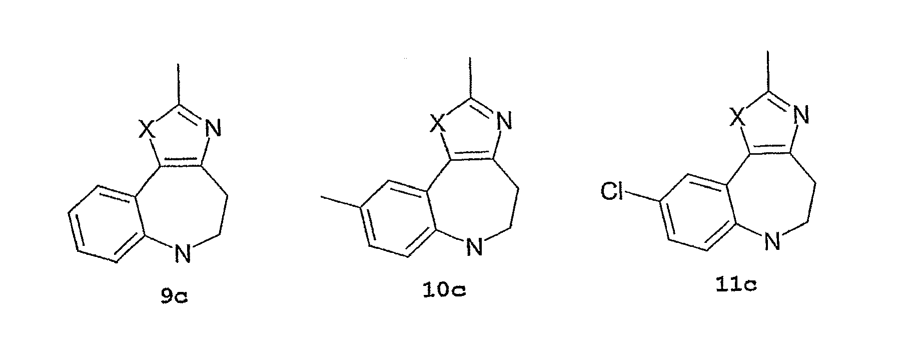

3. The compound according to claim 1, wherein R⁸ is alkyl.

4. The compound according to claim 1, wherein R⁹ is alkyl.

5. The compound according to claim 1, wherein R¹ is selected among: 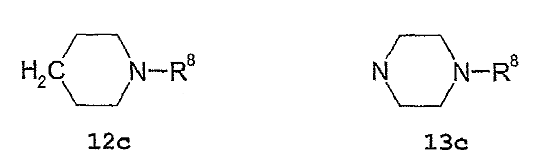 For example:

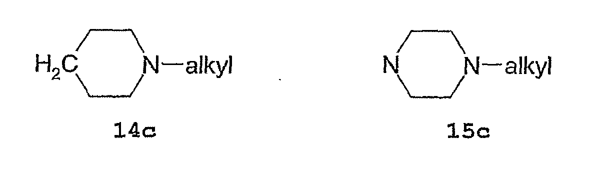

6. The compound according to claim 1, wherein V is NH.

7. The compound according to claim 1, wherein W is C(=O).

8. The compound according to claim 1, wherein a is 1.

9. The compound according to claim 1, wherein V is NH, W is C(=O) and a is 1; as shown in formula 16c: 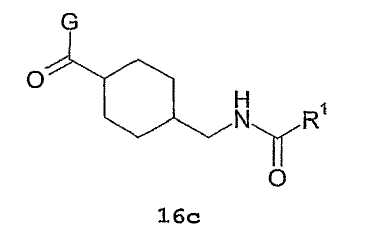 wherein R¹ is selected among general formulae 12c to 15c, preferably R¹ is selected among 14c and 15c.

10. The compound according to claim 1, wherein V is NH, W is C(=O) and a is 1; as shown in formula 17c: 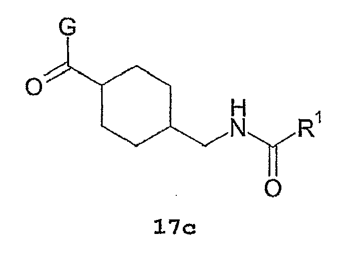 wherein G is selected from general formulae 6c to 11c.

11. The compound according to claim 1, as shown in formula 18c: 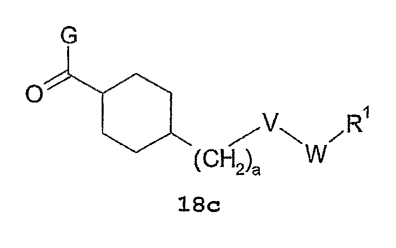 wherein G is selected among general formulae 6c to 11c and R¹ is selected among general formulae 12c to 15c; preferably R¹ is selected among 14c and 15c.

12. The compound according to claim 1, as shown in formula 19c, wherein V is NH, W is C(=O) and a is 1: 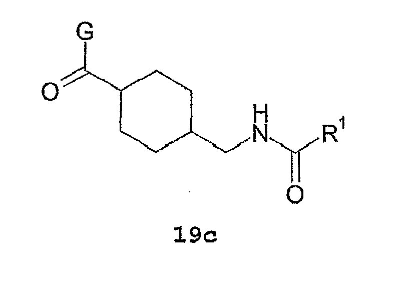 and wherein G is selected among general formulae 6c to 11c, and R¹ is selected among general formulae 12c to 15c; preferably R¹ is selected among 14c and 15c.

13. The compound according to claim 1, selected among the group consisting of:
1-(3,3-Dimethyl-butyl)-piperidine-4-carboxylic acid [4- (3,6-dimethyl-4, 10-dihydro-3H-2,3,4,9-tetraaza-benzo[f]azulene-9-carbonyl)-cyclohexylmethylamide;
1-(3,3-Dimethyl-butyl)-piperidine-4-carboxylic acid [4-(3-methyl-4, 10-dihydro-3H-2,3,4,9-tetraaza-benzo[f]azulene-9-carbonyl) -cyclohexylmethyl]-amide;
1-(3,3-Dimethyl-butyl)-piperidine-4-carboxylic acid [4-(6-chloro-3-methyl-4, 10-dihydro-3H-2,3,4,9-tetraaza-benzo[f]azulene-9-carbonyl)-cyclohexylmethyl]-amide;
1-Cyclopropylmethyl-piperidine-4-carboxylic acid [4-(3, 6-dimethyl-4,10-dihydro-3H-2,3,4,9-tetraaza-benzo[f]azulene-9-carbonyl)-cyclohexylmethyl]-amide;
1-Cyclopropylmethyl-piperidine-4-carboxylic acid [4-(3-methyl-4, 10-dihydro-3H-2,3,4,9-tetraaza-benzo[f]azulene-9-carbonyl)-cyclohexylmethyl]-amide;
1-cyclopropylmethyl-piperidine-4-carboxylic acid [4-(6-chloro-3-methyl-4, 10-dihydro-3H-2,3,4,9-tetraaza-benzo[f]azulene-9-carbonyl)-cyclohexyimethyl]-amide;
4-(2,2-Dimethyl-propyl)-piperazine-1-carboxylic acid [4-(3,6-dimethyl-4, 10-dihydro-3H-2,3,4,9-tetraaza-benzo[f]azulene-9-carbonyl)-cyclohexylmethyl]-amide;
4-(3,3-Dimethyl-butyl)-piperazine-1-carboxylic acid [4-(3,6-dimethyl-4, 10-dihydro-3H-2,3,4,9-tetraaza-benzo[f]azulene-9-carbonyl)-cyclohexylmethyl]-amide;
4-(3,3-Dimethyl-butyl)-piperazine-1-carboxylic acid [4-(3-methyl-4, 10-dihydro-3H-2,3,4,9-tetraaza-benzo[f]azulene-9-carbonyl)-cyclohexylmethyl]-amide;
4-(3,3-Dimethyl-butyl)-piperazine-1-carboxylic acid [4-(6-chloro-3-methyl-4, 10-dihydro-3H-2,3,4,9-tetraaza-benzo[f]azulene-9-carbonyl)-cyclohexylmethyl]-amide;
4-(3-Methyl-butyl)-piperazine-1-carboxylic acid [4-(3,6-dimethyl-4, 10-dihydro-3H-2,3,4,9-tetraaza-benzo[f]azulene-9-carbonyl)-cyclohexylmethyl]amide;
4-(3-Methyl-butyl)-piperazine-1-carboxyiic acid [4-(6-chloro-3-methyl-4, 10-dihydro-3H-2,3,4,9-tetraaza-benzo[f]azulene-9-carbonyl)-cyclohexylmethyl]-amide;
4-Cyclopentylmethyl-piperazine-1-carboxylic acid [4-(3,6-dimethyl-4, 10-dihydro-3H-2,3,4,9-tetraaza-benzo[f]azulene-9-carbonyl)-cyclohexylmethyl]-amide;
4-Cyclopentylmethyl-piperazine-1-carboxylic acid [4-(6-chloro-3-methyl-4, 10-dihydro-3H-2,3,4,9-tetraaza-benzo[f]azulene-9-carbonyl)-cyclohexylmethyl]-amide;
4-Cyclopropylmethyl-piperazine-1-carboxylic acid [4-(3,6-dimethyl-4, 10-dihydro-3H-2,3,4,9-tetraaza-benzo[f]azulene-9-carbonyl)-cyclohexylmethyl]-amide;
4-Cyclopropylmethyl-piperazine-1-carboxylic acid [4-(3- methyl-4, 10-dihydro-3H-2,3,4,9-tetraaza-benzo[f]azulene-9-carbonyl)-cyclohexylmethyl]-amide;
4-Cyclopropylmethyl-piperazine-1-carboxylic acid [4-(6-chloro3-methyl-4, 10-dihydro-3H-2,3,4,9-tetraaza-benzo[f]azulene-9-carbonyl)-cyclohexylmethyl]-amide;
4-Isobutyl-piperazine-1-carboxylic acid [4-(3,6-dimethyl-4, 10-dihydro-3H-2,3,4,9-tetraaza-benzo[f]azulene-9-carbonyl)-cyclohexylmethyl]-amide;
4-Isobutyl-piperazine-1-carboxylic acid [4-(6-chloro-3- methyl-4, 10-dihydro-3H-2,3,4,9-tetraaza-benzo[f]azulene-9-carbonyl)-cyclohexylmethyl]-amide;
4-Propyl-piperazine-1-carboxylic acid [4-(3,6-dimethyl-4, 10-dihydro-3H-2,3,4,9-tetraaza-benzo[f]azulene-9-carbonyl)-cyclohexylmethyl]-amide;
4-Propyl-piperazine-1-carboxylic acid [4-(6-chloro-3-methyl-4, 10-dihydro-3H-2,3,4,9-tetraaza-benzo[f]azulene-9-carbonyl)-cyclohexylmethyl]-amide;
Cyclopropanecarboxylic acid [4-(3,6-dimethyl-4,10-dihydro-3H-2,3,4,9-tetraazabenzo[f]azulene-9-carbonyl)-cyclohexylmethyl]-amide;
Cyclopropanecarboxylic acid [4-(3-methyl-4, 10-dihydro-3H-2,3,4,9-tetraazabenzo[f]azulene-9-carbonyl)-cyclohexylmethyl]-amide; and
Cyclopropanecarboxylic acid [4-(6-chloro-3-methyl-4, 10 dihydro-3H-2,3,4,9-tetraaza-benzo[f]azulene-9-carbonyl)-cyclohexylmethyl]-amide.

14. A pharmaceutical composition comprising a compound according to any of the preceding claims, optionally formulated for oral administration, preferably as a tablet, a capsule or a sachet.

15. The pharmaceutical composition according to claim 14, for the treatment of a condition selected among dysmenorrhoea, pre-term labour, hypertension, Raynaud's disease, brain oedema, motion sickness, hyperlipemia, small cell lung cancer, depression, anxiety, hyponatremia, liver cirrhosis and congestive heart failure.

16. Use of a compound according to any of the claims 1 to 13 for the manufacture of a medicament for the treatment of a condition selected among dysmenorrhoea, pre-term labour, hypertension, Raynaud's disease, brain oedema, motion sickness, hyperlipemia, small cell lung cancer, depression, anxiety, hyponatremia, liver cirrhosis and congestive heart failure.
